(19) Europäisches Patentamt
European Patent Office
Office européen des brevets

(11) **EP 0 958 353 B1**

(12) **EUROPEAN PATENT SPECIFICATION**

(45) Date of publication and mention of the grant of the patent:
**04.03.2009 Bulletin 2009/10**

(21) Application number: **97948748.5**

(22) Date of filing: **15.12.1997**

(51) Int Cl.:
*C12N 9/16* (2006.01)

(86) International application number:
**PCT/DK1997/000568**

(87) International publication number:
**WO 1998/028409 (02.07.1998 Gazette 1998/26)**

(54) **PHYTASE POLYPEPTIDES**

POLYPEPTIDE MIT PHYTASE-AKTIVITÄT

POLYPEPTIDES A ACTIVITE PHYTASE

(84) Designated Contracting States:
**AT BE CH DE DK ES FI FR GB GR IE IT LI NL PT SE**

(30) Priority: **20.12.1996 DK 148096**
**20.12.1996 DK 148196**
**18.03.1997 DK 30197**
**07.05.1997 DK 52997**
**01.12.1997 DK 138897**

(43) Date of publication of application:
**24.11.1999 Bulletin 1999/47**

(83) Declaration under Rule 32(1) EPC (expert solution)

(73) Proprietor: **Novozymes A/S**
**2880 Bagsvaerd (DK)**

(72) Inventors:
• **LASSEN, Soeren Flensted**
**DK-2880 Bagsvaerd (DK)**
• **BECH, Lisbeth**
**DK-2880 Bagsvaerd (DK)**
• **OHMANN, Anders**
**DK-2880 Bagsvaerd (DK)**
• **BREINHOLT, Jens**
**DK-2880 Bagsvaerd (DK)**
• **FUGLSANG, Claus, Crone**
**DK-2880 Bagsvaerd (DK)**

(56) References cited:
**EP-A- 0 420 358** **EP-A- 0 684 313**

• **DIE NAHRUNG, Volume 39, No. 5/6, 1995, G.B. QUAGLIA et al., "Biomass and Hydrolytic and Oxidative Enzymes Production by Fungal Growth on Wheat Milling By-Products", pages 483-489.**
• **DIALOG INFORMATION SERVICES, File 5, BIOSIS, Dialog Accession No. 10480269, Biosis No. 96080269, MCELHINNEY C. et al., "Phosphatase Activity of Four Ectomycorrhizal Fungi Found in a Sitka Spruce-Japanese Larch Plantation in Ireland"; & MYCOL. RES., 97(6), 1993, 725-732.**
• **ENZYME MICROB. TECHNOL., Volume 5, Sept. 1983, S.J. HOWSON et al., "Production of Phytate-Hydrolysing Enzyme by Some Fungi", pages 377-382.**
• **PIR DATABASE, Pirl:Jn0482, Accession No. JN0482, PN0023, ULLAH A.H.J. et al., "Aspergillus Ficuum Phytase; Complete Primarystructure Elucidation by Chemical Sequencing", 30 Sep. 1993; & BIOCHEM. BIOPHYS. RES. COMMUN., 192, 1993, 747-753.**

EP 0 958 353 B1

SEQUENCE LISTING

(1) GENERAL INFORMATION:

(i) APPLICANT:

(A) NAME: Novo Nordisk A/S
(B) STREET: Novo Alle
(C) CITY: DK-2880 Bagsvaerd
(D) STATE: Denmark
(E) COUNTRY: Denmark
(F) POSTAL CODE (ZIP): 2880
(G) TELEPHONE: +45 4444 8888
(H) TELEFAX: +45 4449 3256

(ii) TITLE OF INVENTION: Phytase polypeptides

(iii) NUMBER OF SEQUENCES: 32

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:

(A) LENGTH: 12 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:2
(D) OTHER INFORMATION:/product= "OTHER" /note= "X in position 2 is Y or F"

(ix) FEATURE:

(A) NAME/KEY: Modified-site
(B) LOCATION:3
(D) OTHER INFORMATION:/product= "OTHER" /note= "X in position 3 is F or Y"

(xi) SEQUENCE DESCRIPTION: SEQ ID NO: 1:

```
                    Pro Xaa Xaa Pro Xaa Xaa Xaa Tyr Xaa Xaa Pro Pro
                    1               5                   10
```

(2) INFORMATION FOR SEQ ID NO: 2:

(i) SEQUENCE CHARACTERISTICS:

(A) LENGTH: 17 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:3
(D) OTHER INFORMATION:/note= "X in position 3 is N or H"

(ix) FEATURE:

(A) NAME/KEY: Modified-site
(B) LOCATION:4
(D) OTHER INFORMATION:/note= "X in position 4 is I or L"

(ix) FEATURE:

(A) NAME/KEY: Modified-site
(B) LOCATION:12
(D) OTHER INFORMATION:/note= "X in position 12 is F or W"

(xi) SEQUENCE DESCRIPTION: SEQ ID NO: 2:

```
            Gln Val Xaa Xaa Ile Gln Arg His Gly Ala Arg Xaa Pro Thr Ser Gly
            1               5                   10                  15

            Ala
```

(2) INFORMATION FOR SEQ ID NO: 3:

(i) SEQUENCE CHARACTERISTICS:

(A) LENGTH: 16 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:8
(D) OTHER INFORMATION:/note= "X in position 8 is F or W"

(xi) SEQUENCE DESCRIPTION: SEQ ID NO: 3:

```
     Ile Gln Arg His Gly Ala Arg Xaa Pro Thr Ser Gly Ala Xaa Xaa Arg
      1               5                  10                  15
```

(2) INFORMATION FOR SEQ ID NO: 4:

(i) SEQUENCE CHARACTERISTICS:

(A) LENGTH: 13 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:4
(D) OTHER INFORMATION:/note= "X in position 4 is D or A"

(ix) FEATURE:

(A) NAME/KEY: Modified-site
(B) LOCATION:5
(D) OTHER INFORMATION:/note= "X in position 5 is S or T"

(ix) FEATURE:

(A) NAME/KEY: Modified-site
(B) LOCATION:6
(D) OTHER INFORMATION:/note= "X in position 6 is A or S"

(ix) FEATURE:

(A) NAME/KEY: Modified-site
(B) LOCATION:7
(D) OTHER INFORMATION:/note= "X in position 7 is T or N"

(ix) FEATURE:

    (A) NAME/KEY: Modified-site
    (B) LOCATION:11
    (D) OTHER INFORMATION:/note= "X in position 11 is A or E"

(xi) SEQUENCE DESCRIPTION: SEQ ID NO: 4:

```
            Arg Val Val Xaa Xaa Xaa Xaa Asn Trp Thr Xaa Gly Phe
            1               5                   10
```

(2) INFORMATION FOR SEQ ID NO: 5:

(i) SEQUENCE CHARACTERISTICS:

    (A) LENGTH: 10 amino acids
    (B) TYPE: amino acid
    (C) STRANDEDNESS:
    (D) TOPOLOGY: linear

(ii) MOLECULE TYPE: peptide

(v) FRAGMENT TYPE: internal

(ix) FEATURE:

    (A) NAME/KEY: Modified-site
    (B) LOCATION:4
    (D) OTHER INFORMATION:/note= "X in position 4 is A or E"

(xi) SEQUENCE DESCRIPTION: SEQ ID NO: 5:

```
            Asn Trp Thr Xaa Gly Phe Xaa Xaa Ala Ser
            1               5                   10
```

(2) INFORMATION FOR SEQ ID NO: 6:

(i) SEQUENCE CHARACTERISTICS:

    (A) LENGTH: 12 amino acids
    (B) TYPE: amino acid
    (C) STRANDEDNESS:
    (D) TOPOLOGY: linear

(ii) MOLECULE TYPE: peptide

(v) FRAGMENT TYPE: internal

(xi) SEQUENCE DESCRIPTION: SEQ ID NO: 6:

```
            Gly Phe Xaa Xaa Ala Ser Xaa Xaa Xaa Xaa Xaa Pro
            1               5                   10
```

(2) INFORMATION FOR SEQ ID NO: 7:

(i) SEQUENCE CHARACTERISTICS:

    (A) LENGTH: 16 amino acids
    (B) TYPE: amino acid
    (C) STRANDEDNESS:
    (D) TOPOLOGY: linear

(ii) MOLECULE TYPE: peptide

(v) FRAGMENT TYPE: internal

(ix) FEATURE:

    (A) NAME/KEY: Modified-site
    (B) LOCATION:2
    (D) OTHER INFORMATION:/note= "X in position 2 is N or D"

(ix) FEATURE:

    (A) NAME/KEY: Modified-site
    (B) LOCATION:3
    (D) OTHER INFORMATION:/note= "X in position 3 is P or E"

(ix) FEATURE:

    (A) NAME/KEY: Modified-site
    (B) LOCATION:6
    (D) OTHER INFORMATION:/note= "X in position 6 is T or L"

(ix) FEATURE:

    (A) NAME/KEY: Modified-site
    (B) LOCATION:7
    (D) OTHER INFORMATION:/note= "X in position 7 is W or F"

(ix) FEATURE:

    (A) NAME/KEY: Modified-site
    (B) LOCATION:10
    (D) OTHER INFORMATION:/note= "X in position 10 is S or K"

(ix) FEATURE:

    (A) NAME/KEY: Modified-site
    (B) LOCATION:13
    (D) OTHER INFORMATION:/note= "X in position 13 is V or T"

(xi) SEQUENCE DESCRIPTION: SEQ ID NO: 7:

```
Pro Xaa Xaa Xaa Arg Xaa Xaa Xaa Xaa Xaa Xaa Xaa Xaa Pro Phe Ser
 1               5                   10                  15
```

(2) INFORMATION FOR SEQ ID NO: 8:

(i) SEQUENCE CHARACTERISTICS:

    (A) LENGTH: 11 amino acids

(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:2
(D) OTHER INFORMATION:/note= "X in position 2 is Q or A"

(ix) FEATURE:

(A) NAME/KEY: Modified-site
(B) LOCATION:3
(D) OTHER INFORMATION:/note= "X in position 3 is V or L"

(ix) FEATURE:

(A) NAME/KEY: Modified-site
(B) LOCATION:11
(D) OTHER INFORMATION:/note= "X in position 11 is G or A"

(xi) SEQUENCE DESCRIPTION: SEQ ID NO: 8:

```
Asp Xaa Xaa Gln Pro Leu Xaa Phe Cys Gly Xaa
1               5                   10
```

(2) INFORMATION FOR SEQ ID NO: 9:

(i) SEQUENCE CHARACTERISTICS:

(A) LENGTH: 19 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:7
(D) OTHER INFORMATION:/note= "X in position 7 is Y or F"

(ix) FEATURE:

(A) NAME/KEY: Modified-site

(B) LOCATION:17
(D) OTHER INFORMATION:/note= "X in position 17 is E or A"

(xi) SEQUENCE DESCRIPTION: SEQ ID NO: 9:

```
Phe Val Glu Ser Gln Xaa Xaa Ala Arg Xaa Xaa Gly Xaa Gly Asp Phe
1               5                   10                  15

Xaa Lys Cys
```

(2) INFORMATION FOR SEQ ID NO: 10:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:4
(D) OTHER INFORMATION:/note= "X in position 4 is A or E"

(xi) SEQUENCE DESCRIPTION: SEQ ID NO: 10:

```
Asn Trp Thr Xaa Gly Phe
1               5
```

(2) INFORMATION FOR SEQ ID NO: 11:

(i) SEQUENCE CHARACTERISTICS:

(A) LENGTH: 6 amino acids
(B) TYPE: amino acid
(C) STRANDEDNESS:
(D) TOPOLOGY: linear

(ii) MOLECULE TYPE: peptide

(v) FRAGMENT TYPE: internal

(ix) FEATURE:

(A) NAME/KEY: Modified-site
(B) LOCATION:3
(D) OTHER INFORMATION:/note= "X in position 3 is F or Y"

(xi) SEQUENCE DESCRIPTION: SEQ ID NO: 11:

```
Asp Lys Xaa Tyr Gly Thr
1               5
```

(2) INFORMATION FOR SEQ ID NO: 12:

    (i) SEQUENCE CHARACTERISTICS:

        (A) LENGTH: 7 amino acids
        (B) TYPE: amino acid
        (C) STRANDEDNESS:
        (D) TOPOLOGY: linear

    (ii) MOLECULE TYPE: peptide

    (v) FRAGMENT TYPE: internal

    (ix) FEATURE:

        (A) NAME/KEY: Modified-site
        (B) LOCATION:5
        (D) OTHER INFORMATION:/note= "X in position 5 is F or Y"

    (xi) SEQUENCE DESCRIPTION: SEQ ID NO: 12:

```
Asp Leu Asp Lys Xaa Tyr Gly
1               5
```

(2) INFORMATION FOR SEQ ID NO: 13:

    (i) SEQUENCE CHARACTERISTICS:

        (A) LENGTH: 5 amino acids
        (B) TYPE: amino acid
        (C) STRANDEDNESS:
        (D) TOPOLOGY: linear

    (ii) MOLECULE TYPE: peptide

    (v) FRAGMENT TYPE: internal

    (ix) FEATURE:

        (A) NAME/KEY: Modified-site
        (B) LOCATION:4
        (D) OTHER INFORMATION:/note= "X in position 4 is A or E"

    (xi) SEQUENCE DESCRIPTION: SEQ ID NO: 13:

```
Gly Asp Phe Xaa Lys
1             5
```

(2) INFORMATION FOR SEQ ID NO: 14:

    (i) SEQUENCE CHARACTERISTICS:

        (A) LENGTH: 6 amino acids
        (B) TYPE: amino acid
        (C) STRANDEDNESS:
        (D) TOPOLOGY: linear

(ii) MOLECULE TYPE: peptide

(v) FRAGMENT TYPE: internal

(ix) FEATURE:

    (A) NAME/KEY: Modified-site
    (B) LOCATION:3
    (D) OTHER INFORMATION:/note= "X in position 3 is N or H"

(ix) FEATURE:

    (A) NAME/KEY: Modified-site

    (B) LOCATION:4
    (D) OTHER INFORMATION:/note= "X in position 4 is I or L"

(xi) SEQUENCE DESCRIPTION: SEQ ID NO: 14:

```
                              Gln Val Xaa Xaa Ile Gln
                              1               5
```

(2) INFORMATION FOR SEQ ID NO: 15:

(i) SEQUENCE CHARACTERISTICS:

    (A) LENGTH: 26 base pairs
    (B) TYPE: nucleic acid
    (C) STRANDEDNESS: single
    (D) TOPOLOGY: linear

(ii) MOLECULE TYPE: other nucleic acid (A) DESCRIPTION: /desc = "sense primer"

(iv) ANTI-SENSE: NO

(xi) SEQUENCE DESCRIPTION: SEQ ID NO: 15:

    CCCAAGCTTA AYTGGACNGM NGGNTT     26

(2) INFORMATION FOR SEQ ID NO: 16:

(i) SEQUENCE CHARACTERISTICS:

    (A) LENGTH: 23 base pairs
    (B) TYPE: nucleic acid
    (C) STRANDEDNESS: single
    (D) TOPOLOGY: linear

(ii) MOLECULE TYPE: other nucleic acid (A) DESCRIPTION: /desc = "sense primer"

(iv) ANTI-SENSE: NO

(xi) SEQUENCE DESCRIPTION: SEQ ID NO: 16:

    CCCAAGCTTG AYAARTWYGG NAC     23

(2) INFORMATION FOR SEQ ID NO: 17:

(i) SEQUENCE CHARACTERISTICS:

   (A) LENGTH: 27 base pairs
   (B) TYPE: nucleic acid
   (C) STRANDEDNESS: single
   (D) TOPOLOGY: linear

(ii) MOLECULE TYPE: other nucleic acid (A) DESCRIPTION: /desc = "anti-sense primer"

(iv) ANTI-SENSE: YES

(xi) SEQUENCE DESCRIPTION: SEQ ID NO: 17:

   GCTCTAGACR TARWAYTTRT CNARRTC          27

(2) INFORMATION FOR SEQ ID NO: 18:

   (i) SEQUENCE CHARACTERISTICS:

   (A) LENGTH: 25 base pairs
   (B) TYPE: nucleic acid
   (C) STRANDEDNESS: single
   (D) TOPOLOGY: linear

(ii) MOLECULE TYPE: other nucleic acid (A) DESCRIPTION: /desc = "anti-sense primer"

(iv) ANTI-SENSE: YES

(xi) SEQUENCE DESCRIPTION: SEQ ID NO: 18:

   GCTCTAGACA YTTNKCRAAR TCNCC          25

(2) INFORMATION FOR SEQ ID NO: 19:

   (i) SEQUENCE CHARACTERISTICS:

   (A) LENGTH: 26 base pairs
   (B) TYPE: nucleic acid
   (C) STRANDEDNESS: single
   (D) TOPOLOGY: linear

(ii) MOLECULE TYPE: other nucleic acid (A) DESCRIPTION: /desc = "sense primer"

(iv) ANTI-SENSE: NO

(xi) SEQUENCE DESCRIPTION: SEQ ID NO: 19:

   CCCAAGCTTC ARGTNMAYMT NATHCA          26

(2) INFORMATION FOR SEQ ID NO: 20:

   (i) SEQUENCE CHARACTERISTICS:

   (A) LENGTH: 27 base pairs
   (B) TYPE: nucleic acid
   (C) STRANDEDNESS: single
   (D) TOPOLOGY: linear

(ii) MOLECULE TYPE: other nucleic acid (A) DESCRIPTION: /desc = "anti-sense primer"

(iv) ANTI-SENSE: YES

(xi) SEQUENCE DESCRIPTION: SEQ ID NO: 20:

```
GCTCTAGACR AANCCNKCNG TCCARTT        27
```

(2) INFORMATION FOR SEQ ID NO: 21:

(i) SEQUENCE CHARACTERISTICS:

(A) LENGTH: 1501 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Agrocybe pediades
(B) STRAIN: CBS 900.96

(ix) FEATURE:

(A) NAME/KEY: CDS
(B) LOCATION:17..1375

(ix) FEATURE:

(A) NAME/KEY: sig_peptide
(B) LOCATION:17..94

(ix) FEATURE:

(A) NAME/KEY: mat_peptide
(B) LOCATION:95..1375

(xi) SEQUENCE DESCRIPTION: SEQ ID NO: 21:

```
GGATCCGAAT TCACTT ATG TCC CTC TTC ATC GGC GGC TGT TTG CTC GTG        49
                  Met Ser Leu Phe Ile Gly Gly Cys Leu Leu Val
                  -26 -25                     -20

TTT TTA CAG GCG AGC GCA TAC GGC GGC GTC GTG CAG GCC ACA TTC GTG        97
Phe Leu Gln Ala Ser Ala Tyr Gly Gly Val Val Gln Ala Thr Phe Val
-15              -10                     -5                      1

CAG CCG TTT TTC CCT CCA CAG ATT CAG GAC TCT TGG GCA GCT TAT ACA        145
Gln Pro Phe Phe Pro Pro Gln Ile Gln Asp Ser Trp Ala Ala Tyr Thr
          5                     10                      15
```

```
CCA TAT TAT CCT GTT CAG GCG TAC ACG CCT CCC CCG AAG GAT TGC AAG        193
Pro Tyr Tyr Pro Val Gln Ala Tyr Thr Pro Pro Pro Lys Asp Cys Lys
        20                  25                  30

ATC ACA CAA GTT AAC ATT ATT CAA CGA CAT GGT GCC CGC TTT CCG ACA        241
Ile Thr Gln Val Asn Ile Ile Gln Arg His Gly Ala Arg Phe Pro Thr
    35                  40                  45

TCG GGG GCA GGC ACA AGG ATC CAA GCA GCT GTG AAG AAG CTT CAA TCA        289
Ser Gly Ala Gly Thr Arg Ile Gln Ala Ala Val Lys Lys Leu Gln Ser
50                  55                  60                      65

GCT AAA ACC TAT ACG GAT CCT CGT CTC GAC TTT CTG ACC AAC TAT ACC        337
Ala Lys Thr Tyr Thr Asp Pro Arg Leu Asp Phe Leu Thr Asn Tyr Thr
                70                  75                  80

TAT ACC CTT GGT CAC GAC GAT CTC GTA CCG TTT GGA GCG CTT CAA TCA        385
Tyr Thr Leu Gly His Asp Asp Leu Val Pro Phe Gly Ala Leu Gln Ser
                85                  90                  95

TCA CAA GCT GGA GAG GAA ACG TTT CAA CGA TAC TCG TTT CTG GTG TCC        433
Ser Gln Ala Gly Glu Glu Thr Phe Gln Arg Tyr Ser Phe Leu Val Ser
            100                 105                 110

AAA GAG AAC TTA CCT TTT GTA AGA GCT TCG AGT TCC AAT CGA GTC GTC        481
Lys Glu Asn Leu Pro Phe Val Arg Ala Ser Ser Ser Asn Arg Val Val
        115                 120                 125

GAC TCA GCT ACC AAC TGG ACG GAA GGT TTT TCT GCG GCC AGT CAC CAC        529
Asp Ser Ala Thr Asn Trp Thr Glu Gly Phe Ser Ala Ala Ser His His
130                 135                 140                 145

GTC TTG AAT CCC ATT CTC TTT GTA ATC CTC TCA GAA AGT CTC AAT GAC        577
Val Leu Asn Pro Ile Leu Phe Val Ile Leu Ser Glu Ser Leu Asn Asp
                150                 155                 160

ACG CTT GAC GAT GCC ATG TGC CCT AAC GCG GGC TCC TCC GAC CCG CAG        625
Thr Leu Asp Asp Ala Met Cys Pro Asn Ala Gly Ser Ser Asp Pro Gln
            165                 170                 175

ACT GGT ATC TGG ACC TCG ATA TAC GGG ACG CCT ATT GCC AAC CGA CTA        673
Thr Gly Ile Trp Thr Ser Ile Tyr Gly Thr Pro Ile Ala Asn Arg Leu
            180                 185                 190

AAT CAG CAG GCT CCG GGT GCA AAT ATT ACA GCT GCC GAT GTG TCG AAC        721
Asn Gln Gln Ala Pro Gly Ala Asn Ile Thr Ala Ala Asp Val Ser Asn
        195                 200                 205

CTT ATA CCG CTT TGC GCA TTC GAG ACG ATA GTA AAG GAG ACG CCA AGT        769
Leu Ile Pro Leu Cys Ala Phe Glu Thr Ile Val Lys Glu Thr Pro Ser
210                 215                 220                 225

CCT TTC TGT AAT TTG TTC ACC CCC GAA GAG TTC GCA CAG TTT GAA TAT        817
Pro Phe Cys Asn Leu Phe Thr Pro Glu Glu Phe Ala Gln Phe Glu Tyr
                230                 235                 240

TTC GGT GAC CTG GAC AAG TTC TAT GGG ACA GGT TAT GGA CAA CCG TTA        865
Phe Gly Asp Leu Asp Lys Phe Tyr Gly Thr Gly Tyr Gly Gln Pro Leu
            245                 250                 255

GGA CCT GTG CAA GGT GTC GGC TAC ATC AAT GAA CTT CTT GCC CGA CTC        913
Gly Pro Val Gln Gly Val Gly Tyr Ile Asn Glu Leu Leu Ala Arg Leu
            260                 265                 270

ACA GAA ATG CCA GTT CGA GAT AAC ACC CAG ACG AAC AGG ACA CTC GAC        961
Thr Glu Met Pro Val Arg Asp Asn Thr Gln Thr Asn Arg Thr Leu Asp
    275                 280                 285

TCT TCT CCG CTT ACA TTT CCC CTC GAC CGC AGT ATC TAC GCT GAC CTC       1009
Ser Ser Pro Leu Thr Phe Pro Leu Asp Arg Ser Ile Tyr Ala Asp Leu
290                 295                 300                 305

TCG CAC GAT AAC CAA ATG ATC GCG ATA TTT TCA GCG ATG GGT CTT TTC       1057
Ser His Asp Asn Gln Met Ile Ala Ile Phe Ser Ala Met Gly Leu Phe
            310                 315                 320
```

57

```
AAC CAG AGT TCA CCT TTG GAT CCG TCC TTC CCC AAC CCC AAG CGT ACT     1105
Asn Gln Ser Ser Pro Leu Asp Pro Ser Phe Pro Asn Pro Lys Arg Thr
        325                     330                 335

TGG GTC ACC AGT CGG CTT ACG CCT TTC AGC GCG AGA ATG GTC ACT GAG     1153
Trp Val Thr Ser Arg Leu Thr Pro Phe Ser Ala Arg Met Val Thr Glu
        340                     345                 350

CGG TTG CTG TGT CAA AGG GAT GGG ACA GGG AGC GGT GGA CCA TCC AGG     1201
Arg Leu Leu Cys Gln Arg Asp Gly Thr Gly Ser Gly Gly Pro Ser Arg
    355                     360                 365

ATC ATG CGG AAT GGA AAT GTG CAG ACG TTT GTG AGG ATT CTT GTC AAC     1249
Ile Met Arg Asn Gly Asn Val Gln Thr Phe Val Arg Ile Leu Val Asn
370                     375                 380                 385

GAT GCT TTA CAG CCT TTG AAG TTC TGC GGA GGG GAC ATG GAT AGT TTG     1297
Asp Ala Leu Gln Pro Leu Lys Phe Cys Gly Gly Asp Met Asp Ser Leu
                390                 395                 400

TGT ACT CTG GAA GCG TTC GTC GAG AGC CAG AAG TAT GCA CGA GAG GAT     1345
Cys Thr Leu Glu Ala Phe Val Glu Ser Gln Lys Tyr Ala Arg Glu Asp
            405                 410                 415

GGT CAA GGC GAT TTT GAA AAA TGT TTT GAT TAAATATTGC AGTATGCTCA       1395
Gly Gln Gly Asp Phe Glu Lys Cys Phe Asp
        420                 425

GTGAGTAGAC TACAGTGCAG GCCCTGTAAC TCTTGTATTG TGTTTCTGGA ATTCCTCGGA   1455

GCGTAGTTTG TAGCAAAAAA AAAAAAAAAA AAATTCCTGC GGCCGC                  1501
```

(2) INFORMATION FOR SEQ ID NO: 22:

    (i) SEQUENCE CHARACTERISTICS:

        (A) LENGTH: 453 amino acids
        (B) TYPE: amino acid
        (D) TOPOLOGY: linear

    (ii) MOLECULE TYPE: protein

    (xi) SEQUENCE DESCRIPTION: SEQ ID NO: 22:

```
Met Ser Leu Phe Ile Gly Gly Cys Leu Leu Val Phe Leu Gln Ala Ser
-26 -25              -20                 -15

Ala Tyr Gly Gly Val Val Gln Ala Thr Phe Val Gln Pro Phe Phe Pro
-10           -5                  1                   5

Pro Gln Ile Gln Asp Ser Trp Ala Ala Tyr Thr Pro Tyr Tyr Pro Val
            10              15                  20

Gln Ala Tyr Thr Pro Pro Pro Lys Asp Cys Lys Ile Thr Gln Val Asn
        25              30              35

Ile Ile Gln Arg His Gly Ala Arg Phe Pro Thr Ser Gly Ala Gly Thr
    40              45              50

Arg Ile Gln Ala Ala Val Lys Lys Leu Gln Ser Ala Lys Thr Tyr Thr
55              60              65                  70

Asp Pro Arg Leu Asp Phe Leu Thr Asn Tyr Thr Tyr Thr Leu Gly His
            75              80              85

Asp Asp Leu Val Pro Phe Gly Ala Leu Gln Ser Ser Gln Ala Gly Glu
            90              95              100

Glu Thr Phe Gln Arg Tyr Ser Phe Leu Val Ser Lys Glu Asn Leu Pro
        105             110             115

Phe Val Arg Ala Ser Ser Ser Asn Arg Val Val Asp Ser Ala Thr Asn
    120             125             130

Trp Thr Glu Gly Phe Ser Ala Ala Ser His His Val Leu Asn Pro Ile
135             140             145                 150
```

```
Leu Phe Val Ile Leu Ser Glu Ser Leu Asn Asp Thr Leu Asp Asp Ala
                155             160             165
Met Cys Pro Asn Ala Gly Ser Ser Asp Pro Gln Thr Gly Ile Trp Thr
                170             175             180
Ser Ile Tyr Gly Thr Pro Ile Ala Asn Arg Leu Asn Gln Gln Ala Pro
                185             190             195
Gly Ala Asn Ile Thr Ala Ala Asp Val Ser Asn Leu Ile Pro Leu Cys
        200             205             210
Ala Phe Glu Thr Ile Val Lys Glu Thr Pro Ser Pro Phe Cys Asn Leu
215             220             225             230
Phe Thr Pro Glu Glu Phe Ala Gln Phe Glu Tyr Phe Gly Asp Leu Asp
                235             240             245
Lys Phe Tyr Gly Thr Gly Tyr Gly Gln Pro Leu Gly Pro Val Gln Gly
                250             255             260
Val Gly Tyr Ile Asn Glu Leu Leu Ala Arg Leu Thr Glu Met Pro Val
                265             270             275
Arg Asp Asn Thr Gln Thr Asn Arg Thr Leu Asp Ser Ser Pro Leu Thr
        280             285             290
Phe Pro Leu Asp Arg Ser Ile Tyr Ala Asp Leu Ser His Asp Asn Gln
295             300             305             310
Met Ile Ala Ile Phe Ser Ala Met Gly Leu Phe Asn Gln Ser Ser Pro
                315             320             325
Leu Asp Pro Ser Phe Pro Asn Pro Lys Arg Thr Trp Val Thr Ser Arg
                330             335             340
Leu Thr Pro Phe Ser Ala Arg Met Val Thr Glu Arg Leu Leu Cys Gln
                345             350             355
Arg Asp Gly Thr Gly Ser Gly Gly Pro Ser Arg Ile Met Arg Asn Gly
        360             365             370
Asn Val Gln Thr Phe Val Arg Ile Leu Val Asn Asp Ala Leu Gln Pro
375             380             385             390
Leu Lys Phe Cys Gly Gly Asp Met Asp Ser Leu Cys Thr Leu Glu Ala
                395             400             405
Phe Val Glu Ser Gln Lys Tyr Ala Arg Glu Asp Gly Gln Gly Asp Phe
                410             415             420
Glu Lys Cys Phe Asp
                425
```

(2) INFORMATION FOR SEQ ID NO: 23:

(i) SEQUENCE CHARACTERISTICS:

(A) LENGTH: 1593 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Peniophora lycii

(B) STRAIN: CBS 686.96

(ix) FEATURE:

(A) NAME/KEY: sig_peptide
(B) LOCATION:123..212

(ix) FEATURE:

(A) NAME/KEY: mat_peptide
(B) LOCATION:213..1439

(ix) FEATURE:

(A) NAME/KEY: CDS
(B) LOCATION:123..1439

(xi) SEQUENCE DESCRIPTION: SEQ ID NO: 23:

```
GGATCCGAAT TCCATCTTCT GCTCTGACCT CCATCTCGCT GAGCGGCCGA CGAGAACCTA          60

GGGGCTCTAA GTCCACGTAC TATCGCCGCG CCTGTGAAGG CCCCATACCA GCCCTTATCG         120

AT ATG GTT TCT TCG GCA TTC GCA CCT TCC ATC CTA CTT AGC TTG ATG           167
   Met Val Ser Ser Ala Phe Ala Pro Ser Ile Leu Leu Ser Leu Met
   -30              -25                  -20

TCG AGT CTT GCT TTG AGC ACG CAG TTC AGC TTT GTT GCG GCG CAG CTA          215
Ser Ser Leu Ala Leu Ser Thr Gln Phe Ser Phe Val Ala Ala Gln Leu
-15              -10                  -5                        1

CCT ATC CCC GCA CAA AAC ACA AGT AAT TGG GGG CCT TAC GAT CCC TTC          263
Pro Ile Pro Ala Gln Asn Thr Ser Asn Trp Gly Pro Tyr Asp Pro Phe
              5                  10                  15

TTT CCC GTC GAA CCG TAT GCA GCT CCG CCG GAA GGG TGC ACA GTG ACA          311
Phe Pro Val Glu Pro Tyr Ala Ala Pro Pro Glu Gly Cys Thr Val Thr
              20                  25                  30

CAG GTC AAC CTG ATT CAG AGG CAC GGC GCG CGT TGG CCC ACA TCC GGC          359
Gln Val Asn Leu Ile Gln Arg His Gly Ala Arg Trp Pro Thr Ser Gly
    35                  40                  45

GCG CGG TCG CGG CAG GTC GCC GCC GTA GCG AAG ATA CAA ATG GCG CGA          407
Ala Arg Ser Arg Gln Val Ala Ala Val Ala Lys Ile Gln Met Ala Arg
50                  55                  60                  65

CCA TTC ACG GAT CCC AAG TAT GAG TTC CTC AAC GAC TTC GTG TAC AAG          455
Pro Phe Thr Asp Pro Lys Tyr Glu Phe Leu Asn Asp Phe Val Tyr Lys
              70                  75                  80

TTC GGC GTC GCC GAT CTG CTA CCG TTC GGG GCT AAC CAA TCG CAC CAA          503
Phe Gly Val Ala Asp Leu Leu Pro Phe Gly Ala Asn Gln Ser His Gln
              85                  90                  95

ACC GGC ACC GAT ATG TAT ACG CGC TAC AGT ACA CTA TTT GAG GGC GGG          551
Thr Gly Thr Asp Met Tyr Thr Arg Tyr Ser Thr Leu Phe Glu Gly Gly
              100                 105                 110

GAT GTA CCC TTT GTG CGC GCG GCT GGT GAC CAA CGC GTC GTT GAC TCC          599
Asp Val Pro Phe Val Arg Ala Ala Gly Asp Gln Arg Val Val Asp Ser
    115                 120                 125

TCG ACG AAC TGG ACG GCA GGC TTT GGC GAT GCT TCT GGC GAG ACT GTT          647
Ser Thr Asn Trp Thr Ala Gly Phe Gly Asp Ala Ser Gly Glu Thr Val
130                 135                 140                 145

CTC CCG ACG CTC CAG GTT GTG CTT CAA GAA GAG GGG AAC TGC ACG CTC          695
Leu Pro Thr Leu Gln Val Val Leu Gln Glu Glu Gly Asn Cys Thr Leu
              150                 155                 160

TGC AAT AAT ATG TGC CCG AAT GAA GTG GAT GGT GAC GAA TCC ACA ACG          743
Cys Asn Asn Met Cys Pro Asn Glu Val Asp Gly Asp Glu Ser Thr Thr
              165                 170                 175

TGG CTG GGG GTC TTT GCG CCG AAC ATC ACC GCG CGA TTG AAC GCT GCT          791
Trp Leu Gly Val Phe Ala Pro Asn Ile Thr Ala Arg Leu Asn Ala Ala
              180                 185                 190

GCG CCG AGT GCC AAC CTC TCA GAC AGC GAC GCG CTC ACT CTC ATG GAT          839
Ala Pro Ser Ala Asn Leu Ser Asp Ser Asp Ala Leu Thr Leu Met Asp
    195                 200                 205

ATG TGC CCG TTC GAC ACT CTC AGC TCC GGG AAC GCC AGC CCC TTC TGT          887
Met Cys Pro Phe Asp Thr Leu Ser Ser Gly Asn Ala Ser Pro Phe Cys
210                 215                 220                 225
```

```
GAC CTA TTT ACC GCG GAG GAG TAT GTG TCG TAC GAG TAC TAC TAT GAC        935
Asp Leu Phe Thr Ala Glu Glu Tyr Val Ser Tyr Glu Tyr Tyr Tyr Asp
            230             235             240

CTC GAC AAG TAC TAT GGC ACG GGC CCC GGG AAC GCT CTC GGT CCT GTC        983
Leu Asp Lys Tyr Tyr Gly Thr Gly Pro Gly Asn Ala Leu Gly Pro Val
            245             250             255

CAG GGC GTC GGA TAC GTC AAT GAG CTG CTT GCA CGC TTG ACC GGC CAA       1031
Gln Gly Val Gly Tyr Val Asn Glu Leu Leu Ala Arg Leu Thr Gly Gln
        260             265             270

GCC GTT CGA GAC GAG ACG CAG ACG AAC CGC ACG CTC GAC AGC GAC CCT       1079
Ala Val Arg Asp Glu Thr Gln Thr Asn Arg Thr Leu Asp Ser Asp Pro
        275             280             285

GCA ACA TTC CCG CTG AAC CGT ACG TTC TAC GCC GAC TTC TCG CAT GAT       1127
Ala Thr Phe Pro Leu Asn Arg Thr Phe Tyr Ala Asp Phe Ser His Asp
290             295             300             305

AAC ACC ATG GTG CCC ATC TTT GCG GCG CTC GGG CTC TTC AAC GCC ACC       1175
Asn Thr Met Val Pro Ile Phe Ala Ala Leu Gly Leu Phe Asn Ala Thr
            310             315             320

GCC CTC GAC CCG CTG AAG CCC GAC GAG AAC AGG TTG TGG GTG GAC TCT       1223
Ala Leu Asp Pro Leu Lys Pro Asp Glu Asn Arg Leu Trp Val Asp Ser
            325             330             335

AAG CTG GTA CCG TTC TCT GGA CAT ATG ACG GTC GAG AAG CTG GCA TGT       1271
Lys Leu Val Pro Phe Ser Gly His Met Thr Val Glu Lys Leu Ala Cys
        340             345             350

TCT GGG AAG GAG GCG GTC AGG GTG CTC GTG AAC GAC GCG GTG CAG CCG       1319
Ser Gly Lys Glu Ala Val Arg Val Leu Val Asn Asp Ala Val Gln Pro
        355             360             365

CTG GAG TTC TGC GGA GGT GTT GAT GGG GTG TGC GAG CTT TCG GCT TTC       1367
Leu Glu Phe Cys Gly Gly Val Asp Gly Val Cys Glu Leu Ser Ala Phe
370             375             380             385

GTA GAG AGC CAG ACG TAT GCG CGG GAG AAT GGG CAA GGC GAC TTC GCC       1415
Val Glu Ser Gln Thr Tyr Ala Arg Glu Asn Gly Gln Gly Asp Phe Ala
            390             395             400

AAG TGC GGC TTT GTT CCG TCG GAA TAGCGGGAGA CCGTCTATGC TACACAGTAA      1469
Lys Cys Gly Phe Val Pro Ser Glu
            405

TTGTGTACTC TATAGCACTG TAGCTGTACT TACAAGTCGT AGGGTACGAT CGTACTTACG      1529

CTCGTTTATT GATCCTTCCT TTAAAAAAAA AAAAAAAAAA AAAAAAAAAA ATTCCTGCGG      1589

CCGC                                                                  1593
```

(2) INFORMATION FOR SEQ ID NO: 24:

(i) SEQUENCE CHARACTERISTICS:

(A) LENGTH: 439 amino acids
(B) TYPE: amino acid
(D) TOPOLOGY: linear

(ii) MOLECULE TYPE: protein

(xi) SEQUENCE DESCRIPTION: SEQ ID NO: 24:

```
Met Val Ser Ser Ala Phe Ala Pro Ser Ile Leu Leu Ser Leu Met Ser
-30                 -25                 -20                 -15

Ser Leu Ala Leu Ser Thr Gln Phe Ser Phe Val Ala Ala Gln Leu Pro
            -10                 -5                      1

Ile Pro Ala Gln Asn Thr Ser Asn Trp Gly Pro Tyr Asp Pro Phe Phe
        5                 10              15

Pro Val Glu Pro Tyr Ala Ala Pro Pro Glu Gly Cys Thr Val Thr Gln
    20              25              30
```

Val Asn Leu Ile Gln Arg His Gly Ala Arg Trp Pro Thr Ser Gly Ala
35              40                  45                    50

Arg Ser Arg Gln Val Ala Ala Val Ala Lys Ile Gln Met Ala Arg Pro
                55                  60                  65

Phe Thr Asp Pro Lys Tyr Glu Phe Leu Asn Asp Phe Val Tyr Lys Phe
            70                  75                  80

Gly Val Ala Asp Leu Leu Pro Phe Gly Ala Asn Gln Ser His Gln Thr
        85                  90                  95

Gly Thr Asp Met Tyr Thr Arg Tyr Ser Thr Leu Phe Glu Gly Gly Asp
    100                 105                 110

Val Pro Phe Val Arg Ala Ala Gly Asp Gln Arg Val Val Asp Ser Ser
115                 120                 125                 130

Thr Asn Trp Thr Ala Gly Phe Gly Asp Ala Ser Gly Glu Thr Val Leu
            135                 140                 145

Pro Thr Leu Gln Val Val Leu Gln Glu Glu Gly Asn Cys Thr Leu Cys
            150                 155                 160

Asn Asn Met Cys Pro Asn Glu Val Asp Gly Asp Glu Ser Thr Thr Trp
            165                 170                 175

Leu Gly Val Phe Ala Pro Asn Ile Thr Ala Arg Leu Asn Ala Ala Ala
    180                 185                 190

Pro Ser Ala Asn Leu Ser Asp Ser Asp Ala Leu Thr Leu Met Asp Met
195                 200                 205                 210

Cys Pro Phe Asp Thr Leu Ser Ser Gly Asn Ala Ser Pro Phe Cys Asp
            215                 220                 225

Leu Phe Thr Ala Glu Glu Tyr Val Ser Tyr Glu Tyr Tyr Tyr Asp Leu
        230                 235                 240

Asp Lys Tyr Tyr Gly Thr Gly Pro Gly Asn Ala Leu Gly Pro Val Gln
    245                 250                 255

Gly Val Gly Tyr Val Asn Glu Leu Leu Ala Arg Leu Thr Gly Gln Ala
    260                 265                 270

Val Arg Asp Glu Thr Gln Thr Asn Arg Thr Leu Asp Ser Asp Pro Ala
275                 280                 285                 290

Thr Phe Pro Leu Asn Arg Thr Phe Tyr Ala Asp Phe Ser His Asp Asn
            295                 300                 305

Thr Met Val Pro Ile Phe Ala Ala Leu Gly Leu Phe Asn Ala Thr Ala
            310                 315                 320

Leu Asp Pro Leu Lys Pro Asp Glu Asn Arg Leu Trp Val Asp Ser Lys
            325                 330                 335

Leu Val Pro Phe Ser Gly His Met Thr Val Glu Lys Leu Ala Cys Ser
    340                 345                 350

Gly Lys Glu Ala Val Arg Val Leu Val Asn Asp Ala Val Gln Pro Leu
355                 360                 365                 370

Glu Phe Cys Gly Gly Val Asp Gly Val Cys Glu Leu Ser Ala Phe Val
            375                 380                 385

Glu Ser Gln Thr Tyr Ala Arg Glu Asn Gly Gln Gly Asp Phe Ala Lys
            390                 395                 400

Cys Gly Phe Val Pro Ser Glu
            405

(2) INFORMATION FOR SEQ ID NO: 25:

(i) SEQUENCE CHARACTERISTICS:

(A) LENGTH: 1522 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Paxillus involtus
(B) STRAIN: CBS 100231

(ix) FEATURE:

(A) NAME/KEY: CDS
(B) LOCATION:58..1383

(ix) FEATURE:

(A) NAME/KEY: mat_peptide
(B) LOCATION:115..1383

(ix) FEATURE:

(A) NAME/KEY: sig_peptide
(B) LOCATION:58..114

(xi) SEQUENCE DESCRIPTION: SEQ ID NO: 25:

```
GGATCCGAAT TCGGCACTCG TACGGTCCCC CGGTCTACCC TCTGCTCGCC TTGGAAG          57

ATG CTC TTC GGT TTC GTC GCC CTC GCC TGT CTC TTG TCC CTC TCC GAG         105
Met Leu Phe Gly Phe Val Ala Leu Ala Cys Leu Leu Ser Leu Ser Glu
-19           -15               -10                    -5

GTC CTT GCG ACC TCC GTG CCC AAG AAC ACA GCG CCG ACC TTC CCC ATT         153
Val Leu Ala Thr Ser Val Pro Lys Asn Thr Ala Pro Thr Phe Pro Ile
             1               5                   10

CCG GAG AGT GAG CAG CGG AAC TGG TCC CCG TAC TCG CCC TAC TTC CCT         201
Pro Glu Ser Glu Gln Arg Asn Trp Ser Pro Tyr Ser Pro Tyr Phe Pro
         15                  20                  25

CTT GCC GAG TAC AAG GCT CCT CCG GCG GGC TGC CAG ATC AAC CAG GTC         249
Leu Ala Glu Tyr Lys Ala Pro Pro Ala Gly Cys Gln Ile Asn Gln Val
 30                  35              40                      45

AAC ATC ATC CAA AGA CAT GGT GCC CGG TTC CCG ACC TCT GGC GCG ACC         297
Asn Ile Ile Gln Arg His Gly Ala Arg Phe Pro Thr Ser Gly Ala Thr
                 50                  55                  60

ACC CGT ATC AAG GCG GGT TTG ACC AAG TTG CAA GGC GTC CAG AAC TTT         345
Thr Arg Ile Lys Ala Gly Leu Thr Lys Leu Gln Gly Val Gln Asn Phe
                 65                  70                  75

ACC GAC GCC AAA TTC AAC TTC ATC AAG TCG TTC AAG TAC GAT CTC GGT         393
Thr Asp Ala Lys Phe Asn Phe Ile Lys Ser Phe Lys Tyr Asp Leu Gly
             80                  85                  90

AAC TCG GAC CTC GTT CCG TTC GGT GCA GCA CAG TCC TTC GAC GCT GGT         441
Asn Ser Asp Leu Val Pro Phe Gly Ala Ala Gln Ser Phe Asp Ala Gly
         95                  100                 105

CAG GAG GCC TTC GCC CGC TAC TCG AAG CTT GTC AGC AAG AAC AAC CTG         489
Gln Glu Ala Phe Ala Arg Tyr Ser Lys Leu Val Ser Lys Asn Asn Leu
110                 115                 120                 125

CCG TTC ATT CGT GCC GAT GGA AGT GAT CGT GTT GTG GAT TCT GCT ACA         537
Pro Phe Ile Arg Ala Asp Gly Ser Asp Arg Val Val Asp Ser Ala Thr
                 130                 135                 140

AAC TGG ACT GCG GGT TTC GCT TCG GCA AGT CAC AAC ACG GTC CAG CCC         585
Asn Trp Thr Ala Gly Phe Ala Ser Ala Ser His Asn Thr Val Gln Pro
             145                 150                 155

AAG CTG AAC CTG ATT CTC CCG CAA ACT GGC AAT GAT ACC CTG GAA GAT         633
Lys Leu Asn Leu Ile Leu Pro Gln Thr Gly Asn Asp Thr Leu Glu Asp
             160                 165                 170
```

```
AAT ATG TGC CCT GCT GCT GGC GAT TCT GAC CCC CAG GTC AAC GCG TGG      681
Asn Met Cys Pro Ala Ala Gly Asp Ser Asp Pro Gln Val Asn Ala Trp
    175                 180                 185

TTG GCT GTT GCT TTC CCT TCC ATC ACT GCA CGG CTC AAC GCC GCC GCG      729
Leu Ala Val Ala Phe Pro Ser Ile Thr Ala Arg Leu Asn Ala Ala Ala
190                 195                 200                 205

CCC TCT GTC AAC CTC ACC GAC ACG GAC GCG TTC AAC CTC GTC AGT CTC      777
Pro Ser Val Asn Leu Thr Asp Thr Asp Ala Phe Asn Leu Val Ser Leu
                210                 215                 220

TGC GCT TTC TTG ACA GTC TCG AAG GAG AAG AAG AGT GAC TTC TGC ACC      825
Cys Ala Phe Leu Thr Val Ser Lys Glu Lys Lys Ser Asp Phe Cys Thr
            225                 230                 235

CTG TTC GAG GGC ATC CCT GGC TCT TTC GAG GCG TTC GCC TAT GGT GGC      873
Leu Phe Glu Gly Ile Pro Gly Ser Phe Glu Ala Phe Ala Tyr Gly Gly
            240                 245                 250

GAC CTT GAC AAG TTC TAC GGT ACC GGT TAC GGT CAG GAA CTC GGA CCC      921
Asp Leu Asp Lys Phe Tyr Gly Thr Gly Tyr Gly Gln Glu Leu Gly Pro
            255                 260                 265

GTT CAA GGC GTC GGC TAC GTC AAC GAG CTC ATC GCC CGC CTC ACC AAC      969
Val Gln Gly Val Gly Tyr Val Asn Glu Leu Ile Ala Arg Leu Thr Asn
270                 275                 280                 285

TCC GCC GTC CGC GAC AAC ACC CAG ACG AAC CGC ACA CTC GAC GCC TCG     1017
Ser Ala Val Arg Asp Asn Thr Gln Thr Asn Arg Thr Leu Asp Ala Ser
                290                 295                 300

CCC GTA ACC TTC CCG TTG AAC AAG ACG TTC TAC GCC GAT TTC TCC CAC     1065
Pro Val Thr Phe Pro Leu Asn Lys Thr Phe Tyr Ala Asp Phe Ser His
            305                 310                 315

GAC AAC CTC ATG GTC GCC GTC TTC TCC GCC ATG GGC CTC TTC CGC CAG     1113
Asp Asn Leu Met Val Ala Val Phe Ser Ala Met Gly Leu Phe Arg Gln
            320                 325                 330

CCC GCG CCG CTC AGC ACG TCC GTG CCG AAC CCA TGG CGC ACG TGG CGC     1161
Pro Ala Pro Leu Ser Thr Ser Val Pro Asn Pro Trp Arg Thr Trp Arg
    335                 340                 345

ACG AGC TCC CTC GTC CCC TTC TCC GGA CGC ATG GTC GTG GAA CGC CTC     1209
Thr Ser Ser Leu Val Pro Phe Ser Gly Arg Met Val Val Glu Arg Leu
350                 355                 360                 365

AGC TGT TTC GGC ACG ACC AAG GTT CGC GTC CTC GTG CAG GAC CAG GTG     1257
Ser Cys Phe Gly Thr Thr Lys Val Arg Val Leu Val Gln Asp Gln Val
            370                 375                 380

CAG CCG CTC GAG TTC TGC GGG GGT GAT AGG AAC GGG CTG TGC ACG CTT     1305
Gln Pro Leu Glu Phe Cys Gly Gly Asp Arg Asn Gly Leu Cys Thr Leu
            385                 390                 395

GCT AAG TTT GTG GAG AGC CAG ACG TTT GCG AGG AGT GAT GGT GCG GGG     1353
Ala Lys Phe Val Glu Ser Gln Thr Phe Ala Arg Ser Asp Gly Ala Gly
            400                 405                 410

GAC TTT GAG AAG TGC TTC GCG ACC TCG GCG TGAGGATGGA CGAACAAAAT      1403
Asp Phe Glu Lys Cys Phe Ala Thr Ser Ala
            415                 420

TAAATTGGGG TATTTTATCG TATAATTATG GTGTGTGTAG AACATGGGCT CGGGGTCGAT   1463

GGTGAAAAGC AAAGGTTTAT CGTCTAAAAA AAAAAAAAAA AAAAAATTCC TGCGGCCGC    1522
```

(2) INFORMATION FOR SEQ ID NO: 26:

   (i) SEQUENCE CHARACTERISTICS:

(A) LENGTH: 442 amino acids
(B) TYPE: amino acid
(D) TOPOLOGY: linear

(ii) MOLECULE TYPE: protein

(xi) SEQUENCE DESCRIPTION: SEQ ID NO: 26:

```
Met Leu Phe Gly Phe Val Ala Leu Ala Cys Leu Leu Ser Leu Ser Glu
-19          -15               -10                    -5

Val Leu Ala Thr Ser Val Pro Lys Asn Thr Ala Pro Thr Phe Pro Ile
         1               5               10

Pro Glu Ser Glu Gln Arg Asn Trp Ser Pro Tyr Ser Pro Tyr Phe Pro
     15              20              25

Leu Ala Glu Tyr Lys Ala Pro Pro Ala Gly Cys Gln Ile Asn Gln Val
     30          35              40                      45

Asn Ile Ile Gln Arg His Gly Ala Arg Phe Pro Thr Ser Gly Ala Thr
             50              55                  60

Thr Arg Ile Lys Ala Gly Leu Thr Lys Leu Gln Gly Val Gln Asn Phe
             65              70                  75

Thr Asp Ala Lys Phe Asn Phe Ile Lys Ser Phe Lys Tyr Asp Leu Gly
         80              85              90

Asn Ser Asp Leu Val Pro Phe Gly Ala Ala Gln Ser Phe Asp Ala Gly
     95              100             105

Gln Glu Ala Phe Ala Arg Tyr Ser Lys Leu Val Ser Lys Asn Asn Leu
110             115             120                     125

Pro Phe Ile Arg Ala Asp Gly Ser Asp Arg Val Val Asp Ser Ala Thr
             130             135             140

Asn Trp Thr Ala Gly Phe Ala Ser Ala Ser His Asn Thr Val Gln Pro
             145             150             155

Lys Leu Asn Leu Ile Leu Pro Gln Thr Gly Asn Asp Thr Leu Glu Asp
         160             165             170

Asn Met Cys Pro Ala Ala Gly Asp Ser Asp Pro Gln Val Asn Ala Trp
     175             180             185

Leu Ala Val Ala Phe Pro Ser Ile Thr Ala Arg Leu Asn Ala Ala Ala
190             195             200                     205

Pro Ser Val Asn Leu Thr Asp Thr Asp Ala Phe Asn Leu Val Ser Leu
             210             215             220

Cys Ala Phe Leu Thr Val Ser Lys Glu Lys Lys Ser Asp Phe Cys Thr
             225             230             235

Leu Phe Glu Gly Ile Pro Gly Ser Phe Glu Ala Phe Ala Tyr Gly Gly
         240             245             250

Asp Leu Asp Lys Phe Tyr Gly Thr Gly Tyr Gly Gln Glu Leu Gly Pro
     255             260             265

Val Gln Gly Val Gly Tyr Val Asn Glu Leu Ile Ala Arg Leu Thr Asn
270             275             280                     285

Ser Ala Val Arg Asp Asn Thr Gln Thr Asn Arg Thr Leu Asp Ala Ser
             290             295             300

Pro Val Thr Phe Pro Leu Asn Lys Thr Phe Tyr Ala Asp Phe Ser His
         305             310             315

Asp Asn Leu Met Val Ala Val Phe Ser Ala Met Gly Leu Phe Arg Gln
         320             325             330

Pro Ala Pro Leu Ser Thr Ser Val Pro Asn Pro Trp Arg Thr Trp Arg
     335             340             345

Thr Ser Ser Leu Val Pro Phe Ser Gly Arg Met Val Val Glu Arg Leu
350             355             360                     365

Ser Cys Phe Gly Thr Thr Lys Val Arg Val Leu Val Gln Asp Gln Val
```

```
                                  370                    375                    380
          Gln Pro Leu Glu Phe Cys Gly Gly Asp Arg Asn Gly Leu Cys Thr Leu
                      385                    390                    395

          Ala Lys Phe Val Glu Ser Gln Thr Phe Ala Arg Ser Asp Gly Ala Gly
                  400                    405                    410

          Asp Phe Glu Lys Cys Phe Ala Thr Ser Ala
              415                    420
```

(2) INFORMATION FOR SEQ ID NO: 27:

   (i) SEQUENCE CHARACTERISTICS:

      (A) LENGTH: 1642 base pairs
      (B) TYPE: nucleic acid
      (C) STRANDEDNESS: single
      (D) TOPOLOGY: linear

   (ii) MOLECULE TYPE: cDNA

   (vi) ORIGINAL SOURCE:

      (A) ORGANISM: Paxillus involtus
      (B) STRAIN: CBS 100231

   (ix) FEATURE:

      (A) NAME/KEY: CDS
      (B) LOCATION:48..1373

   (ix) FEATURE:

      (A) NAME/KEY: mat_peptide
      (B) LOCATION:105..1373

   (ix) FEATURE:

      (A) NAME/KEY: sig_peptide
      (B) LOCATION:48..104

   (xi) SEQUENCE DESCRIPTION: SEQ ID NO: 27:

```
GGATCCGAAT TCCAGTCCCC AAGCTAATCC TCTGCTCGCC TTGGAAG ATG CAC CTC          56
                                                       Met His Leu
                                                       -19

GGC TTC GTC ACC CTC GCT TGT CTC ATA CAC CTC TCC GAG GTC TTC GCG         104
Gly Phe Val Thr Leu Ala Cys Leu Ile His Leu Ser Glu Val Phe Ala
    -15                 -10                 -5

GCA TCC GTG CCC CGG AAT ATT GCT CCG AAG TTC TCA ATT CCG GAA AGC         152
Ala Ser Val Pro Arg Asn Ile Ala Pro Lys Phe Ser Ile Pro Glu Ser
 1               5               10              15

GAG CAG CGA AAC TGG TCG CCT TAC TCT CCT TAC TTT CCC CTA GCC GAA         200
Glu Gln Arg Asn Trp Ser Pro Tyr Ser Pro Tyr Phe Pro Leu Ala Glu
            20              25              30

TAC AAG GCT CCT CCA GCA GGC TGC GAG ATT AAC CAA GTC AAT ATT ATC         248
Tyr Lys Ala Pro Pro Ala Gly Cys Glu Ile Asn Gln Val Asn Ile Ile
        35              40              45

CAA CGG CAT GGC GCA CGG TTC CCA ACC TCG GGT GCG GCC ACT CGC ATC         296
Gln Arg His Gly Ala Arg Phe Pro Thr Ser Gly Ala Ala Thr Arg Ile
    50              55              60

AAG GCT GGT TTA AGC AAG CTG CAA TCC GTC CAG AAT TTC ACC GAC CCC         344
Lys Ala Gly Leu Ser Lys Leu Gln Ser Val Gln Asn Phe Thr Asp Pro
65              70              75              80

AAA TTC GAC TTC ATC AAG TCG TTC ACA TAC GAT CTT GGT ACT TCC GAC         392
Lys Phe Asp Phe Ile Lys Ser Phe Thr Tyr Asp Leu Gly Thr Ser Asp
            85              90              95

CTC GTG CCA TTC GGC GCA GCA CAA TCA TTC GAT GCC GGC CTG GAG GTC         440
Leu Val Pro Phe Gly Ala Ala Gln Ser Phe Asp Ala Gly Leu Glu Val
            100             105             110
```

```
TTC GCT CGC TAT TCG AAG CTC GTC AGC TCG GAC AAC CTG CCT TTC ATT        488
Phe Ala Arg Tyr Ser Lys Leu Val Ser Ser Asp Asn Leu Pro Phe Ile
        115                 120                 125

CGC TCA GAT GGT AGC GAT CGT GTA GTC GAC ACT GCT ACG AAC TGG ACT        536
Arg Ser Asp Gly Ser Asp Arg Val Val Asp Thr Ala Thr Asn Trp Thr
    130                 135                 140

GCA GGT TTT GCT TCC GCG AGC CGC AAC GCG ATC CAA CCC AAG CTC GAC        584
Ala Gly Phe Ala Ser Ala Ser Arg Asn Ala Ile Gln Pro Lys Leu Asp
145                 150                 155                 160

TTG ATA CTT CCA CAA ACT GGC AAT GAC ACC CTC GAG GAC AAC ATG TGT        632
Leu Ile Leu Pro Gln Thr Gly Asn Asp Thr Leu Glu Asp Asn Met Cys
                165                 170                 175

CCA GCT GCT GGC GAA TCC GAC CCT CAG GTC GAT GCG TGG TTG GCG TCC        680
Pro Ala Ala Gly Glu Ser Asp Pro Gln Val Asp Ala Trp Leu Ala Ser
            180                 185                 190

GCC TTC CCA TCT GTC ACC GCG CAG CTC AAC GCT GCA GCG CCT GGT GCC        728
Ala Phe Pro Ser Val Thr Ala Gln Leu Asn Ala Ala Ala Pro Gly Ala
        195                 200                 205

AAT CTC ACA GAC GCC GAC GCC TTC AAC CTC GTC AGC CTG TGT CCC TTC        776
Asn Leu Thr Asp Ala Asp Ala Phe Asn Leu Val Ser Leu Cys Pro Phe
    210                 215                 220

ATG ACA GTT TCG AAG GAG CAG AAG AGC GAC TTC TGC ACG TTG TTC GAG        824
Met Thr Val Ser Lys Glu Gln Lys Ser Asp Phe Cys Thr Leu Phe Glu
225                 230                 235                 240

GGA ATC CCT GGA TCG TTC GAG GCG TTT GCC TAT GCC GGC GAC CTT GAC        872
Gly Ile Pro Gly Ser Phe Glu Ala Phe Ala Tyr Ala Gly Asp Leu Asp
                245                 250                 255

AAG TTC TAT GGG ACC GGC TAT GGC CAA GCC CTC GGA CCG GTC CAA GGC        920
Lys Phe Tyr Gly Thr Gly Tyr Gly Gln Ala Leu Gly Pro Val Gln Gly
            260                 265                 270

GTC GGC TAC ATC AAC GAG CTC CTT GCA CGC CTG ACC AAC TCC GCA GTG        968
Val Gly Tyr Ile Asn Glu Leu Leu Ala Arg Leu Thr Asn Ser Ala Val
        275                 280                 285

AAC GAC AAC ACA CAG ACG AAC CGC ACA CTC GAC GCC GCA CCA GAC ACG       1016
Asn Asp Asn Thr Gln Thr Asn Arg Thr Leu Asp Ala Ala Pro Asp Thr
    290                 295                 300

TTC CCG CTC AAC AAG ACC ATG TAC GCC GAT TTC TCA CAC GAC AAC CTC       1064
Phe Pro Leu Asn Lys Thr Met Tyr Ala Asp Phe Ser His Asp Asn Leu
305                 310                 315                 320

ATG GTC GCC GTG TTC TCC GCC ATG GGC CTC TTC CGC CAA TCC GCA CCG       1112
Met Val Ala Val Phe Ser Ala Met Gly Leu Phe Arg Gln Ser Ala Pro
                325                 330                 335

CTC AGC ACG TCC ACA CCG GAT CCG AAC CGC ACG TGG CTC ACG AGC TCT       1160
Leu Ser Thr Ser Thr Pro Asp Pro Asn Arg Thr Trp Leu Thr Ser Ser
            340                 345                 350

GTC GTT CCG TTC TCC GCG CGC ATG GCC GTC GAA CGC CTC AGC TGT GCT       1208
Val Val Pro Phe Ser Ala Arg Met Ala Val Glu Arg Leu Ser Cys Ala
        355                 360                 365

GGT ACC ACG AAG GTG CGC GTC CTG GTG CAG GAC CAG GTC CAG CCA CTC       1256
Gly Thr Thr Lys Val Arg Val Leu Val Gln Asp Gln Val Gln Pro Leu
    370                 375                 380

GAG TTC TGC GGC GGC GAC CAG GAT GGG TTG TGC GCG CTA GAC AAG TTC       1304
Glu Phe Cys Gly Gly Asp Gln Asp Gly Leu Cys Ala Leu Asp Lys Phe
385                 390                 395                 400

GTC GAG AGC CAG GCG TAT GCA CGG AGT GGT GGC GCA GGT GAC TTT GAG       1352
Val Glu Ser Gln Ala Tyr Ala Arg Ser Gly Gly Ala Gly Asp Phe Glu
                405                 410                 415
```

```
AAG TGT CTT GCG ACG ACG GTG TGAGATGGGG TAATCTACGG TGAAGCAGCG     1403
Lys Cys Leu Ala Thr Thr Val
                420

GAGAGCCTCT CAACGAATGC AAAGGATAGG TTCGAGGCTT ACTTCATCAA CCTATATCAT     1463

CATAGGACAA GCCCCCCAAT AGCCAGACTC GTCGTTTGAC ATCGTGTATG AAAATAACCC     1523

ACCCACGCAC TCCGCTGCCA CTATTCGCGT GTATCGCATA CTAGGCGTTT TCGCCCAGTT     1583

GAACATGAGC CCATTCTGTC CCCAGTGAAA AAAAAAAAAA AAAAAATTCC TGCGGCCGC      1642
```

(2) INFORMATION FOR SEQ ID NO: 28:

    (i) SEQUENCE CHARACTERISTICS:

        (A) LENGTH: 442 amino acids
        (B) TYPE: amino acid
        (D) TOPOLOGY: linear

    (ii) MOLECULE TYPE: protein

    (xi) SEQUENCE DESCRIPTION: SEQ ID NO: 28:

```
Met His Leu Gly Phe Val Thr Leu Ala Cys Leu Ile His Leu Ser Glu
-19           -15               -10                     -5

Val Phe Ala Ala Ser Val Pro Arg Asn Ile Ala Pro Lys Phe Ser Ile
             1               5               10

Pro Glu Ser Glu Gln Arg Asn Trp Ser Pro Tyr Ser Pro Tyr Phe Pro
     15               20               25

Leu Ala Glu Tyr Lys Ala Pro Pro Ala Gly Cys Glu Ile Asn Gln Val
30               35               40                       45

Asn Ile Ile Gln Arg His Gly Ala Arg Phe Pro Thr Ser Gly Ala Ala
             50               55                       60

Thr Arg Ile Lys Ala Gly Leu Ser Lys Leu Gln Ser Val Gln Asn Phe
             65               70               75

Thr Asp Pro Lys Phe Asp Phe Ile Lys Ser Phe Thr Tyr Asp Leu Gly
         80               85               90

Thr Ser Asp Leu Val Pro Phe Gly Ala Ala Gln Ser Phe Asp Ala Gly
     95               100              105

Leu Glu Val Phe Ala Arg Tyr Ser Lys Leu Val Ser Ser Asp Asn Leu
110              115              120                      125

Pro Phe Ile Arg Ser Asp Gly Ser Asp Arg Val Val Asp Thr Ala Thr
             130              135              140

Asn Trp Thr Ala Gly Phe Ala Ser Ala Ser Arg Asn Ala Ile Gln Pro
         145              150              155

Lys Leu Asp Leu Ile Leu Pro Gln Thr Gly Asn Asp Thr Leu Glu Asp
         160              165              170

Asn Met Cys Pro Ala Ala Gly Glu Ser Asp Pro Gln Val Asp Ala Trp
         175              180              185

Leu Ala Ser Ala Phe Pro Ser Val Thr Ala Gln Leu Asn Ala Ala Ala
190              195              200                      205

Pro Gly Ala Asn Leu Thr Asp Ala Asp Ala Phe Asn Leu Val Ser Leu
             210              215              220

Cys Pro Phe Met Thr Val Ser Lys Glu Gln Lys Ser Asp Phe Cys Thr
             225              230              235

Leu Phe Glu Gly Ile Pro Gly Ser Phe Glu Ala Phe Ala Tyr Ala Gly
         240              245              250

Asp Leu Asp Lys Phe Tyr Gly Thr Gly Tyr Gly Gln Ala Leu Gly Pro
```

```
                    255                    260                      265

        Val Gln Gly Val Gly Tyr Ile Asn Glu Leu Leu Ala Arg Leu Thr Asn
        270                 275                 280                 285

        Ser Ala Val Asn Asp Asn Thr Gln Thr Asn Arg Thr Leu Asp Ala Ala
                        290                 295                 300

        Pro Asp Thr Phe Pro Leu Asn Lys Thr Met Tyr Ala Asp Phe Ser His
                        305                 310                 315

        Asp Asn Leu Met Val Ala Val Phe Ser Ala Met Gly Leu Phe Arg Gln
                        320                 325                 330

        Ser Ala Pro Leu Ser Thr Ser Thr Pro Asp Pro Asn Arg Thr Trp Leu
                        335                 340                 345

        Thr Ser Ser Val Val Pro Phe Ser Ala Arg Met Ala Val Glu Arg Leu
        350                 355                 360                 365

        Ser Cys Ala Gly Thr Thr Lys Val Arg Val Leu Val Gln Asp Gln Val
                        370                 375                 380

        Gln Pro Leu Glu Phe Cys Gly Gly Asp Gln Asp Gly Leu Cys Ala Leu
                        385                 390                 395

        Asp Lys Phe Val Glu Ser Gln Ala Tyr Ala Arg Ser Gly Gly Ala Gly
                        400                 405                 410

        Asp Phe Glu Lys Cys Leu Ala Thr Thr Val
                        415                 420
```

(2) INFORMATION FOR SEQ ID NO: 29:

    (i) SEQUENCE CHARACTERISTICS:

        (A) LENGTH: 1536 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:

        (A) ORGANISM: Trametes pubescens
        (B) STRAIN: CBS 100232

    (ix) FEATURE:

        (A) NAME/KEY: CDS
        (B) LOCATION:79..1407

    (ix) FEATURE:

        (A) NAME/KEY: mat_peptide
        (B) LOCATION:130..1407

    (ix) FEATURE:

        (A) NAME/KEY: sig_peptide

    (B) LOCATION:79..129 (xi) SEQUENCE DESCRIPTION: SEQ ID NO: 29:

```
GGATCCGAAT TCGCCCCCAC ATTCGTTCCA TCTTAGCAGC CGTCCGCGCC CAGGTCTTCG        60

ATAACCCCCC GCGTGACT ATG GCC TTC TCA ATC TTG GCC TCG CTG CTC TTC        111
                     Met Ala Phe Ser Ile Leu Ala Ser Leu Leu Phe
                     -17     -15                 -10

GTG TGT TAT GCA TAC GCC AGG GCT GTG CCC CGT GCA CAT ATC CCG CTC        159
Val Cys Tyr Ala Tyr Ala Arg Ala Val Pro Arg Ala His Ile Pro Leu
    -5                  1               5                       10

CGC GAC ACC TCC GCG TGT CTA GAT GTA ACA CGC GAT GTG CAG CAG AGC        207
Arg Asp Thr Ser Ala Cys Leu Asp Val Thr Arg Asp Val Gln Gln Ser
                15              20                  25

TGG TCC ATG TAC TCT CCC TAT TTC CCG GCA GCA ACT TAT GTG GCT CCG        255
```

```
Trp Ser Met Tyr Ser Pro Tyr Phe Pro Ala Ala Thr Tyr Val Ala Pro
            30              35                  40

CCC GCG AGT TGC CAG ATC AAT CAG GTC CAC ATC ATC CAA CGT CAT GGT      303
Pro Ala Ser Cys Gln Ile Asn Gln Val His Ile Ile Gln Arg His Gly
            45              50                  55

GCA CGC TTT CCC ACG TCT GGC GCA GCA AAG CGC ATC CAG ACA GCA GTA      351
Ala Arg Phe Pro Thr Ser Gly Ala Ala Lys Arg Ile Gln Thr Ala Val
        60              65                  70

GCG AAG CTG AAG GCC GCG TCC AAC TAC ACC GAT CCC CTG CTC GCG TTC      399
Ala Lys Leu Lys Ala Ala Ser Asn Tyr Thr Asp Pro Leu Leu Ala Phe
75                  80                  85                  90

GTT ACG AAC TAC ACC TAC AGC TTA GGT CAG GAC AGC CTC GTT GAA CTC      447
Val Thr Asn Tyr Thr Tyr Ser Leu Gly Gln Asp Ser Leu Val Glu Leu
                95                  100                 105

GGT GCG ACT CAG TCC TCC GAA GCG GGC CAG GAG GCA TTC ACG CGG TAC      495
Gly Ala Thr Gln Ser Ser Glu Ala Gly Gln Glu Ala Phe Thr Arg Tyr
            110                 115                 120

TCA TCC CTC GTG AGC GCG GAC GAG CTT CCC TTC GTT CGG GCG TCG GGC      543
Ser Ser Leu Val Ser Ala Asp Glu Leu Pro Phe Val Arg Ala Ser Gly
            125                 130                 135

TCA GAT CGC GTC GTT GCG ACT GCC AAC AAC TGG ACT GCA GGT TTC GCG      591
Ser Asp Arg Val Val Ala Thr Ala Asn Asn Trp Thr Ala Gly Phe Ala
        140                 145                 150

CTT GCG AGC TCA AAC AGC ATC ACG CCC GTG CTC TCA GTC ATC ATT TCC      639
Leu Ala Ser Ser Asn Ser Ile Thr Pro Val Leu Ser Val Ile Ile Ser
155                 160                 165                 170

GAA GCG GGC AAT GAC ACC CTC GAC GAC AAC ATG TGC CCC GCT GCA GGC      687
Glu Ala Gly Asn Asp Thr Leu Asp Asp Asn Met Cys Pro Ala Ala Gly
                175                 180                 185

GAT TCG GAT CCC CAG GTC AAT CAA TGG CTC GCG CAG TTC GCA CCG CCG      735
Asp Ser Asp Pro Gln Val Asn Gln Trp Leu Ala Gln Phe Ala Pro Pro
            190                 195                 200

ATG ACT GCT CGC CTC AAC GCA GGC GCG CCC GGC GCG AAC CTC ACG GAC      783
Met Thr Ala Arg Leu Asn Ala Gly Ala Pro Gly Ala Asn Leu Thr Asp
        205                 210                 215

ACG GAC ACC TAC AAC CTG CTC ACG CTA TGC CCG TTC GAG ACT GTA GCC      831
Thr Asp Thr Tyr Asn Leu Leu Thr Leu Cys Pro Phe Glu Thr Val Ala
        220                 225                 230

ACC GAG CGG CGT AGT GAA TTC TGC GAC ATC TAC GAG GAG CTG CAG GCG      879
Thr Glu Arg Arg Ser Glu Phe Cys Asp Ile Tyr Glu Glu Leu Gln Ala
235                 240                 245                 250

GAA GAC GCC TTC GCG TAC AAT GCC GAT CTC GAC AAG TTC TAC GGC ACT      927
Glu Asp Ala Phe Ala Tyr Asn Ala Asp Leu Asp Lys Phe Tyr Gly Thr
                255                 260                 265

GGA TAC GGC CAG CCC CTC GGA CCC GTG CAA GGC GTC GGG TAC ATC AAC      975
Gly Tyr Gly Gln Pro Leu Gly Pro Val Gln Gly Val Gly Tyr Ile Asn
            270                 275                 280

GAG CTC ATC GCG CGC CTC ACC GCG CAG AAC GTG TCC GAC CAC ACG CAG      1023
Glu Leu Ile Ala Arg Leu Thr Ala Gln Asn Val Ser Asp His Thr Gln
            285                 290                 295

ACG AAC AGC ACA CTC GAC TCC TCG CCC GAG ACG TTC CCG CTC AAC CGC      1071
Thr Asn Ser Thr Leu Asp Ser Ser Pro Glu Thr Phe Pro Leu Asn Arg
        300                 305                 310

ACG CTC TAC GCG GAC TTC TCG CAC GAC AAC CAG ATG GTC GCG ATC TTC      1119
Thr Leu Tyr Ala Asp Phe Ser His Asp Asn Gln Met Val Ala Ile Phe
315                 320                 325                 330

TCG GCC ATG GGT CTC TTC AAC CAG TCC GCG CCG CTC GAC CCG ACG ACG      1167
```

```
Ser Ala Met Gly Leu Phe Asn Gln Ser Ala Pro Leu Asp Pro Thr Thr
            335                 340                 345

CCC GAC CCC GCG CGC ACG TTC CTC GTC AAG AAG ATC GTG CCG TTC TCC      1215
Pro Asp Pro Ala Arg Thr Phe Leu Val Lys Lys Ile Val Pro Phe Ser
            350                 355                 360

GCG CGC ATG GTC GTC GAG CGC CTC GAC TGC GGC GGT GCG CAG AGC GTG      1263
Ala Arg Met Val Val Glu Arg Leu Asp Cys Gly Gly Ala Gln Ser Val
            365                 370                 375

CGC CTG CTC GTG AAC GAC GCA GTG CAG CCG CTG GCG TTC TGC GGG GCG      1311
Arg Leu Leu Val Asn Asp Ala Val Gln Pro Leu Ala Phe Cys Gly Ala
        380                 385                 390

GAC ACG AGC GGG GTG TGC ACG CTG GAC GCG TTT GTC GAG AGC CAG GCG      1359
Asp Thr Ser Gly Val Cys Thr Leu Asp Ala Phe Val Glu Ser Gln Ala
395                 400                 405                 410

TAC GCG CGG AAC GAT GGC GAG GGC GAC TTC GAG AAG TGC TTC GCG ACA      1407
Tyr Ala Arg Asn Asp Gly Glu Gly Asp Phe Glu Lys Cys Phe Ala Thr
            415                 420                 425

TAGTTCCAGG TGTAGATACC CGGGGAAGAT GTACTCTCTA GACACCTCGC ATGTACTTAT    1467

CGATTAGAAA GAGACCCTGG CTGCTCTGCC CTCAAAAAAA AAAAAAAAAA AAAAAATTCC    1527

TGCGGCCGC                                                           1536
```

(2) INFORMATION FOR SEQ ID NO: 30:

    (i) SEQUENCE CHARACTERISTICS:

        (A) LENGTH: 443 amino acids
        (B) TYPE: amino acid
        (D) TOPOLOGY: linear

    (ii) MOLECULE TYPE: protein

    (xi) SEQUENCE DESCRIPTION: SEQ ID NO: 30:

```
Met Ala Phe Ser Ile Leu Ala Ser Leu Leu Phe Val Cys Tyr Ala Tyr
-17     -15             -10                     -5

Ala Arg Ala Val Pro Arg Ala His Ile Pro Leu Arg Asp Thr Ser Ala
     1           5               10                      15

Cys Leu Asp Val Thr Arg Asp Val Gln Gln Ser Trp Ser Met Tyr Ser
             20               25                  30

Pro Tyr Phe Pro Ala Ala Thr Tyr Val Ala Pro Pro Ala Ser Cys Gln
         35               40                  45

Ile Asn Gln Val His Ile Ile Gln Arg His Gly Ala Arg Phe Pro Thr
         50               55                  60

Ser Gly Ala Ala Lys Arg Ile Gln Thr Ala Val Ala Lys Leu Lys Ala
     65               70               75

Ala Ser Asn Tyr Thr Asp Pro Leu Leu Ala Phe Val Thr Asn Tyr Thr
 80               85                  90                      95

Tyr Ser Leu Gly Gln Asp Ser Leu Val Glu Leu Gly Ala Thr Gln Ser
             100              105                 110

Ser Glu Ala Gly Gln Glu Ala Phe Thr Arg Tyr Ser Ser Leu Val Ser
             115              120                 125

Ala Asp Glu Leu Pro Phe Val Arg Ala Ser Gly Ser Asp Arg Val Val
         130              135                 140

Ala Thr Ala Asn Asn Trp Thr Ala Gly Phe Ala Leu Ala Ser Ser Asn
     145              150                 155

Ser Ile Thr Pro Val Leu Ser Val Ile Ile Ser Glu Ala Gly Asn Asp
160              165                 170                     175
```

```
Thr Leu Asp Asp Asn Met Cys Pro Ala Ala Gly Asp Ser Asp Pro Gln
            180                 185                 190

Val Asn Gln Trp Leu Ala Gln Phe Ala Pro Pro Met Thr Ala Arg Leu
            195                 200                 205

Asn Ala Gly Ala Pro Gly Ala Asn Leu Thr Asp Thr Asp Thr Tyr Asn
            210                 215                 220

Leu Leu Thr Leu Cys Pro Phe Glu Thr Val Ala Thr Glu Arg Arg Ser
    225                 230                 235

Glu Phe Cys Asp Ile Tyr Glu Glu Leu Gln Ala Glu Asp Ala Phe Ala
240                 245                 250                 255

Tyr Asn Ala Asp Leu Asp Lys Phe Tyr Gly Thr Gly Tyr Gly Gln Pro
            260                 265                 270

Leu Gly Pro Val Gln Gly Val Gly Tyr Ile Asn Glu Leu Ile Ala Arg
            275                 280                 285

Leu Thr Ala Gln Asn Val Ser Asp His Thr Gln Thr Asn Ser Thr Leu
            290                 295                 300

Asp Ser Ser Pro Glu Thr Phe Pro Leu Asn Arg Thr Leu Tyr Ala Asp
    305                 310                 315

Phe Ser His Asp Asn Gln Met Val Ala Ile Phe Ser Ala Met Gly Leu
320                 325                 330                 335

Phe Asn Gln Ser Ala Pro Leu Asp Pro Thr Thr Pro Asp Pro Ala Arg
            340                 345                 350

Thr Phe Leu Val Lys Lys Ile Val Pro Phe Ser Ala Arg Met Val Val
            355                 360                 365

Glu Arg Leu Asp Cys Gly Gly Ala Gln Ser Val Arg Leu Leu Val Asn
    370                 375                 380

Asp Ala Val Gln Pro Leu Ala Phe Cys Gly Ala Asp Thr Ser Gly Val
    385                 390                 395

Cys Thr Leu Asp Ala Phe Val Glu Ser Gln Ala Tyr Ala Arg Asn Asp
400                 405                 410                 415

Gly Glu Gly Asp Phe Glu Lys Cys Phe Ala Thr
            420                 425
```

(2) INFORMATION FOR SEQ ID NO: 31:

(i) SEQUENCE CHARACTERISTICS:

(A) LENGTH: 276 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Schizophyllum sp.
(B) STRAIN: CBS 443.97

(ix) FEATURE:

(A) NAME/KEY: misc_feature

(B) LOCATION:1..276
(D) OTHER INFORMATION:/product= "PCR-fragment generated with SEQ ID NOs 15 and 17"

(xi) SEQUENCE DESCRIPTION: SEQ ID NO: 31:

```
TCTGCCGCAT CTGACGGTGT CTATAACCCC GTCCTCAACC TGATTATATC AGAAGAGCTT       60

AACGACACCC TCGATGATGC GATGTGCCCG AACGTCGGCG AATCGGACGC CCAAACGGAC      120

GAATGGACGT CTATTTACGC AGCGCCCATC GCTGAGCGTC TGAACAACAA CGCCGTGGGC      180

GCTAACCTGA CCACCACGAA CGTTTACAAC CTCATGTCTT TATGCCCCTT CGACACGCTT      240

GCGAAGGAGA CGCCGAGCCC CTTCTGCGAT CTCTTT                               276
```

(2) INFORMATION FOR SEQ ID NO: 32:

(i) SEQUENCE CHARACTERISTICS:

(A) LENGTH: 92 amino acids
(B) TYPE: amino acid
(C) STRANDEDNESS: single
(D) TOPOLOGY: linear

(ii) MOLECULE TYPE: peptide

(v) FRAGMENT TYPE: internal

(vi) ORIGINAL SOURCE:

(A) ORGANISM: Schizophyllum sp.
(B) STRAIN: CBS 443.97

(ix) FEATURE:

(A) NAME/KEY: Region
(B) LOCATION:1..92
(D) OTHER INFORMATION:/note= "the deduced amino acid sequence of SEQ ID NO: 31"

(xi) SEQUENCE DESCRIPTION: SEQ ID NO: 32:

```
Ser Ala Ala Ser Asp Gly Val Tyr Asn Pro Val Leu Asn Leu Ile Ile
1               5                   10                  15

Ser Glu Glu Leu Asn Asp Thr Leu Asp Asp Ala Met Cys Pro Asn Val
            20                  25                  30

Gly Glu Ser Asp Ala Gln Thr Asp Glu Trp Thr Ser Ile Tyr Ala Ala
        35                  40                  45

Pro Ile Ala Glu Arg Leu Asn Asn Asn Ala Val Gly Ala Asn Leu Thr
        50                  55                  60

Thr Thr Asn Val Tyr Asn Leu Met Ser Leu Cys Pro Phe Asp Thr Leu
65                  70                  75                  80

Ala Lys Glu Thr Pro Ser Pro Phe Cys Asp Leu Phe
                85                  90
```

**Description**

**FIELD OF INVENTION**

The present invention relates to isolated polypeptides having phytase activity, the corresponding cloned DNA sequences, a method of producing such polypeptides, and the use thereof for a number of industrial applications, as defined in the claims.

**BACKGROUND OF THE INVENTION**

Phytic acid or myo-inositol 1,2,3,4,5,6-hexakis dihydrogen phosphate (or for short myo-inositol hexakisphosphate) is the primary source of inositol and the primary storage form of phosphate in plant seeds. In fact, it is naturally formed during the maturation of seeds and cereal grains. In the seeds of legumes it accounts for about 70% of the phosphate content and is structurally integrated with the protein bodies as phytin, a mixed potassium, magnesium and calcium salt of inositol. Seeds, cereal grains and legumes are important components of food and feed preparations, in particular of animal feed preparations. But also in human food cereals and legumes are becoming increasingly important.

The phosphate moieties of phytic acid chelates divalent and trivalent cations such as metal ions, i.a. the nutritionally essential ions of calcium, iron, zinc and magnesium as well the trace minerals mangane, copper and molybdenum.

Besides, the phytic acid also to a certain extent binds proteins by electrostatic interaction. At a pH below the isoelectric point, pI, of the protein, the positively charged protein binds directly with phytate. At a pH above pI, the negatively charged protein binds via metal ions to phytate.

Phytic acid and its salts, phytates, are often not metabolized, since they are not absorbable from the gastro intestinal system, i.e. neither the phosphorous thereof, nor the chelated metal ions, nor the bound proteins are nutritionally available.

Accordingly, since phosphorus is an essential element for the growth of all organisms, food and feed preparations need to be supplemented with inorganic phosphate. Quite often also the nutritionally essential ions such as iron and calcium, must be supplemented. And, besides, the nutritional value of a given diet decreases, because of the binding of proteins by phytic acid. Accordingly, phytic acid is often termed an anti-nutritional factor.

Still further, since phytic acid is not metabolized, the phytate phosphorus passes through the gastrointestinal tract of such animals and is excreted with the manure, resulting in an undesirable phosphate pollution of the environment resulting e.g. in eutrophication of the water environment and extensive growth of algae.

Phytic acid or phytates, said terms being, unless otherwise indicated, in the present context used synonymously or at random, are degradable by phytases.

In most of those plant seeds which contain phytic acid, endogenous phytase enzymes are also found. These enzymes are formed during the germination of the seed and serve the purpose of liberating phosphate and, as the final product, free myo-inositol for use during the plant growth.

When ingested, the food or feed component phytates are in theory hydrolyzable by the endogenous plant phytases of the seed in question, by phytases stemming from the microbial flora in the gut and by intestinal mucosal phytases. In practice, however the hydrolyzing capability of the endogenous plant phytases and the intestinal mucosal phytases, if existing, is far from sufficient for increasing significantly the bioavailibility of the bound or constituent components of phytates. However, when the process of preparing the food or feed involve germination, fermentation or soaking, the endogenous phytase might contribute to a greater extent to the degradation of phytate.

In ruminant or polygastric animals such as horses and cows the gastro intestinal system hosts microorganisms capable of degrading phytic acid. However, this is not so in monogastric animals such as human beings, poultry and swine. Therefore, the problems indicated above are primarily of importance as regards such monogastric animals.

The production of phytases by plants as well as by microorganisms has been reported. Amongst the microorganisms, phytase producing bacteria as well as phytase producing fungi are known.

From the *plant* kingdom, e.g. a wheat-bran phytase is known (Thomlinson et al, Biochemistry, 1 (1962), 166-171). An alkaline phytase from lilly pollen has been described by Barrientos et al, Plant. Physiol., 106 (1994), 1489-1495.

Amongst the *bacteria*, phytases have been described which are derived from *Bacillus subtilis* (Paver and Jagannathan, 1982, Journal of Bacteriology 151:1102-1108) and *Pseudomonas* (Cosgrove, 1970, Australian Journal of Biological Sciences 23:1207-1220). Still further, a phytase from *E. coli* has been purified and characterized by Greiner et al, Arch. Biochem. Biophys., 303, 107-113, 1993). However, this enzyme is probably an acid phosphatase.

Phytase producing *yeasts* are also described, such as *Saccharomyces cerevisiae* (Nayini et al, 1984, Lebensmittel Wissenschaft und Technologie 17:24-26. However, this enzyme is probably a myo-inositol monophosphatase (Wodzinski et al, Adv. Appl. Microbiol., 42, 263-303). AU-A-24840/95 describes the cloning and expression of a phytase of the yeast *Schwanniomyces occidentalis*.

There are several descriptions of phytase producing *filamentous fungi*, however only belonging to the fungal phyllum of Ascomycota (*ascomycetes*). In particular, there are several references to phytase producing ascomycetes of the *Aspergillus* genus such as *Aspergillus terreus* (Yamada et al., 1986, Agric. Biol. Chem. 322:1275-1282). Also, the cloning and expression of the phytase gene from *Aspergillus niger* var. *awamori* has been described (Piddington et al., 1993, Gene 133:55-62). EP 0 420 358 describes the cloning and expression of a phytase of Aspergillus ficuum (niger). EP 0 684 313 describes the cloning and expression of phytases of the ascomycetes Myceliophthora thermophila and Aspergillus terreus.

Die Nahrung 39 (1995) 5/6 483-489 relates to bioconversion of wheat bran and shorts, by-products of the milling process, by white-rot Basidiomycete Lentinus edodes.

Mycological Research 97(6)(1993) 725-732 relates to phosphatase activity of four ectomy-corrhizal fungi found in a Sitka spruce-Japanese larch plantation in Ireland.

## NOMENCLATURE AND POSITION SPECIFICITY OF PHYTASES

In the present context a phytase is an enzyme which catalyzes the hydrolysis of phytate (*myo*-inositol hexakisphosphate) to (1) *myo*-inositol and/or (2) mono-, di-, tri-, tetra- and/or penta-phosphates thereof and (3) inorganic phosphate. In the following, for short, the above compounds are sometimes referred to as IP6, I, IP1, IP2, IP3, IP4, IP5 and P, respectively. This means that by action of a phytase, IP6 is degraded into P + one or more of the components IP5, IP4, IP3, IP2, IP1 and I. Alternatively, *myo*-inositol carrying in total n phosphate groups attached to positions p, q, r,.. is denoted $Ins(p,q,r,..)P_n$. For convenience Ins (1, 2, 3, 4, 5, 6) $P_6$ (phytic acid) is abbreviated PA.

According to the Enzyme nomenclature database ExPASy (a repository of information relative to the nomenclature of enzymes primarily based on the recommendations of the Nomenclature Committee of the International Union of Biochemistry and Molecular Biology (IUBMB) describing each type of characterized enzyme for which an EC (Enzyme Commission) number has been provided), two different types of phytases are known: A so-called 3-phytase (*myo*-inositol hexaphosphate 3-phosphohydrolase, EC 3.1.3.8) and a so-called 6-phytase (*myo*-inositol hexaphosphate 6-phosphohydrolase, EC 3.1.3.26). The 3-phytase hydrolyses first the ester bond at the 3-position, whereas the 6-phytase hydrolyzes first the ester bond at the 6-position.

### Inositolphosphate nomenclature

Considering the primary hydrolysis products of a phytase acting on phytic acid, some of the resulting esters are diastereomers and some are enantiomers. Generally, it is easier to discriminate between diastereomers, since they have different physical properties, whereas it is much more difficult to discriminate between enantiomers which are mirror images of each other.

Thus, $Ins(1,2,4,5,6)P_5$ (3-phosphate removed) and $Ins(1,2,3,4,5)P_5$ (6-phosphate removed) are diastereomers and easy to discriminate, whereas $Ins(1,2,4,5,6)P_5$ (3-phosphate removed) and Ins (2, 3, 4, 5, 6) $P_5$ (1-phosphate removed) are enantiomers. The same holds true for the pair $Ins(1,2,3,4,5)P_5$ (6-phosphate removed) and Ins (1, 2, 3, 5, 6) $P_5$ (4-phosphate removed). Accordingly, of the 6 penta-phosphate esters resulting from the first step of the phytase catalyzed hydrolysis of phytic acid, you can only discriminate easily between those esters in which the 2-, 3-, 5- and 6-phosphate has been removed, i.e. you have four diastereomers only, each of the remaining two esters being an enantiomer of one each of these compounds (4- and 6- are enantiomers, as are 1- and 3-).

### Use of lowest-locant rule

It should be noted here, that when using the notations Ins (2, 3, 4, 5, 6) $P_5$ and Ins (1, 2, 3, 5, 6) $P_5$, a relaxation of the previous recommendations on the numbering of the atoms of *myo*-inositol has been applied. This relaxation of the lowest-locant rule is recommended by the Nomenclature Committee of the International Union of Biochemistry (Biochem. J. (1989) 258, 1-2) whenever authors wish to bring out structural relationships.

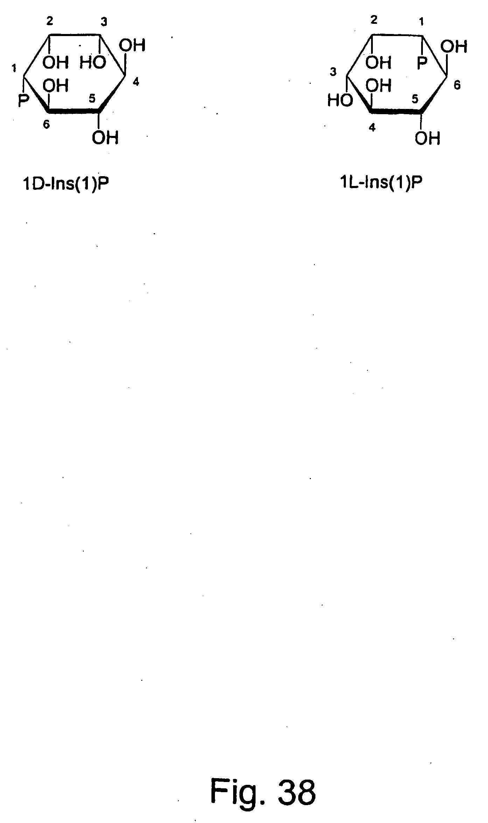

In this lowest-locant rule, the L- and D-nomenclature is recommended: Inositolphosphate, phosphate esters of myo-inositol, are generally designated 1D- (or 1L-) -$Ins(r,s,t,u,w,x)P_n$, n indicating the numer of phosphate groups and the locants r,s,t,u,w and x, their positions. The positions are numbered according to the Nomenclature Committee of the International Union of Biochemistry (NC-IUB) cited above (and the references herein), and 1D or 1L is used so as to make a substituent have the lowest possible locant or number ("lowest-locant rule"). Accordingly, 1L-myo-inositol-1-phosphate (1L-Ins(1)P, an intermediary product in the biosynthesis of inositol) and 1D-myo-inositol-1-phosphate (1DIns (1)P, a component of phospholipids), are numbered as it is apparent from Fig. 38.

Phytase specificity

As said above, phytases are divided according to their specificity in the initial hydrolysis step, viz. according to which phosphate-ester group is hydrolyzed first.

As regards the specificity of known phytases, plant phytases are generally said to be 6-phytases. However the lilly pollen phytase is said to be a 5-phytase. The microorganism derived phytases are mainly said to be 3-phytases. E.g. the ExPASy database mentioned above refers for 3-phytases to four phytases of Aspergillus awamori (strain ALK0243) and Aspergillus niger (strain NRRL 3135) (Gene 133:55-62 (1993) and Gene 127:87-94 (1993)).

Using now the D-/L-notation (in which the D- and L-configuration refer to the 1-position), the wheat-bran phytase hydrolyzes first the phosphate ester group in the L-6 position, whereas the 3-phytases hydrolyzes first the phosphate ester group in position D-3.

The specificity can be examined in several ways, e.g by HPLC or by NMR spectroscopy. These methods, however, do not immediately allow the discrimination between hydrolysis of e.g. the phosphate-ester groups in positions D-6 and L-6, since the products of the hydrolysis, $D-Ins(1,2,3,4,5)P_5$ and $L-Ins(1,2,3,4,5)P_5$, are enantiomers (mirror images), and therefore have identical NMR spectres.

In other words, in the present context a 6-phytase means either of a L-6- or a D-6-phytase or both, viz. a phytase being a L-6-phytase, a D-6-phytase or a ((D-6-)+(L-6-))-phytase (having both activities). The latter is sometimes also designated D/L-6-phytase.

## SUMMARY OF THE INVENTION

It is an object of the present invention to provide alternative phytases, in particular with superior properties such as increased heat stability or faster release of phosphate from phytate, and which can be produced in commercially useful quantities.

The present inventors have surprisingly found a whole sub-family of fungal phytases of interesting properties. This sub-family of phytases is characterized by having a high degree of conserved regions or partial sequences in common (consensus sequences). Representatives of this sub-family have turned up to be advantageous as compared to known phytases as regards various enzyme properties, such as e.g. position specificity and specific activity.

It is presently contemplated that the phytase consensus sequences of the present invention are common to all basidiomycete phytases.

In the present context a basidiomycete means a microorganism of the phyllum Basidiomycota. This phyllum of Basidiomycota is comprised in the fungal kingdom together with e.g. the phyllum Ascomycota ("ascomycetes"). Reference can be had to Jülich, 1981, Higher Taxa of Basidiomycetes; Ainsworth & Bisby, 1995, Dictionary of the Fungi; Hansen & Knudsen (Eds.), Nordic Macromycetes, vol. 2 (1992) and 3 (1997). Alternatively, a fungal taxonomy data base (NIH Data Base (Entrez)) is available via the internet on World Wide Web at the following address: http://www3.ncbi.nlm.nih.gov/Taxonomy/tax.html.

A method of screening for such phytases using PCR is also given, as are general procedures for isolating and purifying these phytase enzyme using recombinant DNA technology.

In a first aspect, an isolated polypeptide having phytase activity and being derived from the phyllum Basidiomycota is disclosed.

In a second aspect, an isolated polypeptide having phytase activity and comprising at least one of several consensus sequences is disclosed.

In a third aspect, an isolated polypeptide having phytase activity and being encoded by a DNA sequence which hybridizes under medium to high stringency with the product of a PCR reaction using a suitable set of primers derived from alignments disclosed herein and a target sequence, e.g. a DNA library is disclosed.

In a fourth aspect, an isolated polypeptide having 6-phytase activity and being derived from a fungus is dislosed.

In a fifth aspect, isolated polypeptides having phytase activity and being homologous to five specific sequences are disclosed.

In further aspects, cloned DNA sequences encoding the above polypeptides, as well as vectors and host cells comprising these cloned DNA sequences are disclosed.

The use of the phytase of the invention for liberating inorganic phosphate from phytic acid, as well as some more specific uses, and compositions, in particular food and feed preparations and additives comprising the phytase of the invention are also disclosed.

Generally, terms and expressions as used herein are to be interpreted as is usual in the art. In cases of doubt, however, the definitions of the present description might be useful.

**GENERAL DEFINITIONS**

By the expression "an isolated polypeptide/enzyme having/exhibiting phytase activity" or "an isolated phytase" is meant any peptide or protein having phytase activity (vide below) and which is essentially free of other non-phytase polypeptides, e.g., at least about 20% pure, preferably at least about 40% pure, more preferably about 60% pure, even more preferably about 80% pure, most preferably about 90% pure, and even most preferably about 95% pure, as determined by SDS-PAGE. Sometimes such polypeptide is alternatively referred to as a "purified" phytase.

The definition of "an isolated polypeptide" also include fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) of the present invention. Techniques for producing fusion polypeptides are known in the art, and include, ligating the coding sequences encoding the polypeptides so that they are in frame and that expression of the fused polypeptide is under control of the same promoter(s) and terminator.

The expression "polypeptide or enzyme exhibiting phytase activity" or "phytase" is intended to cover any enzyme capable of effecting the liberation of inorganic phosphate or phosphorous from various myo-inositol phosphates. Examples of such myo-inositol phosphates (phytase substrates) are phytic acid and any salt thereof, e.g. sodium phytate or potassium phytate or mixed salts. Also any stereoisomer of the mono-, di-, tri-, tetra- or penta-phosphates of myo-inositol might serve as a phytase substrate.

In accordance with the above definition, the phytase activity can be determined using any assay in which one of these substrates is used. In the present context (unless otherwise specified) the phytase activity is determined in the unit of FYT, one FYT being the amount of enzyme that liberates 1 $\mu$mol inorganic ortho-phosphate per min. under the following conditions: pH 5.5; temperature 37˚C; substrate: sodium phytate ($C_6H_6O_{24}P_6Na_{12}$) in a concentration of 0.0050 mol/l. Suitable phytase assays are described in the experimental part.

"Polypeptide homology" or "amino acid homology" is determined as the degree of identity between two sequences. The homology may suitably be determined by means of computer programs known in the art such as GAP provided in the GCG version 8 program package (Program Manual for the Wisconsin Package, Version 8, Genetics Computer Group, 575 Science Drive, Madison, Wisconsin, USA 53711) (Needleman, S.B. and Wunsch, C.D., (1970), Journal of Molecular Biology, 48, 443-453. Using GAP with the following settings for polypeptide sequence comparison: GAP creation penalty of 5.0 and GAP extension penalty of 0.3.

In the present context a "6-phytase" means a phytase which hydrolyzes first the 6-position in phytic acid or has a preference for these positions (plural is used since this term covers two positions). In particular, more than 50% of the hydrolysis product of the first step is Ins(1,2,3,4,5)$P_5$ and/or Ins (1, 2, 3, 5, 6) $P_5$. Preferably these two compounds comprise at least 60%, more preferably at least 70%, still more preferably at least 80%, especially at least 90% and mostly preferred more than 95% of the product of the initial hydrolysis step of PA.

The other specificity terms such as e.g. "3-phytase," "(3+6)-phytase" "6D-phytase" and "6L-phytase" are to be interpreted correspondingly, including the same preferred embodiments.

The terms "a phytase encoding part of a DNA sequence cloned into a plasmid present in a deposited E. coli strain" and "a phytase encoding part of the corresponding DNA sequence presented in the sequence listing" are presently believed to be identical, and accordingly they may be used interchangeably.

Primarily, the term "a phytase encoding part" used in connection with a DNA sequence means that region of the DNA sequence which is translated into a polypeptide sequence having phytase activity. Often this is the region between a first "ATG" start codon ("AUG" codon in mRNA) and a stop codon ("TAA", "TAG" or "TGA") first to follow.

However, the polypeptide translated as described above often comprises, in addition to a mature sequence exhibiting phytase activity, an N-terminal signal sequence and/or a pro-peptide sequence. Generally, the signal sequence guides the secretion of the polypeptide and the pro-peptide guides the folding of the polypeptide. For further information see Egnell, P. et al. Molecular Microbiol. 6(9):1115-19 (1992) or Stryer, L., "Biochemistry" W.H., Freeman and Company/New York, ISBN 0-7167-1920-7. Therefore, the term "phytase encoding part" is also intended to cover the DNA sequence corresponding to the mature part of the translated polypeptide or to each of such mature parts, if several exist.

Still further, any fragment of such sequence encoding a polypeptide fragment, which still retains some phytase activity, is to be included in this definition.

An isolated DNA molecule or, alternatively, a "cloned DNA sequence" "a DNA construct," "a DNA segment" or "an isolated DNA sequence" refers to a DNA molecule or sequence which can be cloned in accordance with standard cloning procedures used in genetic engineering to relocate the DNA segment from its natural location to a different site where it will be replicated. The term refers generally to a nucleic acid sequence which is essentially free of other nucleic acid sequences, e.g., at least about 20% pure, preferably at least about 40% pure, more preferably about 60% pure, even more preferably about 80% pure, most preferably about 90% pure, and even most preferably about 95% pure, as determined by agarose gel electrophoresis. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

The degree of identity or "homology" between two nucleic acid sequences may be determined by means of computer programs known in the art such as GAP provided in the GCG program package (Program Manual for the Wisconsin Package, Version 8, August 1996, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3.

Suitable experimental conditions for determining whether a given DNA or RNA sequence "hybridizes" to a specified nucleotide or oligonucleotide probe involves presoaking of the filter containing the DNA fragments or RNA to examine for hybridization in 5 x SSC (Sodium chloride/Sodium citrate), (J. Sambrook, E.F. Fritsch, and T. Maniatis, 1989, Molecular Cloning, A Laboratory Manual, 2d edition, Cold Spring Harbor, New York) for 10 min, and prehybridization of the filter in a solution of 5 x SSC, 5 x Denhardt's solution (Sambrook et al. 1989), 0.5 % SDS and 100 µg/ml of denatured sonicated salmon sperm DNA (Sambrook et al. 1989), followed by hybridization in the same solution containing a concentration of 10 ng/ml of a random-primed (Feinberg, A. P. and Vogelstein, B. (1983) Anal. Biochem. 132:6-13), $^{32}$P-dCTP-labeled (specific activity > 1 x $10^9$ cpm/µg) probe for 12 hours at approximately 45°C.

The filter is then washed twice for 30 minutes in 2 x SSC, 0.5 % SDS at at least 55°C (low stringency), at at least 60°C (medium stringency), at at least 65°C (medium/high stringency), at at least 70°C (high stringency), or at at least 75°C (very high stringency).

Molecules to which the oligonucleotide probe hybridizes under these conditions are detected using an x-ray film.

It has been found that it is possible to theoretically predict whether or not two given DNA sequences will hybridize under certain specified conditions.

Accordingly, as an alternative to the above described experimental method the determination whether or not an analogous DNA sequence will hybridize to the nucleotide probe described above, can be based on a theoretical calculation of the Tm (melting temperature) at which two heterologous DNA sequences with known sequences will hybridize under specified conditions (e.g. with respect to cation concentration and temperature).

In order to determine the melting temperature for heterologous DNA sequences (Tm(hetero)) it is necessary first to determine the melting temperature (Tm(homo)) for homologous DNA sequences.

The melting temperature (Tm(homo)) between two fully complementary DNA strands (homoduplex formation) may be determined by use of the following formula,

$$\text{Tm(homo)} = 81.5°\text{C} + 16.6(\log \text{M}) + 0.41(\%\text{GC}) - 0.61\ (\%\ \text{form}) - 500/\text{L}$$

("Current protocols in Molecular Biology". John Wiley and Sons, 1995), wherein

| | |
|---|---|
| "M" denotes | the molar cation concentration in wash buffer, |
| "%GC" | % Guanine (G) and Cytosine (C) of total number of bases in the DNA sequence, |
| "% form" | % formamid in the wash buffer, and |
| "L" | the length of the DNA sequence. |

The Tm determined by the above formula is the Tm of a homoduplex formation (Tm(homo)) between two fully complementary DNA sequences. In order to adapt the Tm value to that of two heterologous DNA sequences, it is assumed that a 1% difference in nucleotide sequence between the two heterologous sequences equals a 1°C decrease in Tm ("Current protocols in Molecular Biology". John Wiley and Sons, 1995). Therefore, the Tm(hetero) for the heteroduplex formation is found by subtracting the homology % difference between the analogous sequence in question and the nucleotide probe described above from the Tm(homo). The DNA homology percentage to be subtracted is calculated as described herein (*vide supra*).

The term "vector" is intended to include such terms/objects as "nucleic acid constructs," "DNA constructs," "expression vectors" or "recombinant vectors."

The nucleic acid construct comprises a nucleic acid sequence of the present invention operably linked to one or more control sequences capable of directing the expression of the coding sequence in a suitable host cell under conditions compatible with the control sequences.

"Nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature.

The term nucleic acid construct may be synonymous with the term expression cassette when the nucleic acid construct contains all the control sequences required for expression of a coding sequence of the present invention.

The term "coding sequence" as defined herein primarily comprises a sequence which is transcribed into mRNA and translated into a polypeptide of the present invention when placed under the control of the above mentioned control sequences. The boundaries of the coding sequence are generally determined by a translation start codon ATG at the 5'-terminus and a translation stop codon at the 3'-terminus. A coding sequence can include, but is not limited to, DNA, cDNA, and recombinant nucleic acid sequences.

The term "control sequences" is defined herein to include all components which are necessary or advantageous for expression of the coding sequence of the nucleic acid sequence. Each control sequence may be native or foreign to the nucleic acid sequence encoding the polypeptide. Such control sequences include, but are not limited to, a leader, a polyadenylation sequence, a propeptide sequence, a promoter, a signal sequence, and a transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide.

In the expression vector, the DNA sequence encoding the phytase should be operably connected to a suitable promoter and terminator sequence. The promoter may be any DNA sequence which shows transcriptional activity in the host cell of choice and may be derived from genes encoding proteins which are either homologous or heterologous to the host cell. The procedures used to ligate the DNA sequences coding for the phytase, the promoter and the terminator and to insert them into suitable vectors are well known to persons skilled in the art (cf. e.g. Sambrook et al., (1989), Molecular Cloning. A Laboratory Manual, Cold Spring Harbor, NY).

The recombinant expression vector may be any vector (e.g., a plasmid or virus) which can be conveniently subjected to recombinant DNA procedures and can bring about the expression of the nucleic acid sequence.

More than one copy of a nucleic acid sequence encoding a polypeptide of the present invention may be inserted into the host cell to amplify expression of the nucleic acid sequence. Stable amplification of the nucleic acid sequence can be obtained by integrating at least one additional copy of the sequence into the host cell genome using methods well known in the art and selecting for transformants.

The procedures used to ligate the elements described above to construct the recombinant expression vectors of the present invention are well known to one skilled in the art (see, *e.g.*, Sambrook *et al.,* 1989, *supra*).

A "host cell" or "recombinant host cell" encompasses any progeny of a parent cell which is not identical to the parent cell due to mutations that occur during replication.

The cell is preferably transformed with a vector comprising a nucleic acid sequence of the invention followed by integration of the vector into the host chromosome.

"Transformation" means introducing a vector comprising a nucleic acid sequence of the present invention into a host cell so that the vector is maintained as a chromosomal integrant or as a self-replicating extra-chromosomal vector. Integration is generally considered to be an advantage as the nucleic acid sequence is more likely to be stably maintained in the cell. Integration of the vector into the host chromosome may occur by homologous or non-homologous recombination as described above.

The host cell may be a unicellular microorganism, *e.g.*, a prokaryote, or a non-unicellular microorganism, e.g., a eukaryote. Examples of a eukaryote cell is a mammalian cell, an insect cell, a plant cell or a fungal cell. Useful mammalian cells include Chinese hamster ovary (CHO) cells, HeLa cells, baby hamster kidney (BHK) cells, COS cells, or any number of other immortalized cell lines available, *e.g.*, from the American Type Culture Collection.

In a preferred embodiment, the host cell is a fungal cell. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth *et al.,* 1995, *supra*, page 171) and all mitosporic fungi (Hawksworth *et al.,* 1995, *supra*).

Fungal cells may be transformed by a process involving protoplast formation, transformation of the protoplasts, and regeneration of the cell wall in a manner known *per se*.

The present invention also relates to a transgenic plant or plant part, such as a plant seed, or plant cell as claimed. Also compositions and uses of such plant or plant part are within the scope of the invention, especially its use as feed and food or additives therefore, along the lines of the present use and food/feed claims.

The transgenic plant can be dicotyledonous or monocotyledonous, for short a dicot or a monocot. Of primary interest are such plants which are potential food or feed components and which comprise phytic acid. A normal phytic acid level of feed components is 0.1-100 g/kg, or more usually 0.5-50 g/kg, most usually 0.5-20 g/kg. Examples of monocot plants are grasses, such as meadow grass (blue grass, Poa), forage grass such as festuca, lolium, temperate grass, such as Agrostis, and cereals, e.g. wheat, oats, rye, barley, rice, sorghum and maize (corn).

Examples of dicot plants are legumes, such as lupins, pea, bean and soybean, and cruciferous (family Brassicaceae), such as cauliflower, oil seed rape and the closely related model organism Arabidopsis thaliana.

Such transgenic plant etc. is capable of degrading its own phytic acid, and accordingly the need for adding such enzymes to food or feed comprising such plants is alleviated. Preferably, the plant or plant part, e.g. the seeds, are ground or milled, and possibly also soaked before being added to the food or feed or before the use, e.g. intake, thereof, with a view to adapting the speed of the enzymatic degradation to the actual use.

If desired, the plant produced enzyme can also be recovered from the plant. In certain cases the recovery from the plant is to be preferred with a view to securing a heat stable formulation in a potential subsequent pelleting process.

Examples of plant parts are stem, callus, leaves, root, fruits, seeds, tubers etc. But also any plant tissue is included in this definition.

Any plant cell, whatever the tissue origin, is included in the definition of plant cells above.

Also included within the scope of the invention are the progeny of such plants, plant parts and plant cells.

The skilled man will known how to construct a DNA expression construct for insertion into the plant in question, paying regard i.a. to whether the enzyme should be excreted in a tissue specific way. Of relevance for this evaluation is the stability (pH-stability, degradability by endogenous proteases etc.) of the phytase in the expression compartments of the plant. He will also be able to select appropriate regulatory sequences such as promoter and terminator sequences, and signal or transit sequences if required (Tague et al, Plant, Phys., 86, 506, 1988).

The plant, plant part etc. can be transformed with this DNA construct using any known method. An example of such method is the transformation by a viral or bacterial vector such as bacterial species of the genus Agrobacterium genetically engineered to comprise the gene encoding the phytase of the invention. Also methods of directly introducing the phytase DNA into the plant cell or plant tissue are known in the art, e.g. micro injection and electroporation (Gasser et al, Science, 244, 1293; Potrykus, Bio/Techn. 8, 535, 1990; Shimamoto et al, Nature, 338, 274, 1989).

Following the transformation, the transformants are screened using any method known to the skilled man, following which they are regenerated into whole plants.

These plants etc. as well as their progeny then carry the phytase encoding DNA as a part of their genetic equipment.

In general, reference is had to WO 9114782A and WO 9114772A.

Agrobacterium tumefaciens mediated gene transfer is the method of choice for generating transgenic dicots (for review Hooykas & Schilperoort, 1992. Plant Mol. Biol. 19: 15-38). Due to host range limitations it is generally not possible to transform monocots with the help of A. tumefaciens. Here, other methods have to be employed. The method of choice for generating transgenic monocots is particle bombardment (microscopic gold or tungsten particles coated with the transforming DNA) of embryonic calli or developing embryos (Christou, 1992. Plant J. 2: 275-281; Shimamoto, 1994. Curr. Opin. Biotechnol. 5: 158-162; Vasil et al., 1992. Bio/Technology 10: 667-674).

Also other systems for the delivery of free DNA into these plants, including viral vectors (Joshi & Joshi, 1991. FEBS Lett. 281: 1-8), protoplast transformation via polyethylene glycol or electroporation (for review see Potyrkus, 1991. Annu. Rev. Plant Physiol. Plant Mol. Biol. 42: 205-225), microinjection of DNA into mesophyll protoplasts (Crossway et al., 1986. Mol. Gen. Genet. 202: 79-85), and macroinjection of DNA into young floral tillers of cereal plants (de la Pena et al., 1987. Nature 325: 274-276) are preferred methods.

In general, the cDNA or gene encoding the phytase of the invention is placed in an expression cassette (e.g. Pietrzak et al., 1986. Nucleic Acids Res. 14: 5857-5868) consisting of a suitable promoter active in the target plant and a suitable terminator (termination of transcription). This cassette (of course including a suitable selection marker, see below) will be transformed into the plant as such in case of monocots via particle bombardment. In case of dicots the expression cassette is placed first into a suitable vector providing the T-DNA borders and a suitable selection marker which in turn are transformed into Agrobacterium tumefaciens. Dicots will be transformed via the Agrobacterium harbouring the expression cassette and selection marker flanked by T-DNA following standard protocols (e.g. Akama et al., 1992. Plant Cell Reports 12: 7-11). The transfer of T-DNA from Agrobacterium to the Plant cell has been recently reviewed (Zupan & Zambryski, 1995. Plant Physiol. 107: 1041-1047). Vectors for plant transformation via Agrobacterium are commercially available or can be obtained from many labs that construct such vectors (e.g. Deblaere et al., 1985. Nucleic Acids Res. 13: 4777-4788; for review see Klee et al., 1987. Annu. Rev. Plant Physiol. 38: 467-486).

Available plant promoters: Depending on the process under manipulation, organ- and/or cell-specific expression as well as appropriate developmental and environmental control may be required. For instance, it is desirable to express a phytase cDNA in maize endosperm etc. The most commonly used promoter has been the constitutive 35S-CaMV promoter Franck et al., 1980. Cell 21: 285-294). Expression will be more or less equal throughout the whole plant. This promoter has been used successfully to engineer herbicide- and pathogen-resistant plants (for review see Stitt & Sonnewald, 1995. Annu. Rev. Plant Physiol. Plant Mol. Biol. 46: 341-368). Organ-specific promoters have been reported for storage sink tissues such as seeds, potato tubers, and fruits (Edwards & Coruzzi, 1990. Annu. Rev. Genet. 24: 275-303), and for metabolic sink tissues such as meristems (Ito et al., 1994. Plant Mol. Biol. 24: 863-878).

The medium used to culture the transformed host cells may be any conventional medium suitable for growing the host cells in question. The expressed phytase may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

Preferred host cells are a strain of *Fusarium, Trichoderma* or *Aspergillus,* in particular a strain of *Fusarium graminearum*, *Fusarium venenatum, Fusarium cerealis, Fusarium sp*. having the identifying characteristic of *Fusarium* ATCC 20334, as further described in PCT/US/95/07743, *Trichoderma harzianum* or *Trichoderma reesei, Aspergillus niger* or *Aspergillus oryzae.*

## BRIEF DESCRIPTION OF THE DRAWINGS

In the detailed description of the invention below, reference is had to the drawings, of which

fig. 1      is the nucleotide sequence of the phyA cDNA and the deduced primary struture of PHYA phytase from Peniophora lycii (the signal peptide is boxed and the restriction sites used for cDNA cloning are underlined);

fig. 2      is the nucleotide sequence of a phytase from Agrocybe pediades, as in fig. 1;

fig. 3      is the nucleotide sequence of a first phytase, PHYA1, from Paxillus involtus, as in fig. 1, except for the box referring to the SignalP V1.1 prediction of the signal peptide;

fig. 4      is the nucleotide sequence of a second phytase, PHYA2, from Paxillus involtus, as in fig. 3;

fig. 5      is the nucleotide sequence of a phytase from Trametes pubescens, as in fig. 3;

fig. 6      is an alignment of the deduced amino acid sequences of the encoded phytases of figs. 1-5, identical residues in at least three of the sequences being indicated by a grey box;

fig. 7      is an alignment of the five phytases of fig. 6 together with five known phytases which all belong to the fungal phyllum of Ascomycota, identical residues in at least seven of the sequences being indicated by a grey box;

fig. 8      is a pH-activity curve of the Peniophora phytase;

fig. 9      a pH-stability curve thereof;

fig. 10      a temperature-activity curve thereof;

fig. 11      a temperature-stability curve thereof;

fig. 12      a Differential Scanning Calorimetry (DSC) curve thereof;

figs. 13-14      NMR spectra, stacked plots (up to 24h), showing the product profiling of an Aspergillus niger and the Peniophora phytase, respectively;

figs. 15-16      NMR spectra as above, but stacked plots up to 4.5h;

figs. 17-19      NMR profiles observed after 20 minutes (at pH 5.5), 24 hours (at pH 5.5) and 20 minutes (at pH 3.5), respectively;

fig. 20      curves showing concentration versus time of Ins(1,2)P2 and Ins(2)P, respectively;

fig. 21-22      curves showing the release of inorganic phosphate versus time from corn at pH 5.5 and pH 3.5, respectively;

fig. 23      is a pH-activity curve of the Agrocybe phytase;

fig. 24      a pH-stability curve thereof;

fig. 25      a temperature-activity curve thereof;

fig. 26          a temperature-stability curve thereof;

fig. 27          a Differential Scanning Calorimetry (DSC) curve thereof;

figs. 28-29      NMR spectra, stacked plots (up to 24h), showing the product profiling of an Aspergillus niger and the Agrocybe phytase, respectively;

figs. 30-31      NMR spectra as above, but stacked plots up to 4.5h;

fig. 32-33       NMR profiles observed after 20 minutes and 24 hours, respectively;

figs. 34-35      curves showing concentration versus time of Ins(1,2)P2 and Ins(2)P, respectively;

figs. 36-37      curves showing the release of inorganic phosphate versus time from corn at pH 5.5 and pH 3.5, respectively; and

fig. 38          the structure of 1D- and 1L-myo-inositol-1-phosphate (P = -OPO$_3^{-2}$).

**DEPOSITIONS**

Isolates of the strains of *Peniophora lycii, Agrocybe pediades, Paxillus involtus*, and *Trametes pubescens* from which phytases of the invention were obtained have been deposited according to the Budapest Treaty on the International Recognition of the Deposit of Microorganisms for the Purposes of Patent Procedure at the Centraalbureau voor Schimmel-cultures, P.O. Box 273, 3740 AG Baarn, The Netherlands, (CBS), as follows:

Deposit date      : 4th of December 1996
Depositor's ref.  : NN006113
CBS No.           : *Peniophora lycii* CBS No. 686.96

Deposit date      : 4th of December 1996
Depositor's ref.  : NN009289
CBS No.           : *Agrocybe pediades* CBS No. 900.96

Deposit date      : 28h of November 1997
Depositor's ref.  : NN005693
CBS No.           : *Paxillus involtus* CBS No. 100231

Deposit date      : 28h of November 1997
Depositor's ref.  : NN009343
CBS No.           : *Trametes pubescens* CBS No. 100232

Still further, the expression plasmids (shuttle vector) pYES 2.0 comprising the full length cDNA sequences encoding these phytases of the invention have been transformed into strains of *Escherichia coli* which were deposited according to the Budapest Treaty on the International Recognition of the Deposit of Microorganisms for the Purposes of Patent Procedure at the Deutsche Sammlung von Mikroorganismen und Zellkulturen GmbH., Mascheroder Weg 1b, D-38124 Braunschweig, Germany, (DSM), as follows, respectively (two phytases of *Paxillus involtus*) :

Deposit date      : 2nd of December 1996
Depositor's ref.  : NN 049282
DSM No.           : Escherichia coli DSM No. 11312

Deposit date      : 2nd of December 1996
Depositor's ref.  : NN 049283
DSM No.           : Escherichia coli DSM No. 11313

(continued)

| Deposit date | : 12th of November 1997 |
|---|---|
| Depositor's ref. | : NN 049342 |
| DSM No. | : Escherichia coli DSM No. 11842 |

| Deposit date | : 12th of November 1997 |
|---|---|
| Depositor's ref. | : NN 049343 |
| DSM No. | : Escherichia coli DSM No. 11843 |

| Deposit date | : 12th of November 1997 |
|---|---|
| Depositor's ref. | : NN 049344 |
| DSM No. | : Escherichia coli DSM No. 11844 |

## DETAILED DESCRIPTION OF THE INVENTION

The phytases of the invention derived from basidiomycetes are preferably derived from the classes *Gasteromycetes*, *Hymenomycetes, Urediniomycetes, Ustilaginomycetes* or from unclassified *Basidiomycota*, more preferably from the class *Hymenomycetes*.

The phytases derived from the class *Hymenomycetes* are preferably derived from strains of the orders *Agaricales, Aphyllophorales*, *Auriculariales, Boletales, Cantharellales, Ceratobasidiales, Dacrymycetales, Echinodontiaceae, Hericiales, Stereales, Thelephorales, Tremellales, Tulasnellales* or from the class of mitosporic *Hymenomycetes*.

Other preferred orders are *Coriolales, Hyphodermatales, Schizophyllales, Hymenochaetales* and *Phanerochaetales*.

Below, preferred families of some of these orders are listed, and examples of preferred genera within each family are added in parentheses behind each family.

Preferred families of the order *Aphyllophorales* are *Polyporaceae* (e.g. genus *Trametes, Bjerkandera, Irpex, Oxyporus, Trichaptum, Daedalea, Fomes), Coniophoraceae* (e.g. genus *Coniophora), Corticiaceae* (e.g. genus *Hyphoderma, Trechispora, Steccherinum*, *Merulius, Peniophora), Schizophyllaceae* (e.g. genus *Schizophyllum*).

Preferred families of the order *Agaricales* are *Bolbitiaceae* (e.g. genus *Agrocybe, Conocybe, Bolbitius), Coprinaceae* (e.g. genus *Coprinus, Panaeolus), Pluteaceae* (e.g. genus *Volvariella), Podaxaceae* (e.g. genus *Podaxis), Tricholomataceae* (e.g. genus *Marasmiellus, Strobilurus, Lyophyllum, Cystoderma, Merismodes), Strophariaceae* (e.g. genus *Stropharia, Hypholoma*).

A preferred family of the order *Auriculariales* is *Exidiaceae* (e.g. genus Exidia).

A preferred family of the order *Dacrymetales* is *Dacrymycetaceae* (e.g. genus *Femsjonia*).

Preferred families of the order *Stereales* are *Hyphodermataceae* (e.g. genus *Hyphodontia*) and *Stereaceae* (e.g. genus *Amylostereum* and *Stereum*).

A preferred family of the order *Boletales* is *Paxillaceae* (e.g. genus *Paxillus* and *Hygrophoropsis*).

A preferred family of the order *Thelophorales* is *Thelephoraceae* (e.g. genus *Typhula*).

Some examples of preferred strains of the above genera are *Stropharia cubensis* (in particular ATCC 13966), *Agrocybe pediades* (in particular CBS 900.96), *Bjerkandera adusta* (in particular CBS 580.95), *Trametes zonatella, Trametes pubescens* (in particular CBS 100232), *Paxillus involtus* (in particular CBS 100231), *Trametes hirsuta* (in particular DSM 2987), *Peniophora quercina, Hyphoderma argillaceum, Scizophyllum sp.* (in particular CBS 443.97), *Peniophora lycii* (in particular CBS 686.96), *Amylostereum chailletii, Oxyporus sp.* (in particular CBS 422.97). Further examples of preferred strains are listed in Example 5, Table 6.

The phytases have at least one of 14 partial amino acid sequences in common, the so-called consensus sequences, which are entered in the sequence listing as SEQ ID NOs: 1-14.

In the sequence listing, the amino acid three-letter-code is used, and some of the amino acids are denoted Xaa, which generally means "any amino acid" interpreted as below. In case of some of these Xaa-positions, however, a note is entered in the sequence listing to the effect that for instance X in position NN means any of two amino acids, cf. below.

In the claims, the amino acid one-letter-code is used in these sequences, and "X" denotes any amino acid including the non-naturally occuring ones and including any structurally or functionally similar variants thereof. Denotations like "[A/E]" mean any of the amino acids A and E. Accordingly, if in a partial sequence of a given formula two of such multiple choices exist, the number of sequences covered by the formula is $2^2$. Likewise, if there are "N" such multiple choices in a given formula, the number of sequences covered by this formula is $2^N$.

SEQ ID NOs: 1 to 9 are listed in the order of N-terminal to C-terminal end of the polypeptides. SEQ ID NOs:

10 to 14 are the amino acid sequences corresponding to the PCR probes specifically listed in Example 5 (viz. corresponding to the 522-sense and 540-anti-sense; 537-sense; 538-sense; 525-anti-sense and 539-anti-sense primers, respectively).

In preferred embodiments, the isolated polypeptide comprises at least two, three, four, five, six, seven, eight, nine, ten, eleven, twelve, thirteen or all fourteen sequences of SEQ ID Nos: 1-14, preferably at the positions indicated in the claims.

The sets of amino acid sequences reflect the PCR experiments of Examples 5 and 6 (viz. those primer-sets which are proposed).

Also some deletions seem to be characteristic for this phytase sub-family. Some regions of specific deletions are listed.

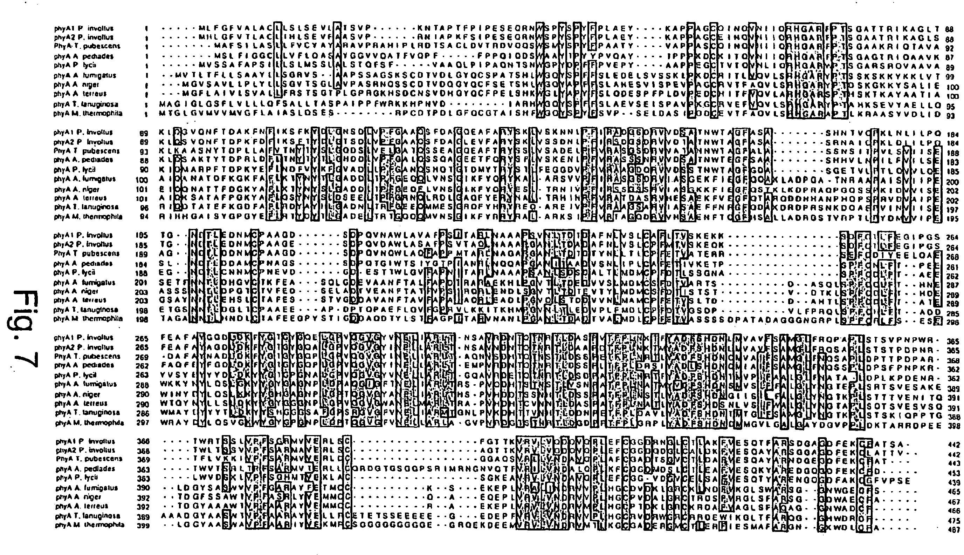

These partial sequences have been identified i.a. on the basis of the alignment shown in Fig. 7. In this alignment the five phytases at the top, viz. phyA1_P.involtus, phyA2_P.involtus, phyA_T.pubescens, phyA_A.pediades and phyA_P.lycii, are all derived from basidiomycetes and have been cloned as reported in the experimental section hereof. The five phytases listed at the bottom of the alignment are all derived from ascomycetes and their sequences are known from the prior art mentioned previously. Please refer to Example 4 hereof for further details and explanations with respect to the alignments, and please refer to Example 5 for one way of carrying out screening for phytases of this sub-family. Examples 8-18 describe the purification and characterization of five phytases of the invention, viz. the phytases of *Peniophora lycii, Agrocybe pediades, Paxillus involtus* and *Trametes pubescens.*

The conditions corresponding to the term "medium to high stringency" are found in the general definition part above, viz. the washing conditions are 2 X SSC and a temperature of 65°C. Preferably, the washing temperature is 65°C or even higher, e.g. 70, 75, 80 or even 85°C, coresponding to high stringency, very high stringency or exceptionally high stringency.

Preferably, the PCR reaction is performed with a template or a target sequence, e.g. a nucleotide sequence, which can be e.g. genomic DNA or cDNA. However, for instance mRNA can also be used as a template. Genomic DNA need not be isolated, the PCR reaction can also be conducted directly on for instance fungal mycelium.

Alternatively, the PCR reaction is performed with the wild-type gene of any PE variant thereof. In particular, at least one PCR band is obtained using at least one primer set on the wild type gene.

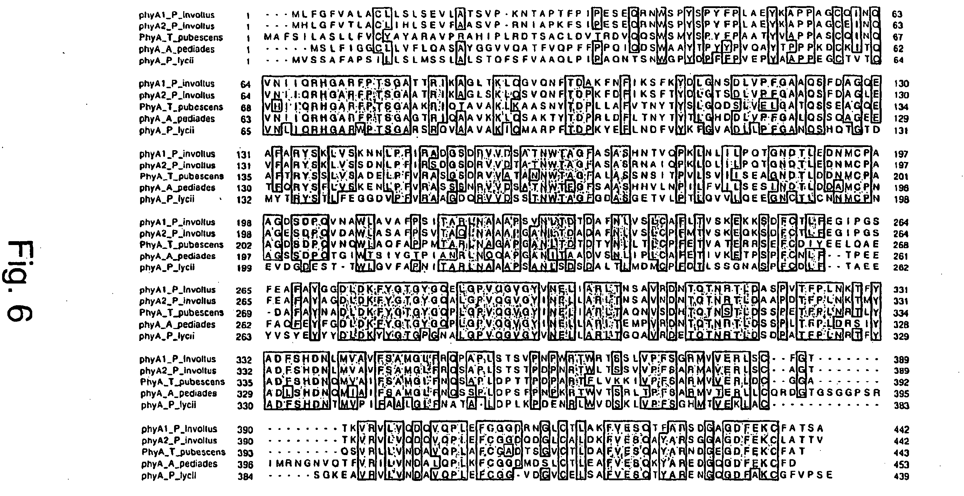

Some specific primers for the PCR reaction are suggested in the experimental part. However, the skilled man is certainly able to propose also other specific primers from the alignment at fig. 6 (basidiomycete phytases) which primers seem characteristic for basidiomycete phytases, i.e. he has to consider also the alignment at fig. 7 (showing basidiomycete phytases as well as known ascomycete phytases). Therefore, claim 6 refers in general to such primers, as the skilled man would suggest be specific for basidiomycete phytases, based on his common general knowledge and the alignments at figs. 6 and 7.

As regards 6-phytases, the invention relates to such phytases derived from any fungus. "Fungal" is defined as described above and this term includes i.a. basidiomycetes. How to interpret the specificity is explained in the general definitions part hereof. It is noted, that in the present context, the concept of "a 6-phytase" means any of a D6-, L6- or D/L-6-phytase. Surprisingly, it has turned up that such fungal 6-phytases are of a superior performance as compared to known fungal 3-phytases, reference being had to Examples 10-12 hereof revealing the Peniophora phytase as a 6-phytase and of a highly superior peformance as compared to the known Aspergillus phytases. In particular, e.g. the initial rate of hydrolysis of PA is increased and a very plausible explanation of this fact could be that this phytase is a 6-phytase, since these positions (4- and 6- in PA) are less sterically hindered than any of the other positions.

Preferably, the fungal 6-phytase is a basidiomycete phytase, still more preferably of the class, sub-class, orders, genera and strains as described at the beginning of this section headed "Detailed description of the invention." In another preferred embodiment the phytase is a D6-phytase. In another preferred embodiment the phytase is a L6-phytase.

This application also discloses e.g. the use of a fungal 6-phytase in feed and food, compositions comprising such fungal 6-phytase, in particular food and feed additives, and the use of such fungal 6-phytase for liberating inorganic phosphate from phytic acid or phytate.

In a preferred embodiment, the phytase is a (3+6)-phytase, viz. any of a D3-/D6-; L3-/L6-; D3-/L6-; L3-/D6-phytase, reference being had in particular to Examples 15-17 herein regarding the *Agrocybe* phytase which is also of superior performance as compared to the known *Aspergillus* phytase.

Preferably, the (3+6)-phytase is a basidiomycete phytase, still more preferably of the class, sub-class, orders, genera and strains as described at the beginning of this section headed "Detailed description of the invention."

Still more preferably, the (3+6)-phytase has a slight preference for one of these positions, in particular the 6-position.

The phytases of the invention are all more than 50% homologous to the isolated phytases of *Agrocybe pediades, Peniophora lycii, Paxillus involtus* (phyA1 and phyA2) and *Trametes pubescens*, respectively, corresponding to SEQ ID Nos: 22, 24, 26, 28 and 30, respectively (or the mature polypeptides thereof or any fragment thereof still retaining phytase activity). For a definition of "homologous", please refer to the section headed "General definitions." The homology to known phytases appear i.a. from Table 1 in Example 4. Preferably, the number of amino acids in the fragments referred to above is at least 50%, more preferably at least 60%, still more preferably at least 70%, even more preferably at least 80%, in particular at least 90% of the number of amino acids of the sequences listed in the sequence listing. This is so also for any polypeptide fragment disclosed herein.

Preferably, all amino acid homologies of the present application are at least 55%, or at least 60%, or at least 65%, especially at least 70%. Preferably, the degree of homology is at least 80%, more preferably at least 90%, still more preferably at least 95%, especially at least 97%.

Certain fragments of the amino acid sequence of SEQ ID NO 22 derived from *Agrocybe* are still exhibiting phytase activity. As described in more detail in the experimental part below (Examples 13-15), when expressed in yeast approximately 80% of the Agrocybe phytase enzyme has the N-terminal amino acid of Val (amino acid no. 27 in SEQ ID NO 22), whereas approximately 20% has the N-terminal amino acid of Thr (amino acid no. 25 in SEQ ID NO 22). When expressed in Aspergillus approximately 2/3 has the N-terminal amino acid of Phe (amino acid no.31 in SEQ ID NO 22), whereas approximately 1/3 has the N-terminal amino acid of Gln (amino acid no. 28 in SEQ ID NO 22). Accordingly, there are at least these four possible mature amino acid sequences.

Analogously, a fragment of the amino acid sequence of SEQ ID NO 24 derived from *Peniophora*, is also still exhibiting phytase activity. As described in more detail in the experimental part below, when expressed in Aspergillus, the Peniophora phytase has an N-terminal amino acid sequence of Leu-Pro-Ile-Pro-Ala-Gln-Asn- (amino acids no. 31-37 in SEQ ID NO 24). Accordingly the sequence of amino acids nos. 31-439 of SEQ ID No 24 is presently believed to be a mature phytase sequence.

The phytases may be defined as being encoded by a phytase encoding part of

> i) the DNA sequences listed in the sequence listing as SEQ ID Nos: 21, 23, 25, 27 and 29, respectively (phyA1 and phyA2 of *Paxillus involtus*); or
> ii) the DNA sequences cloned into plasmid pYES 2.0 present in *E. coli* DSM 11313, 11312, 11842, 11843 and 11844, respectively; or

analogues or derivatives thereof which are at least 50% homologous thereto.

For the definition of a "phytase encoding part" please refer to the general definitions section.

The five DNA sequences are obtainable directly from the deposited parent strains or from the deposited E. coli strains by extraction of plasmid DNA by methods known in the art (Sambrook et al. (1989) Molecular cloning: A laboratory manual, Cold Spring Harbor lab., Cold Spring Harbor, NY).

The DNA molecule may be defined by encoding a phytase, and being selected from:

> (a) the specific nucleotide sequences of the *Agrocybe, Peniophora, Paxillus* and *Trametes* phytases (phyA1 and phyA2 of *Paxillus*), e.g. DNA as shown in the sequence listings having the SEQ ID nos indicated (or their complementary strands);
> (b) the same sequences as under (a) but expressed via the deposited plasmid clones;
> (c) sequences which are at least 55% homologous to these sequences;
> (d) sequences hybridizing under low stringency with the sequences of (a) or (b);
> (e) sequences which do not hybridize because of the degeneracy of the genetic code but encode the specific polypeptides or phytase active fragments thereof.

For a definition of "hybridization," please refer to the section headed "General definitions," which also lists some preferred hybridization conditions.

With respect to the homology part in feature (c), the degree of homology to the nucleic acid sequence set forth under heading (a) and (b) is at least about 55%, (still encoding an polypeptide exhibiting phytase activity). In particular, the homology is at least 60%, or at least 65%, especially at least 70%, preferably at least about 80%, more preferably at least about 90%, even more preferably at least about 95%, and most preferably at least about 97%. In particular, the degree of homology is based on a comparison with the entire sequences listed or their complementary strand or any of the sub-sequences thereof corresponding to a "mature" phytase.

The homology of the DNA of selected phytases of the invention to known phytases appear i.a. from Table 1 in Example 4.

For the nucleotides corresponding preferred embodiments apply as for the polypeptides (reference to Examples 5, 6 and 7; "medium to high stringency" means the washing conditions are 2 X SSC and a temperature of 65°C, preferably 65°C or even higher, e.g. 70, 75, 80 or even 85°C; the PCR reaction is performed with a target nucleotide sequence, e.g. DNA, for instance genomic DNA or cDNA (or mRNA); wild-type gene of any PE variant thereof).

The DNA sequences of the invention can also be cloned by any general method involving

- cloning, in suitable vectors, a cDNA library from any organism expected to produce the phytase of interest,
- transforming suitable yeast host cells with said vectors,
- culturing the host cells under suitable conditions to express any enzyme of interest encoded by a clone in the cDNA library,
- screening for positive clones by determining any phytase activity of the enzyme produced by such clones, and
- isolating the enzyme encoding DNA from such clones.

A general isolation method has been disclosed in WO 93/11249 and WO 94/14953. A detailed description of the screening methods is given in the experimental part.

A primer, probe, oligonucleotide molecule/DNA molecule can be derived from the alignment of fig. 6. Only primers specific or unique for the novel phytases of the invention are of course included herein, viz. one has to consider also the alignment at fig. 7.

A method of identifying further phytase producing cells, in particular microorganisms, is disclosed. In particular, this is also a method of selecting or screening for phytase producing microorganisms. The concept of "cell" is here generally to be defined as the term "host cell" in the general definitions part hereof). Preferred cells are microorganism cells, in particular fungal cells, preferably of the phyllum Basidiomycota. More preferred basidiomycete cells are listed in the very beginning of the detailed description of the invention.

Any source, in particular any microorganism, can be selected to provide a template or a target sequence, usually in the form of genomic DNA, cDNA or mRNA.

General references to the PCR reaction, including standard reaction conditions, are found in the experimental part. As regards the selection of suitable primers, reference is had to the remarks above.

Preferably, it has to be verified that the amplified PCR fragment derived from the template is specific.

Some examples of suitable verification procedures are:

a) running an electrophoresis in agarose gels revealing the existence of a PCR band corresponding to the amplified PCR fragment; and, if desired, also
b) controlling that the size of the amplified PCR-fragment is as expected; and, if desired, also
c) isolating and sequencing the PCR fragment or band to show a high degree of homology to the parent sequences, from which the primers were derived.

Ad b): The potential presence of introns, (an example: 50 bases out of 300), is one of several reasons for allowing a deviation from exact size match. Preferably, the size of the amplified PCR-fragment (including introns) as measured for instance by the number of bases is within the ranges of 50-150%, 60-140%, 70-130%, 80-120%, 85-115%, 90-110%, 95-105% of the number of bases/nucleotides inbetween the primers in the parent sequences (fig. 6), from which the primers were derived. In another preferred embodiment (excluding introns), the size of the amplified PCR-fragment is within the range of 80-120%, 85-115%, 90-110%, 95-105% of the number of bases inbetween the primers in the parent sequences (fig. 6), from which the primers were derived.

Ad c): Preferably the degree of homology is more than 50, 60, 70, 75, 80, 85, 90, 95% homology (determined as described above). Alternatively, it is checked if the amplified PCR-fragment comprises at least one of the conserved regions inbetween the primers used, said conserved regions being shown in grey shading at fig. 6. Preferably, the fragment comprises at least two, three, four, five etc. or all of such conserved regions. Another way of checking homology is by checking presence of areas of deletions characteristic for the parent sequences of fig. 6. A further way of checking homology is checking characteristic distances between conserved regions.

Finally, the invention also relates generally to the use of the polypeptide according to the invention for liberating (or catalyzing the liberation of) phosphorous from any phytase substrate, in particular inorganic phosphate from phytate or phytic acid; alternatively for converting phytate to inorganic phosphate and (myo-inositol and/or mono-, di-, tri-, tetra-, penta-phosphate esters thereof). This encompasses any process wherein the phytase of the invention excerts its phytase activity as previously defined.

More specific uses according to the invention are in human food or animal feed preparations or in additives for such preparations, wherein the phytase i.a. serves the purposes of

(i) reducing the phytate level of manure,
(ii) improving the digestibility, promoting the growth, or improving the food and feed utilization or its conversion efficiency, i.a. by making available proteins, which would otherwise have been bound by phytate,
(iii) preventing malnutrition or diseases such as anemia caused by essential ions and phosphate lacking, i.e. improving the bioavailibility of minerals or increasing the absorption thereof, eliminating the need for adding supplemental phosphate and ions etc.

In particular, the phytases of the invention can also be used in chicken food to improve egg shell quality (reduction of losses due to breaking), cf. e.g. The Merck Veterinary Manual, (Seventh edition, Merck & CO., Inc., Rahway, N.J., U.S.A., 1991; p.1268); Jeroch et al; Bodenkultur Vol. 45, No. 4 pp. 361-368 (1994); Poultry Science, Vol. 75, No. 1 pp. 62-68 (1996); Canadian Journal of Animal Science Vol. 75 , No. 3 pp. 439-444 (1995 ); Poultry Science Vol. 74 , No. 5 pp. 784-787 (1995) and Poultry Science Vol. 73 , No. 10 pp. 1590-1596 (1994).

A "feed" and a "food," respectively, means any natural or artificial diet, meal or the like or components of such meals intended or suitable for being eaten, taken in, digested, by an animal and a human being, respectively.

The phytase may exert its effect *in vitro* or *in vivo*, i.e. before intake or in the stomach of the individual, respectively. Also a combined action is possible.

A phytase composition according to the invention always comprises at least one phytase of the invention.

Generally, phytase compositions are liquid or dry.

Liquid compositions need not contain anything more than the phytase enzyme, preferably in a highly purified form. Usually, however, a stabilizer such as glycerol, sorbitol or mono propylen glycol is also added. The liquid composition may also comprise other additives, such as salts, sugars, preservatives, pH-adjusting agents, proteins, phytate (a phytase substrate). Typical liquid compositions are aqueous or oil-based slurries. The liquid compositions can be added to a food or feed after an optional pelleting thereof.

Dry compositions may be spraydried compositions, in which case the composition need not contain anything more than the enzyme in a dry form. Usually, however, dry compositions are so-called granulates which may readily be mixed with e.g. food or feed components, or more preferably, form a component of a premix. The particle size of the enzyme granulates preferably is compatible with that of the other components of the mixture. This provides a safe and convenient mean of incorporating enzymes into e.g. animal feed.

Agglomeration granulates are prepared using agglomeration technique in a high shear mixer (e.g. Lödige) during which a filler material and the enzyme are co-agglomerated to form granules. Absorption granulates are prepared by having cores of a carrier material to absorp/be coated by the enzyme.

Typical filler materials are salts such as disodium sulphate. Other fillers are kaolin, talc, magnesium aluminium silicate and cellulose fibres. Optionally, binders such as dextrins are also included in agglomeration granulates.

Typical carrier materials are starch, e.g. in the form of cassava, corn, potato, rice and wheat. Salts may also be used.

Optionally, the granulates are coated with a coating mixture. Such mixture comprises coating agents, preferably hydrophobic coating agents, such as hydrogenated palm oil and beef tallow, and if desired other additives, such as calcium carbonate or kaolin.

Additionally, phytase compositions may contain other substituents such as colouring agents, aroma compounds, stabilizers, vitamins, minerals, other feed or food enhancing enzymes etc. This is so in particular for the so-called premixes.

A "food or feed additive" is an essentially pure compound or a multi component composition intended for or suitable for being added to food or feed. In particular it is a substance which by its intended use is becoming a component of a food or feed product or affects any characteristics of a food or feed product. It is composed as indicated for phytase compositions above. A typical additive usually comprises one or more compounds such as vitamins, minerals or feed enhancing enzymes and suitable carriers and/or excipients.

In a preferred embodiment, the phytase compositions of the invention additionally comprises an effective amount of one or more feed enhancing enzymes, in particular feed enhancing enzymes selected from the group consisting of α-galactosidases, β-galactosidases, in particular lactases, other phytases, β-glucanases, in particular endo-β-1,4-glucanases and endo-β-1,3(4)-glucanases, cellulases, xylosidases, galactanases, in particular arabinogalactan endo-1,4-β-galactosidases and arabinogalactan endo-1,3-β-galactosidases, endoglucanases, in particular endo-1,2-β-glucanase, endo-1,3-α-glucanase, and endo-1,3-β-glucanase, pectin degrading enzymes, in particular pectinases, pectinesterases, pectin lyases, polygalacturonases, arabinanases, rhamnogalacturonases, rhamnogalacturonan acetyl esterases, rhamnogalacturonan-α-rhamnosidase, pectate lyases, and α-galacturonisidases, mannanases, β-mannosidases, mannan acetyl esterases, xylan acetyl esterases, proteases, xylanases, arabinoxylanases and lipolytic enzymes such as lipases, phospholipases and cutinases.

The animal feed additive of the invention is supplemented to the mono-gastric animal before or simultaneously with the diet. Preferably, the animal feed additive of the invention is supplemented to the mono-gastric animal simultaneously with the diet. In a more preferred embodiment, the animal feed additive is added to the diet in the form of a granulate or a stabilized liquid.

An effective amount of phytase in food or feed is from about 10-20.000; preferably from about 10 to 15.000, more preferably from about 10 to 10.000, in particular from about 100 to 5.000, especially from about 100 to about 2.000 FYT/kg feed or food.

Examples of other specific uses of the phytase of the invention is in soy processing and in the manufacture of inositol or derivatives thereof.

The invention also relates to a method for reducing phytate levels in animal manure, wherein the animal is fed a feed comprising an effective amount of the phytase of the invention. As stated in the beginning of the present application one important effect thereof is to reduce the phosphate pollution of the environment.

Also within the scope of the invention is the use of a phytase of the invention during the preparation of food or feed preparations or additives, i.e. the phytase excerts its phytase activity during the manufacture only and is not active in the final food or feed product. This aspect is relevant for instance in dough making and baking.

The invention also relates to substantially pure biological cultures of the deposited microorganisms and to strains comprising, as a part of their genetic equipment, a DNA sequence encoding a phytase of the invention. Included within the definition of a substantially pure biological culture is any mutant of said strains having retained the phytase encoding capability.

The invention is described in further detail in the following examples which are not in any way intended to limit the scope of the invention.

**EXAMPLES**

Materials and Methods

Media:

*Phytate replication plates:*

> Add to 200ml of melted SC agar
> 20ml 20% galactose
> 800$\mu$l 5% threonine
> 25ml solution A
> 25ml solution B
> 200$\mu$l Trace element solution (DSM Catalogue 141)

*Solution A:*

> 6g $CaCl_2$, $2H_2O$
> 8g $MgCl_2$, $6H_2O$
> add $ddH_2O$ to 11
> pH = 6.5

*Solution B*:

> 35.12g Na-phytate
> add $H_2O$ to 11
> pH = 6.5

*Medium A:*

| | |
|---|---|
| Yeast Nitrogen Base w/o Amino acids (Difco0919) | 7.5 g/l |
| Succinic acid (Merck 822260) | 11.3 g/l |
| NaOH (Merck 6498) | 6.8 g/l |
| Casamino acid w/o vitamin (Difco 0288) | 5.6 g/l |
| tryptophan (Merck 8374) | 0.1 g/l |
| Threonine | 1.0 g/l |
| Na-phytate (35.12 g/l pH 6.5) | 125 ml/l |

(continued)

| | |
|---|---|
| Galactose | 20.0 g/l |
| Trace metal (DSM 141) | 1.0 ml/l |

*Trace metal solution:*

| | |
|---|---|
| Nitrilotriacetic acid | 1.50 g |
| $MgSO_4$, 7 $H_2O$ | 3.00 g |
| $MnSO_4.2H_2O$ | 0.50 g |
| NaCl | 1.00 g |
| $FeSO_4$, $7H_2O$ | 0.10 g |
| $CoSO_4.7H_2O$ | 0.18 g |
| $CaCl_2$, $2H_2O$ | 0.10 g |
| $ZnSO_4$, $7H_2O$ | 0.18 g |
| $CuSO_4$, $5H_2O$ | 0.01 g |
| $KAl(SO_4)_2$, $12H_2O$ | 0.02 g |
| $H_3BO_3$ | 0.01 g |
| $Na_2MoO_4$, $2H_2O$ | 0.01 g |
| $NiCl_2$, $6H_2O$ | 0.025 g |
| $Na_2Se_3O$, $5H_2O$ | 0.30 g |
| Distilled water | 1 l |
| pH 7.0 | |

First dissolve nitrilotriacetic acid and adjust pH to 6.5 with KOH, then add minerals. Final pH 7.0 (with KOH).

*Medium B*:

Similar to medium A except for glucose is added as a C-source instead of galactose.

*YPD*:

10 g yeast extract, 20 g peptone, $H_2O$ to 900 ml. Autoclaved, 100 ml 20% glucose (sterile filtered) added.

*YPM:*

10 g yeast extract, 20 g peptone, $H_2O$ to 900 ml. Autoclaved, 100 ml 20% maltodextrin (sterile filtered) added.

*10 x Basal salt:*

75 g yeast nitrogen base, 113 g succinic acid, 68 g NaOH, $H_2O$ ad 1000 ml, sterile filtered.

*SC-URA*:

100 ml 10 x Basal salt, 28 ml 20% casamino acids without vitamins, 10 ml 1% tryptophan, $H_2O$ ad 900 ml, autoclaved, 3.6 ml 5% threonine and 100 ml 20% glucose or 20% galactose added.

*SC-agar*:

SC-URA, 20 g/l agar added.

*SC-variant agar:*

20 g agar, 20 ml 10 x Basal salt, $H_2O$ ad 900 ml, autoclaved

Phytase activity assay

The phytase activity can be measured using the following assay:

10 µl diluted enzyme samples (diluted in 0.1 M sodium acetate, 0.01 % Tween20, pH 5.5) were added into 250 µl 5 mM sodium phytate (Sigma) in 0.1 M sodium acetate, 0.01 % Tween20, pH 5.5 (pH adjusted after dissolving the sodium phytate; the substrate was preheated) and incubated for 30 minutes at 37°C. The reaction was stopped by adding 250 µl 10 % TCA and free phosphate was measured by adding 500 µl 7.3 g $FeSO_4$ in 100 ml molybdate reagent (2.5 g $(NH_4)_6Mo_7O_{24}.4H_2O$ in 8 ml $H_2SO_4$ diluted to 250 ml). The absorbance at 750 nm was measured on 200 µl samples in 96 well microtiter plates. Substrate and enzyme blanks were included. A phosphate standard curve was also included (0-2 mM phosphate). 1 FYT equals the amount of enzyme that releases 1 µmol phosphate/min at the given conditions.

General molecular biology methods

Unless otherwise mentioned the DNA manipulations and transformations were performed using standard methods of molecular biology (Sambrook et al. (1989) Molecular cloning: A laboratory manual, Cold Spring Harbor lab., Cold Spring Harbor, NY; Ausubel, F. M. et al. (eds.) "Current protocols in Molecular Biology". John Wiley and Sons, 1995; Harwood, C. R., and Cutting, S. M. (eds.) "Molecular Biological Methods for Bacillus". John Wiley and Sons, 1990).

Unless otherwise specified all enzymes for DNA manipulations, such as e.g. restriction endonucleases, ligases etc., were obtained from New England Biolabs, Inc. The enzymes were used according to the specifications of the suppliers.

**EXAMPLE 1**

**Cloning and expression of a phytase from *Peniophora lycii* CBS No. 686.96**

Deposited organisms:

*Peniophora lycii* CBS No. 686.96 comprises a phytase encoding DNA sequence of the invention.

*Escherichia coli* DSM NO 11312 contains the plasmid comprising the full length cDNA sequence, coding for a phytase of the invention, in the shuttle vector pYES 2.0.

Other strains:

Yeast strain: The *Saccharomyces cerevisiae* strain used was W3124 (van den Hazel, H.B; Kielland-Brandt, M.C.; Winther, J.R. in Eur. J. Biochem., 207, 277-283, 1992; (MATa; ura 3-52; leu 2-3, 112; his 3-D200; pep 4-1137; prc1::HIS3; prb1:: LEU2; cir+).

*E. coli* strain: DH10B (Life Technologies)

Plasmids:

The *Aspergillus* expression vector pHD414 is a derivative of the plasmid p775 (described in EP 238 023). The construction of pHD414 is further described in WO 93/11249.

pYES 2.0 (Invitrogen)

pA2phy2 (See example 1)

Expression cloning in yeast

Expression cloning in yeast was done as described by H. Dalboege et al. (H. Dalboege et al Mol. Gen. Genet (1994) 243:253-260.; WO 93/11249; WO 94/14953). All individual steps of Extraction of total RNA, cDNA synthesis, Mung bean nuclease treatment, Blunt-ending with T4 DNA polymerase, and Construction of libraries was done according to the references mentioned above.

Fermentation procedure of *Peniophora lycii* CBS No. 686.96 for mRNA isolation:

*Peniophora lycii* CBS 686.96 was inoculated from a plate with outgrown mycelium into a shake flask containing 100 ml medium B (soya 30 g/l, malto dextrin 15 g/l, bacto peptone 5 g/l, pluronic 0.2 g/l). The culture was incubated stationary at 26°C for 15 days. The resulting culture broth was filtered through miracloth and the mycelium was frozen down in liquid nitrogen.

mRNA was isolated from mycelium from this culture as described in (H. Dalboege et al Mol. Gen. Genet (1994) 243:253-260.; WO 93/11249; WO 94/14953).

Extraction of total RNA was performed with guanidinium thiocyanate followed by ultracentrifugation through a 5.7 M CsCl cushion, and isolation of poly(A)$^+$RNA was carried out by oligo(dT)-cellulose affinity chromatography using the procedures described in WO 94/14953.

cDNA synthesis:

Double-stranded cDNA was synthesized from 5 mg poly(A)$^+$ RNA by the RNase H method (Gubler and Hoffman (1983) Gene 25:263-269, Sambrook et al. (1989) Molecular cloning: A laboratory manual, Cold Spring Harbor lab., Cold Spring Harbor, NY). The poly(A)$^+$ RNA (5 $\mu$g in 5 $\mu$l of DEPC-treated water) was heated at 70°C for 8 min. in a pre-siliconized, RNase-free Eppendorph tube, quenched on ice and combined in a final volume of 50 $\mu$l with reverse transcriptase buffer (50 mM Tris-Cl, pH 8.3, 75 mM KCl, 3 mM MgCl$_2$, 10 mM DTT, Bethesda Research Laboratories) containing 1 mM of dATP, dGTP and dTTP and 0.5 mM 5-methyl-dCTP (Pharmacia), 40 units human placental ribonuclease inhibitor (RNasin, Promega), 1.45 $\mu$g of oligo(dT)$_{18}$-Not I primer (Pharmacia) and 1000 units SuperScript II RNase H reverse transcriptase (Bethesda Research Laboratories). First-strand cDNA was synthesized by incubating the reaction mixture at 45°C for 1 hour. After synthesis, the mRNA:cDNA hybrid mixture was gelfiltrated through a MicroSpin S-400 HR (Pharmacia) spin column according to the manufacturer's instructions.

After the gelfiltration, the hybrids were diluted in 250 $\mu$l second strand buffer (20 mM Tris-Cl, pH 7.4, 90 mM KCl, 4.6 mM MgCl$_2$, 10 mM (NH$_4$)$_2$SO$_4$, 0.16 mM bNAD+) containing 200 $\mu$l of each dNTP, 60 units *E. coli* DNA polymerase I (Pharmacia), 5.25 units RNase H *(*Promega) and 15 units *E. coli* DNA ligase (Boehringer Mannheim). Second strand cDNA synthesis was performed by incubating the reaction tube at 16°C for 2 hours and additional 15 min. at 25°C. The reaction was stopped by addition of EDTA to a final concentration of 20 mM followed by phenol and chloroform extractions.

Mung bean nuclease treatment:

The double-stranded cDNA was precipitated at -20°C for 12 hours by addition of 2 vols 96% EtOH, 0.2 vol 10 M NH$_4$Ac, recovered by centrifugation, washed in 70% EtOH, dried and resuspended in 30 $\mu$l Mung bean nuclease buffer (30 mM NaAc, pH 4.6, 300 mM NaCl, 1 mM ZnSO$_4$, 0.35 mM DTT, 2% glycerol) containing 25 units Mung bean nuclease (Pharmacia). The single-stranded hair-pin DNA was clipped by incubating the reaction at 30°C for 30 min., followed by addition of 70 $\mu$l 10 mM Tris-Cl, pH 7.5, 1 mM EDTA, phenol extraction and precipitation with 2 vols of 96% EtOH and 0.1 vol 3 M NaAc, pH 5.2 on ice for 30 min.

Blunt-ending with T4 DNA polymerase:

The double-stranded cDNAs were recovered by centrifugation and blunt-ended in 30 ml T4 DNA polymerase buffer (20 mM Tris-acetate, pH 7.9, 10 mM MgAc, 50 mM KAc, 1 mM DTT) containing 0.5 mM of each dNTP and 5 units T4 DNA polymerase (New England Biolabs) by incubating the reaction mixture at 16°C for 1 hour. The reaction was stopped by addition of EDTA to a final concentration of 20 mM, followed by phenol and chloroform extractions, and precipitation for 12 hours at -20°C by adding 2 vols 96% EtOH and 0.1 vol 3 M NaAc pH 5.2.

Adaptor ligation, Not I digestion and size selection:

After the fill-in reaction the cDNAs were recovered by centrifugation, washed in 70% EtOH and dried. The cDNA pellet was resuspended in 25 $\mu$l ligation buffer (30 mM Tris-Cl, pH 7.8, 10 mM MgCl$_2$, 10 mM DTT, 0.5 mM ATP) containing 2.5 $\mu$g non-palindromic BstXI adaptors (Invitrogen) and 30 units T4 ligase (Promega) and incubated at 16°C for 12 hours. The reaction was stopped by heating at 65°C for 20 min. and then cooling on ice for 5 min. The adapted cDNA was digested with Not I restriction enzyme by addition of 20 $\mu$l water, 5 $\mu$l 10x Not I restriction enzyme buffer (New England Biolabs) and 50 units Not I (New England Biolabs), followed by incubation for 2.5 hours at 37°C. The reaction was stopped by heating at 65°C for 10 min. The cDNAs were size-fractionated by gel electrophoresis on a 0.8% SeaPlaque GTG low melting temperature agarose gel (FMC) in 1x TBE to separate unligated adaptors and small cDNAs. The cDNA was size-selected with a cut-off at 0.7 kb and rescued from the gel by use of b-Agarase (New England Biolabs) according to the manufacturer's instructions and precipitated for 12 hours at -20°C by adding 2 vols 96% EtOH and 0.1 vol 3 M NaAc pH 5.2.

Construction of libraries:

The directional, size-selected cDNA was recovered by centrifugation, washed in 70% EtOH, dried and resuspended in 30 $\mu$l 10 mM Tris-Cl, pH 7.5, 1 mM EDTA. The cDNAs were desalted by gelfiltration through a MicroSpin S-300 HR (Pharmacia) spin column according to the manufacturer's instructions. Three test ligations were carried out in 10 $\mu$l ligation buffer (30 mM Tris-Cl, pH 7.8, 10 mM MgCl$_2$, 10 mM DTT, 0.5 mM ATP) containing 5 $\mu$l double-stranded cDNA (reaction tubes #1 and #2), 15 units T4 ligase (Promega) and 30 ng (tube #1), 40 ng (tube #2) and 40 ng (tube #3, the vector background control) of BstXI-NotI cleaved pYES 2.0 vector. The ligation reactions were performed by incubation at 16°C for 12 hours, heating at 70°C for 20 min. and addition of 10 $\mu$l water to each tube. 1 $\mu$l of each ligation mixture was electroporated into 40 $\mu$l electrocompetent *E. coli* DH10B cells (Bethesda research Laboratories) as described (Sambrook et al. (1989) Molecular cloning: A laboratory manual, Cold Spring Harbor lab., Cold Spring Harbor, NY). Using the optimal conditions a library was established in E. *coli* consisting of pools. Each pool was made by spreading transformed E. *coli* on LB+ampicillin agar plates giving 15.000-30.000 colonies/plate after incubation at 37°C for 24 hours. 20 ml LB+ampicillin was added to the plate and the cells were suspended herein. The cell suspension was shaked in a 50 ml tube for 1 hour at 37°C. Plasmid DNA was isolated from the cells according to the manufacturer's instructions using QIAGEN plasmid kit and stored at -20°C.

1 $\mu$l aliquots of purified plasmid DNA (100 ng/ml) from individual pools were transformed into *S. cerevisiae* W3124 by electroporation (Becker and Guarante (1991) Methods Enzymol. 194:182-187) and the transformants were plated on SC agar containing 2% glucose and incubated at 30°C.

Identification of positive colonies:

After 3-5 days of growth, the agar plates were replica plated onto a set of the phytate replication plates, and incubated for 3-5 days at 30°C. 1% LSB-agarose (Sigma) containing 0.2M CaCl2 is poured over the plates and after 1-4 days the phytase positive colonies are identified as colonies surrounded by a clearing zone.

Cells from enzyme-positive colonies were spread for single colony isolation on agar, and an enzyme-producing single colony was selected for each of the phytase-producing colonies identified.

Isolation of a cDNA gene for expression in *Aspergillus*:

A phytase-producing yeast colony was inoculated into 20 ml YPD broth in a 50 ml glass test tube. The tube was shaken for 2 days at 30°C. The cells were harvested by centrifugation for 10 min. at 3000 rpm.

DNA was isolated according to WO 94/14953 and dissolved in 50 ml water. The DNA was transformed into *E. coli* by standard procedures. Plasmid DNA was isolated from *E. coli* using standard procedures, and analyzed by restriction enzyme analysis.

The cDNA insert was excised using the restriction enzymes Hind III and Xba I and ligated into the Aspergillus expression vector pHD414 resulting in the plasmid pA2phy2.

The cDNA inset of Qiagen purified plasmid DNA of pA2phy2 (Qiagen, USA) was sequenced with the Taq deoxy terminal cycle sequencing kit (Perkin Elmer, USA) and synthetic oligonucleotide primers using an Applied Biosystems ABI PRISM™ 377 DNA Sequencer according to the manufacturers instructions.

Transformation of *Aspergillus oryzae* or *Aspergillus niger*

Protoplasts are prepared as described in WO 95/02043, p. 16, line 21 - page 17, line 12.

100 $\mu$l of protoplast suspension is mixed with 5-25 $\mu$g of the appropriate DNA in 10 $\mu$l of STC (1.2 M sorbitol, 10 mM Tris-HCl, pH = 7.5, 10 mM CaCl$_2$). Protoplasts are mixed with p3SR2 (an *A. nidulans* amdS gene carrying plasmid) (Tove Christensen et al. Bio/Technology, pp 1419-1422 vol.6, Dec. 1988). The mixture is left at room temperature for 25 minutes. 0.2 ml of 60% PEG 4000 (BDH 29576), 10 mM CaCl$_2$ and 10 mM Tris-HCl, pH 7.5 is added and carefully mixed (twice) and finally 0.85 ml of the same solution is added and carefully mixed. The mixture is left at room temperature for 25 minutes, spun at 2500 g for 15 minutes and the pellet is resuspended in 2 ml of 1.2 M sorbitol. After one more sedimentation the protoplasts are spread on minimal plates (Cove, Biochem. Biophys. Acta 113 (1966) 51-56) containing 1.0 M sucrose, pH 7.0, 10 mM acetamide as nitrogen source and 20 mM CsCl to inhibit background growth. After incubation for 4-7 days at 37°C spores are picked and spread for single colonies. This procedure is repeated and spores of a single colony after the second reisolation is stored as a defined transformant.

Test of *A. oryzae* transformants

Each of the *A. oryzae* transformants are inoculated in 10 ml of YPM (cf. below) and propagated. After 2-5 days of incubation at 30˚C, the supernatant is removed.

The phytase activity is identified by applying 20 μl supernatant to 4 mm diameter holes punched out in 1% LSB-agarose plates containing 0.1M Sodiumacetate pH 4.5 and 0.1% Inositol hexaphosphoric acid. The plates are left over night at 37˚C. A buffer consisting of 0.1M CaCl2 and 0.2M Sodium acetate pH 4.5 is poured over the plates and the plates are left at room temperature for 1h. Phytase activity is then identified as a clear zone.

Fed batch fermentation:

Fed batch fermentation was performed in a medium comprising maltodextrin as a carbon source, urea as a nitrogen source and yeast extract. The fed batch fermentation was performed by inoculating a shake flask culture of *A. oryzae* host cells in question into a medium comprising 3.5% of the carbon source and 0.5% of the nitrogen source. After 24 hours of cultivation at pH 7.0 and 34˚C the continuous supply of additional carbon and nitrogen sources were initiated. The carbon source was kept as the limiting factor and it was secured that oxygen was present in excess. The fed batch cultivation was continued for 4 days.

Isolation of the DNA sequence shown in SEQ ID No. 23:

The phytase encoding part of the DNA sequence shown in SEQ ID No. 23 coding for the phytase of the invention can be obtained from the deposited organism *Escherichia coli* DSM 11312 by extraction of plasmid DNA by methods known in the art (Sambrook et al. (1989) Molecular cloning: A laboratory manual, Cold Spring Harbor lab., Cold Spring Harbor, NY).

Cloning and expression was done by using the Expression cloning in yeast technique as described above.

mRNA was isolated from *Peniophora lycii,* CBS No. 686.96, grown as described above.

Mycelia were harvested after 15 days' growth, immediately frozen in liquid nitrogen and stored at -80˚C. A library from *Peniophora lycii,* CBS No. 686.96, consisting of approx. $9x10^5$ individual clones was constructed in *E. coli* as described with a vector background of 1%. Plasmid DNA from some of the pools was transformed into yeast, and 50-100 plates containing 250-400 yeast colonies were obtained from each pool.

Phytase-positive colonies were identified and isolated as described above and inoculated into 20 ml YPD broth in a 50 ml glass test tube. The tube was shaken for 2 days at 30˚C. The cells were harvested by centrifugation for 10 min. at 3000 rpm. DNA was isolated according to WO 94/14953 and dissolved in 50 μl water. The DNA was transformed into *E. coli* by standard procedures. Plasmid DNA was isolated from *E. coli* using standard procedures, and the DNA sequence of the cDNA encoding the phytase was sequenced with the Taq deoxy terminal cycle sequencing kit (Perkin Elmer, USA) and synthetic oligonucleotide primers using an Applied Biosystems ABI PRISM™ 377 DNA Sequencer according to the manufacturers instructions. The DNA sequence of the cDNA encoding the phytase is shown in SEQ ID No. 23 and the corresponding amino acid sequence is shown in SEQ ID No. 24. In SEQ ID No. 23 DNA nucleotides from No 1 to No. 1320 define a phytase encoding region.

The part of the DNA sequence in SEQ ID NO 23, which is encoding the mature part of the phytase is position 91 to 1320, which corresponds to amino acid position 31-439 in SEQ ID NO 24.

The cDNA is obtainable from the plasmid in DSM 11312.

Total DNA was isolated from a yeast colony and plasmid DNA was rescued by transformation of *E. coli* as described above. In order to express the phytase in *Aspergillus,* the DNA was digested with appropriate restriction enzymes, size fractionated on gel, and a fragment corresponding to the phytase gene was purified. The gene was subsequently ligated to pHD414, digested with appropriate restriction enzymes, resulting in the plasmid pA2phy2.

After amplification of the DNA in *E. coli* the plasmid was transformed into *Aspergillus oryzae* as described above.

Test of *A. oryzae* transformants

Each of the transformants were tested for enzyme activity as described above. Some of the transformants had phytase activity which was significantly larger than the *Aspergillus oryzae* background. This demonstrates efficient expression of the phytase in *Aspergillus oryzae.*

**EXAMPLE 2**

**Cloning and expression of a phytase from *Agrocybe pediades* CBS No. 900.96**

Deposited organisms:

*Agrocybe pediades* CBS No. 900.96 comprises a phytase encoding DNA sequence of the invention.

*Escherichia coli* DSM NO 11313 contains the plasmid comprising the full length cDNA sequence, coding for a phytase of the invention, in the shuttle vector pYES 2.0.

Isolation of the DNA sequence shown in SEQ ID No. 21:

The phytase encoding part of the DNA sequence shown in SEQ ID No. 21 coding for a phytase of the invention can be obtained from the deposited organism *Escherichia coli* DSM 11313 by extraction of plasmid DNA by methods known in the art (Sambrook et al. (1989) Molecular cloning: A laboratory manual, Cold Spring Harbor lab., Cold Spring Harbor, NY).

Cloning and expression was done by using the Expression cloning in yeast technique as described in Example 1.

mRNA was isolated from *Agrocybe pediades,* CBS No. 900.96, grown as described above with agitation to ensure sufficient aeration.

Mycelia were harvested after 3-5 days' growth, immediately frozen in liquid nitrogen and stored at -80˚C. A library from *Agrocybe pediades,* CBS No. 900.96, consisting of approx. $9x10^5$ individual clones was constructed in *E. coli* as described with a vector background of 1%. Plasmid DNA from some of the pools was transformed into yeast, and 50-100 plates containing 250-400 yeast colonies were obtained from each pool.

Phytase-positive colonies were identified and isolated as described above. cDNA inserts were amplified directly from the yeast colonies and characterized as described in the Materials and Methods section above. The DNA sequence of the cDNA encoding the phytase is shown in SEQ ID No. 21 and the corresponding amino acid sequence is shown in SEQ ID No. 22. In SEQ ID No. 21 DNA nucleotides from No 1 to No. 1362 define the phytase encoding region.

The part of the DNA sequence in SEQ ID NO 21, which is encoding the mature part of the phytase is position 79 to 1362, which correspond to amino acid position 27-453 in SEQ ID NO 22.

The cDNA is obtainable from the plasmid in DSM 11313.

Total DNA was isolated from a yeast colony and plasmid DNA was rescued by transformation of *E. coli* as described above. In order to express the phytase in *Aspergillus,* the DNA was digested with appropriate restriction enzymes, size fractionated on gel, and a fragment corresponding to the phytase gene was purified. The gene was subsequently ligated to pHD414, digested with appropriate restriction enzymes, resulting in the plasmid pA3phy3.

After amplification of the DNA in *E. coli* the plasmid was transformed into *Aspergillus oryzae* as described in Example 1.

Test of *A. oryzae* transformants

Each of the transformants were tested for enzyme activity as described in Example 1. Some of the transformants had phytase activity which was significantly larger than the *Aspergillus oryzae* background. This demonstrates efficient expression of the phytase in *Aspergillus oryzae.*

**Example 3**

**Cloning and expression of phytases from *Paxillus involtus* CBS 100231 and *Trametes pubescens* CBS 100232**

Deposited organisms:

*Paxillus involtus* CBS No. 100231 comprises two phytase encoding DNA sequences of the invention and *Trametes pubescens* CBS 100232 comprises a phytase of the invention.

*Escherichia coli* DSM Nos. 11842, 11843 and 11844 contain the plasmids comprising the full length cDNA sequences, coding for these phytases of the invention, in the shuttle vector pYES 2.0, viz. PhyA1, PhyA2 of *Paxillus involtus* and the phytase of *Trametes pubescens,* respectively.

Isolation of the DNA sequences shown in SEQ ID Nos. 25, 27 and 29:

The phytase encoding part of the DNA sequences shown in SEQ ID Nos. 25, 27 and 29 coding for phytases of the invention can be obtained from the deposited organisms *Escherichia coli* DSM 11842, 11843 and 11844, respectively, by extraction of plasmid DNA by methods known in the art (Sambrook et al. (1989) Molecular cloning: A laboratory manual, Cold Spring Harbor lab., Cold Spring Harbor, NY).

Cloning and expression was done by using the Expression cloning in yeast technique as described in Example 1.

mRNA was isolated from the respective microorganisms, grown under phytase producing conditions, e.g. as described above with agitation to ensure sufficient aeration.

Mycelia were harvested after 3-5 days' growth, immediately frozen in liquid nitrogen and stored at -80˚C.

Libraries, consisting of approx. $9 \times 10^5$ individual clones was constructed in *E. coli* as described with a vector background of 1%. Plasmid DNA from some of the pools was transformed into yeast, and 50-100 plates containing 250-400 yeast colonies were obtained from each pool.

Phytase-positive colonies were identified and isolated as described above. cDNA inserts were amplified directly from the yeast colonies and characterized as described in the Materials and Methods section above. The DNA sequences of the cDNA encoding the phytases are shown in SEQ ID Nos. 25, 27 and 29 and the corresponding amino acid sequences are shown in SEQ ID Nos. 26, 28 and 30, respectively.

The cDNA is obtainable from the plasmids in DSM 11842, 11843 and 11844.

Total DNA was isolated from a yeast colony and plasmid DNA was rescued by transformation of *E. coli* as described above. In order to express the phytases in *Aspergillus,* the DNA was digested with appropriate restriction enzymes, size fractionated on gel, and a fragment corresponding to the phytase gene was purified. The gene was subsequently ligated to pHD414, digested with appropriate restriction enzymes.

After amplification of the DNA in *E. coli* the plasmid was transformed into *Aspergillus oryzae* as described in Example 1.

Test of *A. oryzae* transformants

Each of the transformants were tested for enzyme activity as described in Example 1. Some of the transformants had phytase activity which was significantly larger than the *Aspergillus oryzae* background. This demonstrates efficient expression of the phytases in *Aspergillus oryzae.*

The two phytases of *Paxillus involtus* CBS 100231 (PhyA1*P.i.* and PhyA2*P.i.*) and the phytase of *Trametes pubescens* CBS 100232 (PhyA*T.p.*) have the following characteristics:

| | Number of amino acids | Calculated molecular weight (MW) | Isoelectric point (pl) |
|---|---|---|---|
| **PhyA1 *P.i.*** | 423 | 46 K | 6.4 |
| **PhyA2 *P.i.*** | 423 | 45 K | 4.5 |
| **PhyA *T.p.*** | 426 | 46 K | 4.3 |

## Example 4

**Expression cloning and characterization of five phytase (*phyA*) cDNAs from four basidiomycetes *Agrocybe pediades, Peniophora lycii, Paxillus involtus* and *Trametes pubescens.***

Directional cDNA libraries are constructed as described in the previous examples from phytase induced mycelia from the four basidiomycetes *A. pediades, P. lycii, P. involtus* and *T. pubescens*, in the yeast expression vector pYES2.0.

The cDNA libraries are screened for phytase activity, resulting in isolation of five different phytase cDNAs, *phyA P. lycii, phyA A. pediades, phyA1 P. involtus, phyA2 P. involtus,* and *phyA T. pubescens.*

Characterization of the *phyA* cDNA from these clones reveals conserved regions apparently specific to the basidiomycete phytases. This indicates that the basidiomycete phytases belong to their own subfamily within the group of fungal phytases.

The five new phytases are transformed and overexpressed *in A. oryzae* in order to facilitate the purification and characterization of the recombinant enzymes.

Isolation of *phyA* cDNAs by expression cloning in yeast.

The fungal strains *A. pediades, P. lycii, P. involtus* and *T. pubescens* are cultivated stationary on FG-4 medium (30 g/l soy meal, 15 g/l malto dextrine, 5 g/l bacto peptone, 0.2 g/l pluronic).

The accumulation of total phytase activity in the culture supernatants is monitored on a plate assay as described in the section "Test of A. oryzae transformants" of Example 1.

Highest levels of phytase activity are detected after five to fifteen days of growth, and therefore poly(A)+RNAs isolated from mycelia harvested according to this, are used to construct four cDNA libraries in the yeast expression vector pYES2.0. Aliquots of the libraries are transformed into *S. cerevisiae* W3124 and the transformants are plated on SC agar containing 2% glucose and incubated at 30˚C.

Isolation of poly(A)+ RNA and construction of cDNA libraries is performed as described in Example 1 (the section "Fermentation procedure of Peniophora lycii CBS no. 686.96 for mRNA isolation" to the section "Transformation of Aspergillus oryzae or Aspergillus niger."

Identification of positive colonies

Positive colonies are identified as described in Example 1 under the same heading.

Between 20000 and 30000 yeast clones from each library are screened for phytase activity and one to four phytase positive yeast clones are found in each library. The positive colonies correspond to five different phytase genes, *phyA P. lycii* (pC1phy2), *phyA A. pediades* (pC1phy3), *phyA1 P. involtus* (pC1phy5), *phyA2 P. involtus* (pC1phy7), and *phyA T. pubescens* (pC1phy6).

Characterization of the *phyA* cDNAs:

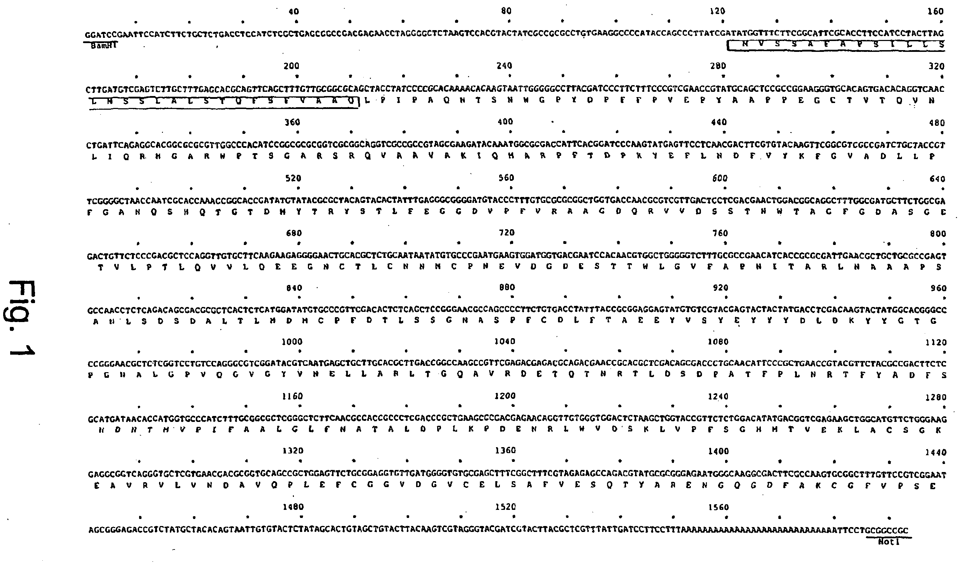

The primary structure of the *phyA* cDNA encoding PhyA of *Peniophora lycii* is shown in Fig. 1. The 1593 bp cDNA from pC1phy2 contains a 1320 bp open reading frame (ORF), coding for a 439 residue polypeptide with a calculated molecular weight of 47560. The *phyA* cDNA encodes an 30 amino acid signal peptide. The mature protein has a calculated molecular weight of 44473 and an isoelectric point of pI 4.15. The cDNA and amino acid sequences are included in the sequence listing, (SEQ ID NO: 23) and (SEQ ID NO: 24), respectively.

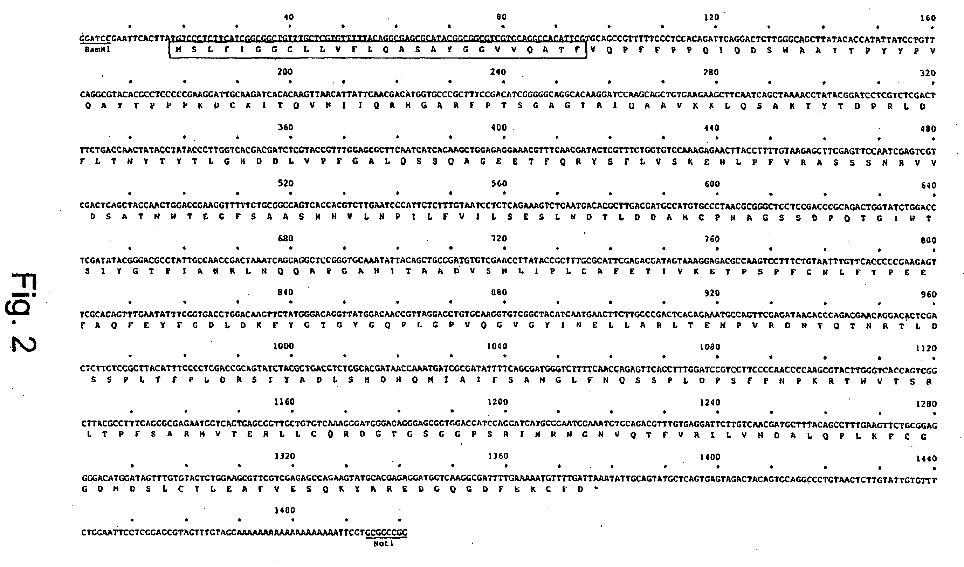

The *phyA* cDNA sequence and the deduced sequence of PHYA from *A. pediades* are presented in Fig. 2. The 1501 bp cDNA from pC1phy3 contains a 1362 ORF coding for a 453 residue polypeptide with a 31 amino acid long signal peptide. The 422 amino acid mature protein has a calculated molecular weight of 46781 and an isoelectric point of pI 4.82. The cDNA and amino acid sequences are included in the sequence listing as (SEQ ID NO: 21) and (SEQ ID NO: 22), respectively.

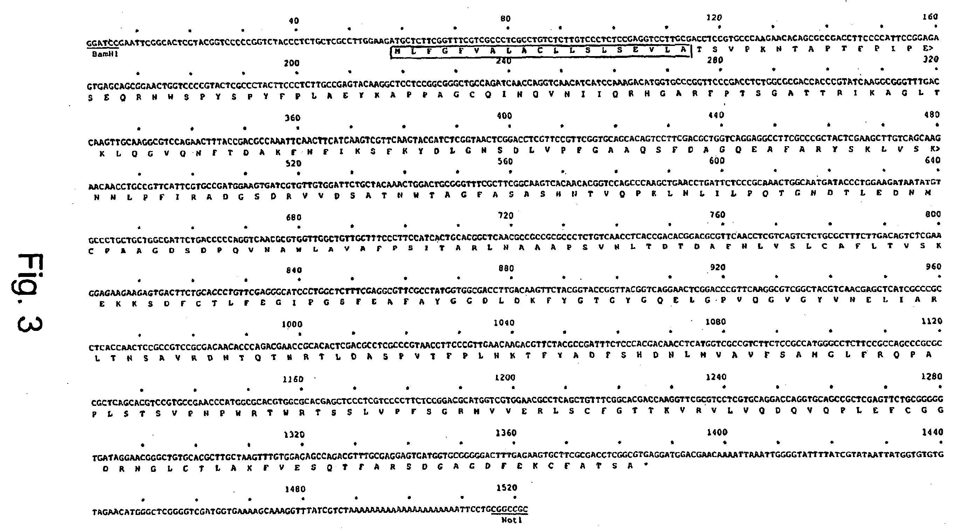

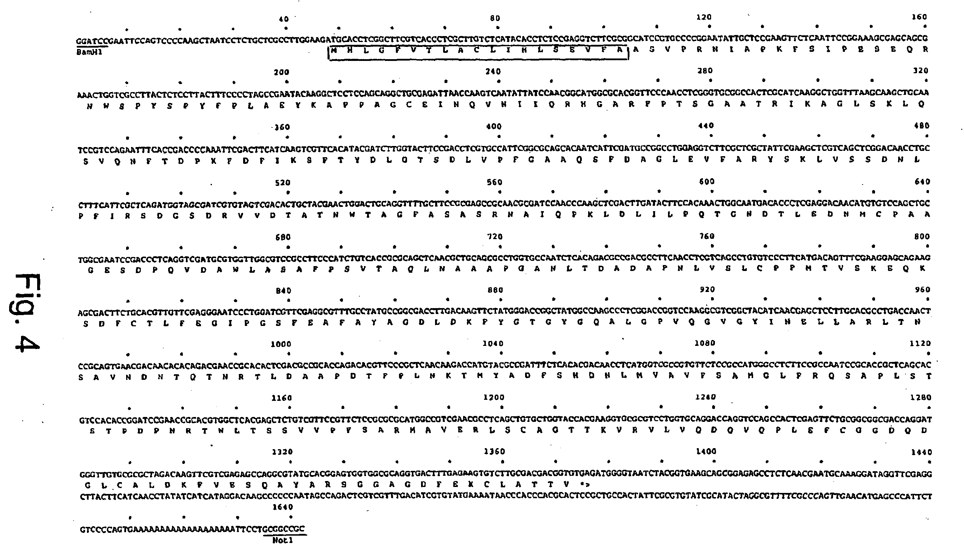

The nucleotide sequences of the *phyA1* and the *phyA2* cDNA cloned from *Paxilus involtus,* and the deduced sequences of PHY1 and PHY2, are shown in Fig. 3. and Fig. 4. respectively.

The 1522 bp insert in pC1phy5 (*phyA1*) contains a 1329 bp ORF coding for a 442 amino acid polypeptide. According to the SignalP V1.1 prediction (Henrik Nielsen, Jacob Engelbrecht, Stren Brunak and Gunnar von Heijne: "Identification of prokaryotic and eukaryotic signal peptides and prediction of their cleavage sites," Protein Engineering 10, 1-6 (1997)), the signal peptide consists of 19 amino acids. The mature protein therefore has a predicted molecular weight of 45932 and a pI of 6.39. The cDNA and amino acid sequences are included in the sequence listing as (SEQ ID NO: 25) and (SEQ ID NO: 26), respectively.

The plasmid pC1phy7 (*phyA2*) contains a 1642 bp insert with a 1329 bp ORF coding for a 442 residue polypeptide. The SignalP V1.1 program referred to above predicts a putative signal peptidase cleavage site between Ala-19 and Ala-20 in the *phyA2* encoded preprotein and thus the predicted molecular weight of the mature protein is 45466 and the predicted isoelectric point is 4.50. The cDNA and amino acid sequences are included in the sequence listing as (SEQ ID NO: 27) and (SEQ ID NO: 28), respectively.

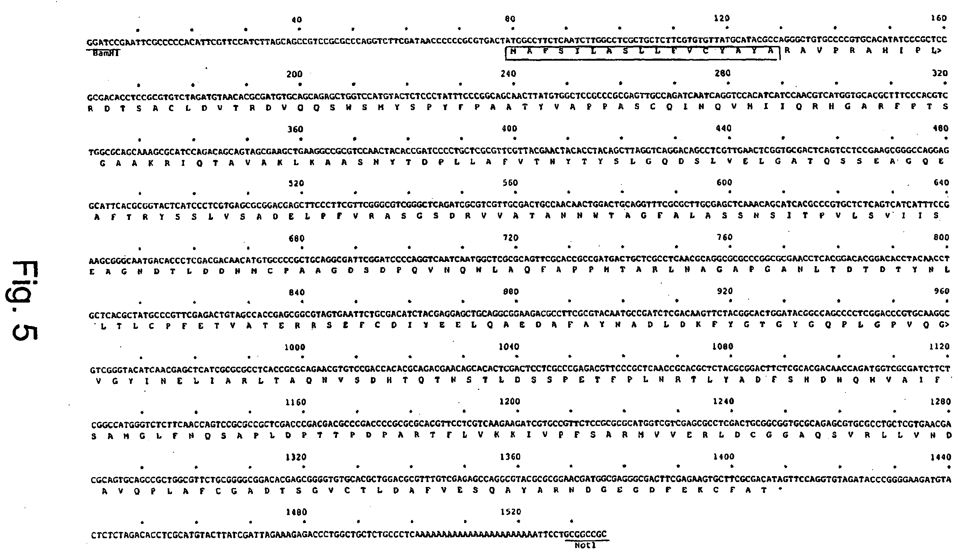

In Fig. 5. the *phyA* cDNA sequence and the deduced sequence of PHYA from *T. pubescens* are shown. The 1536 bp insert in pC1phy6 contains a 1332 bp ORF coding for a 443 residue polypeptide. According to the SignalP V1.1 prediction referred to above, the signal peptide consists of 17 residues. The mature protein therefore consists of 426 amino acids and has a predicted molecular weight of 45905 and a pI of 4.34. The cDNA and amino acid sequences are included in the sequence listing as (SEQ ID NO: 29) and (SEQ ID NO: 30), respectively.

Conserved basidiomycete phytase regions

The overall identities between known phytases of the phyllum Ascomycota and phytases of the invention of the phyllum Basidiomycota and are shown in table 1 below ("X/Y" meaning "DNA/peptide" identity as determined by GAP GCGv8).

In this table, the first five phytases in the leftmost column are basidiomycete phytases, whereas the rest are ascomycete phytases.

## Table 1

Homology of ascomycete and basidiomycete phytases (complete cDNA compared)

| | phyA1 P. involtus | phyA2 P. involtus | phyA T. pubescens | phyA A.pediades | phyA P.lycii | phyA A.fumigatus | phyA A.niger | phyA A.terreus | phyA T.lanuginosa | phyA M.thermophila | phyA T. thermophilus | phyA E. nidulans |
|---|---|---|---|---|---|---|---|---|---|---|---|---|
| phyA1 P. involtus | | 81/85 | 66/62 | 58/58 | 56/56 | 50/40 | 51/40 | 52/41 | 49/40 | 54/41 | 48/37 | 49/40 |
| phyA2 P. involtus | 81/85 | | 66/63 | 59/58 | 57/53 | 49/39 | 50/38 | 51/39 | 48/39 | 54/40 | 50/36 | 49/39 |
| phyA T. pubescens | 66/62 | 66/63 | | 60/61 | 58/51 | 51/40 | 50/41 | 53/39 | 49/40 | 53/41 | 50/39 | 50/39 |
| phyA A.pediades | 58/58 | 59/58 | 60/61 | | 56/52 | 48/37 | 49/38 | 48/38 | 47/37 | 50/39 | 48/37 | 50/42 |
| phyA P.lycii | 56/56 | 57/53 | 58/51 | 56/52 | | 50/41 | 51/42 | 53/41 | 50/39 | 54/45 | 50/43 | 50/40 |
| phyA A.fumigatus | 50/40 | 49/39 | 51/40 | 48/37 | 50/41 | | 65/65 | 64/59 | 59/51 | 58/51 | 62/61 | 65/67 |
| phyA A.niger | 51/40 | 50/38 | 50/41 | 49/38 | 51/42 | 65/65 | | 64/61 | 58/52 | 58/48 | 61/61 | 64/63 |
| phyA A.terreus | 52/41 | 51/39 | 53/39 | 48/38 | 53/41 | 64/59 | 64/61 | | 56/49 | 62/47 | 62/57 | 63/57 |
| phyA T.lanuginosa | 49/40 | 48/39 | 49/40 | 47/37 | 50/39 | 59/52 | 58/53 | 56/49 | | 55/46 | 62/59 | 59/54 |
| phyA M.thermophila | 54/41 | 54/40 | 53/41 | 50/39 | 54/45 | 58/51 | 58/48 | 62/47 | 55/46 | | 57/48 | 57/51 |
| PhyA T. thermophilus | 48/37 | 50/36 | 50/39 | 48/37 | 50/43 | 62/61 | 61/61 | 62/57 | 62/59 | 57/48 | | 60/59 |
| phyA E. nidulans | 49/40 | 49/39 | 50/39 | 50/42 | 50/40 | 65/67 | 64/63 | 63/57 | 59/54 | 57/51 | 60/59 | |
| | | | | | | | | | | | | |
| | | | | | | | | | | | | |

In this experiment, the complete cDNA sequences were compared. According to table 1, the DNA-homology for phytases within the basidiomycetes group is in the range of from 81% to 56% identity, and within the ascomycetes group in the range of from about 65% to 55% identity. Accordingly, the internal group homology seems higher within the group of basidiomycetes phytases as compared to ascomycetes phytases.

The DNA homology of the basidiomycet phytases versus the ascomycet phytases, however, is only in the range of from about 54% to 48%. Accordingly, these two groups as such are more different from each other than the difference observed within each group (and this points towards the discrimination between ascomycete phytases and basidiomycete phytases being legitimate.

This relationship is also visualized in the alignments in Fig. 6. and Fig. 7.

For some of the phytases of Table 1, Table 2 below shows the results when comparing cDNA sequences of ORF and peptide sequences of the mature protein (signal peptide cleaved off).

## Table 2

Homology of selected ascomycete and basidiomycete phytases (ORF cDNA and mature polypeptide compared)

| %ID pep. / %ID DNA | phyA1 P. involtus | phyA2 P. involtus | phyA T. pubescens | phyA A.pediades | phyA P.lycii | phyA A.fumigatus | phyA A.niger | phyA A.terreus | phyA T.lanuginosa | phyA M.thermophila |
|---|---|---|---|---|---|---|---|---|---|---|
| phyA1 P. involtus | | 86 | 63 | 61 | 57 | 40 | 41 | 41 | 40 | 41 |
| phyA2 P. involtus | 81 | | 64 | 61 | 55 | 39 | 39 | 39 | 40 | 41 |
| phyA T. pubescens | 66 | 66 | | 63 | 55 | 41 | 42 | 39 | 40 | 42 |
| phyA A.pediades | 58 | 59 | 60 | | 55 | 39 | 39 | 41 | 37 | 40 |
| phyA P.lycii | 56 | 57 | 58 | 56 | | 42 | 42 | 43 | 41 | 46 |
| phyA A.fumigatus | 50 | 49 | 51 | 48 | 50 | | 67 | 61 | 53 | 53 |
| phyA A.niger | 51 | 50 | 50 | 49 | 51 | 65 | | 62 | 53 | 49 |
| phyA A.terreus | 52 | 51 | 53 | 48 | 53 | 64 | 64 | | 50 | 48 |
| phyA T.lanuginosa | 49 | 48 | 49 | 47 | 50 | 59 | 58 | 56 | | 47 |
| phyA M.thermophila | 54 | 54 | 53 | 50 | 54 | 58 | 58 | 62 | 55 | |

In this table, peptide homologies are indicated in the upper right half of the table, whereas DNA homologies are indicated in the lower left half (both % identity according to GAP GCGv8).

From the alignments at figs. 6 and 7 it is apparent that several sequence motifs are conserved within the five basidiomycete phytases. Based on this alignment several conserved partial sequences have been derived (SEQ ID Nos: 1-14). Still further, some regions of deletions, which are also conserved in the basidiomycete phytases, have also been derived.

Some examples of particularly highly conserved sequences are the so-called Consensus Sequences I, II and III below, the corresponding alignments of which are shown in Tables 3 and 4 below. In these tables, identical residues in at least nine of the sequences are indicated by a grey box and identical residues for the phytases from basidiomycetes are indicated by a white box.

Consensus Sequence I: I-Q-R-H-G-A-R-[F/W]-P-T-S-G-A-X-X-R (SEQ ID NO: 3)

Consensus Sequence II: N-W-T-[A/E]-G-F-X-X-A-S (SEQ ID NO: 5)

Consensus Sequence III: F-V-E-S-Q-X-[Y/F]-A-R-X-X-G-X-G-D-F-[E/A]-K-C (SEQ ID NO: 9)

## Table 3

Partial alignments corresponding to consensus sequences I and II

```
                        AA pos.68 - 83 in P.lycii        AA pos.162 - 171 in P.lycii
phyA1 P. involtus       I Q R H G A R F P T S G A T T R   N W T A G F A S A _ _ _ _ _ _ _ S
phyA2 P.involtus        I Q R H G A R F P T S G A A T R   N W T A G F A S A _ _ _ _ _ _ _ S
phyA T. pubescens       I Q R H G A R F P T S G A A K R   N W T A G F A L A _ _ _ _ _ _ _ S
phyA A. pediades        I Q R H G A R F P T S G A G T R   N W T E G F S A A _ _ _ _ _ _ _ S
phyA P. lycii           I Q R H G A R W P T S G A R S R   N W T A G F G D A _ _ _ _ _ _ _ S
phyA A. fumigatus       L S R H G A R Y P T S S K S K K   K F I E G F Q Q A K L A D P G A _
phyA A. niger           L S R H G A R Y P T D S K G K K   K F I E G F Q S T K L K D P R A Q
phyA A. terreus         L A R H G A R S P T H S K T K A   K F V E G F Q T A R Q D D H H A N
phyA T. lanuginosa      L S R H G A R Y P T A H K S E V   F F N R G F Q D A K D R D P R S N
phyA M. thermophila     L S R H G A R A P T L K R A A S   N F T Q G F H S A L L A D R G S T
```

**Table 4**

Partial alignments corresponding to consensus sequence III

AA pos.415 - 433 in P.lycii

| | | |
|---|---|---|
| phyA1 P. involtus | F V | E S Q | T F | A R | S D | G A | G | D F | E | K | C |
| phyA2 P.involtus | F V | E S Q | A Y | A R | S G | G A | G | D F | E | K | C |
| phyA T. pubescens | F V | E S Q | A Y | A R | N D | G E | G | D F | E | K | C |
| phyA A. pediades | F V | E S Q | K Y | A R | E D | G Q | G | D F | E | K | C |
| phyA P. lycii | F V | E S Q | T Y | A R | E N | G Q | G | D F | A | K | C |
| phyA A. fumigatus | F V | K G L | S W | A R | S G | _ _ | G | N W | G | E | C |
| phyA A. niger | F V | R G L | S F | A R | S G | _ _ | G | D W | A | E | C |
| phyA A. terreus | F V | A G L | S F | A Q | A G | _ _ | G | N W | A | D | C |
| phyA T. lanuginosa | W I | K G L | T F | A R | Q G | _ _ | G | H W | D | R | C |
| phyA M. thermophila | F | I E S M A | F | A R | G N | _ _ | G | K W | D | L | C |

Table 5 below also shows some of the consensus sequences, viz. (SEQ ID NO: 2), (SEQ ID NO: 5) and (SEQ ID NO: 9), respectively, in an alignment as in Fig. 7.

## Table 5

Basidiomycete phytase consensus sequences in alignment

| AA pos. P. lycii | 64 | | | | 70 | | | | | | | 75 | | | | 80 | |
|---|---|---|---|---|---|---|---|---|---|---|---|---|---|---|---|---|---|
| phyA1 P. involtus | Q | V | N | I | I | Q | R | H | G | A | R | F | P | T | S | G | A |
| phyA2 P.involtus | Q | V | N | I | I | Q | R | H | G | A | R | F | P | T | S | G | A |
| phyA T. pubescens | Q | V | H | I | I | Q | R | H | G | A | R | F | P | T | S | G | A |
| phyA A. pediades | Q | V | N | I | I | Q | R | H | G | A | R | F | P | T | S | G | A |
| phyA P. lycii | Q | V | N | L | I | Q | R | H | G | A | R | W | P | T | S | G | A |
| phyA A. fumigatus | L | V | Q | V | L | S | R | H | G | A | R | Y | P | T | S | S | K |
| phyA A. niger | F | A | Q | V | L | S | R | H | G | A | R | Y | P | T | D | S | K |
| phyA A. terreus | F | V | Q | V | L | A | R | H | G | A | R | S | P | T | H | S | K |
| phyA T. lanuginosa | F | V | Q | V | L | S | R | H | G | A | R | Y | P | T | A | H | K |
| phyA M. thermophila | F | A | Q | V | L | S | R | H | G | A | R | A | P | T | L | K | R |

| AA pos. P. lycii | 162 | | | | | | | | 170 | | | | | | | | 171 |
|---|---|---|---|---|---|---|---|---|---|---|---|---|---|---|---|---|---|
| phyA1 P. involtus | N | W | T | A | G | F | A | S | A | _ | _ | _ | _ | _ | _ | _ | S |
| phyA2 P.involtus | N | W | T | A | G | F | A | S | A | _ | _ | _ | _ | _ | _ | _ | S |
| phyA T. pubescens | N | W | T | A | G | F | A | L | A | _ | _ | _ | _ | _ | _ | _ | S |
| phyA A. pediades | N | W | T | E | G | F | S | A | A | _ | _ | _ | _ | _ | _ | _ | S |
| phyA P. lycii | N | W | T | A | G | F | G | D | A | _ | _ | _ | _ | _ | _ | _ | S |
| phyA A. fumigatus | K | F | I | E | G | F | Q | Q | A | K | L | A | D | P | G | A | _ |
| phyA A. niger | K | F | I | E | G | F | Q | S | T | K | L | K | D | P | R | A | Q |
| phyA A. terreus | K | F | V | E | G | F | Q | T | A | R | Q | D | D | H | H | A | N |
| phyA T. lanuginosa | F | F | N | R | G | F | Q | D | A | K | D | R | D | P | R | S | N |
| phyA M. thermophila | N | F | T | Q | G | F | H | S | A | L | L | A | D | R | G | S | T |

| AA pos. P. lycii | 415 | | | | | | | 423 | | | | | | | | | 431 | | |
|---|---|---|---|---|---|---|---|---|---|---|---|---|---|---|---|---|---|---|---|
| phyA1 P. involtus | F | V | E | S | Q | T | F | A | R | S | D | G | A | G | D | F | E | K | C |
| phyA2 P.involtus | F | V | E | S | Q | A | Y | A | R | S | G | G | A | G | D | F | E | K | C |
| phyA T. pubescens | F | V | E | S | Q | A | Y | A | R | N | D | G | E | G | D | F | E | K | C |
| phyA A. pediades | F | V | E | S | Q | K | Y | A | R | E | D | G | Q | G | D | F | E | K | C |
| phyA P. lycii | F | V | E | S | Q | T | Y | A | R | E | N | G | Q | G | D | F | A | K | C |
| phyA A. fumigatus | F | V | K | G | L | S | W | A | R | S | G | _ | _ | G | N | W | G | E | C |
| phyA A. niger | F | V | R | G | L | S | F | A | R | S | G | _ | _ | G | D | W | A | E | C |
| phyA A. terreus | F | V | A | G | L | S | F | A | Q | A | G | _ | _ | G | N | W | A | D | C |
| phyA T. lanuginosa | W | I | K | G | L | T | F | A | R | Q | G | _ | _ | G | H | W | D | R | C |
| phyA M. thermophila | F | I | E | S | M | A | F | A | R | G | N | _ | _ | G | K | W | D | L | C |

Consensus Sequence I (SEQ ID NO: 3), residue position 68 to 83 with the numbering for PHYA *P. lycii* in Fig. 7., is around the active site, and all five basidiomycetes phytases have this consensus sequence I-Q-R-H-G-A-R-[F/W]-P-T-S-G-A-X-X-R with thirteen conserved residues. Still further, four of the five phytases have a fourteenth common residue F75. This is in contrast to the ascomycetes phytases which only have eight conserved residues in the same region (Table 3).

When Consensus Sequence II (SEQ ID NO: 5), AA position 162 - 171 in *P. lycii*, is compared to the ascomycete phytases it can be seen that the basidiomycete phytases lack six to seven residues between *P. lycii* F167 (*A. niger* F177) to *P. lycii* P177 (*A. niger* P194) (See Fig. 7) and that the basidiomycetes phytases overall have a much larger degree of conservation with seven identical residues out of ten (Table 3). The ascomycetes phytases have only three conserved residues out of seventeen in the same region.

Consensus Sequence III (SEQ ID NO: 9), AA pos. 415-433 in P. *lycii*, consists of nineteen residues with thirteen residues conserved in the basidiomycetes phytases. There are three residues in the consensus sequence that are conserved through all the fungal phytases. In the *P. lycii* sequence the residues are A422, G428, and C433 and for *A. niger* they are A454, G458, and C463. All the basidiomycete phytases have five residues between the conserved alanine and glycine while all the ascomycete phytases only have three (Table 4).

Expression of PHYA in *Aspergillus oryzae*

In order to obtain high level expression of the PHYA phytases in *Aspergillus oryzae* for further purification and characterization of the protein, the five *phyA* cDNAs from A. *pediades, P. lycii, P. involtus,* and *T. pubescens* were subcloned into pHD414, a fungal expression vector. The *phyA* cDNA is here inserted 3' to the TAKA-amylase promoter sequence and 5' to the polyA and terminator sequence from the *A. niger* glucoamylase gene. The pHD414 *phyA* constructs were transformed into *A. oryzae* by co-transformation with the *amdS* selection plasmid (see the section "Transformation of Aspergillus oryzae or Aspergillus niger" in Example 1). The transformants were screened for phytase activity in the supernatants, and the highest yielding transformants were selected for fermentation.

Conclusion

The high degree of conserved regions within this group of basidiomycete phytases indicate that they belong to their own subfamily within the group of fungal phytases.

Based on these regions PCR-primers specific for molecular screening of related phytases can be designed (Example 5).

**Example 5**

**Molecular screening (primerset 522/538)**

The following degenerate oligonucleotide primers coding for highly conserved regions within the five basidiomycete phytases have been designed for molecular screening:

522 sense primer:

```
5' - CCC AAG CTT AAY TGG ACN GMN GGN TT - 3'
(SEQ ID NO: 15)
```

corresponds to amino acids N-W-T-[A,E,D]-G-[F,L] with a CCC and HindIII site 5' tail;

537 sense primer:

```
5' - CCC AAG CTT GAY AAR TWY GGN AC - 3'
(SEQ ID NO: 16)
```

corresponds to amino acids D-K-[F,Y]-Y-G-T with a CCC and HindIII site 5' tail;

538 anti-sense primer:

```
5' - GCT CTA GAC RTA RWA YTT RTC NAR RTC - 3'
(SEQ ID NO: 17)
```

corresponds to amino acids D-[F,L]-D-K-[F,Y]-Y-G with a GC and XbaI site 5' tail;

525 anti-sense primer:

```
5' - GCT CTA GAC AYT TNK CRA ART CNC C - 3'
(SEQ ID NO: 18)
```

corresponds to amino acids G-D-F-[A,D,E]-K with a GC and XbaI site 5' tail;

539 sense primer:

```
5' - CCC AAG CTT CAR GTN MAY MTN ATH CA - 3'
(SEQ ID NO: 19)
```

corresponds to amino acids Q-V-[N,H]-[I,L,M]-I-[Q,H] with a CCC and HindIII site 5' tail (SEQ ID NO: 15);

540 anti-sense primer:

```
5' - GCT CTA GAC RAA NCC NKC NGT CCA RTT - 3'
(SEQ ID NO: 20)
```

corresponds to amino acids N-W-T-[A,D,E]-G-F with a GC and XbaI site 5' tail;

wherein N = A, C, G or T; R = A or G; Y = C or T; M = A or C; W = A or T.

The design of the primers is based on the alignment in Fig. 7.

For a general reference to the PCR reaction, reference can be had to e.g. Sambrook et al, Molecular Cloning, a Laboratory Manual, 2nd edition; or Lubert Stryer: Biochemistry, 4th edition, Freeman and Company, New York, 1995, e.g. pp. 132-134.

First, the 522/538 primerset is tested on genomic DNA from selected ascomycetes and basidiomycetes shown in Table 6 below.

The genomic DNA is isolated according to the following procedure:

Procedure for isolation of fungal genomic DNA.

1. Grind mycelia in liquid N2 in a morter
2. Transfer mycelia to an 2.0 ml Eppendorf tube up to the 0.5 ml mark
3. Add 1.0 ml lysis buffer and mix
4. Add 10 μl 4 mg/ml DNase free RNase A (New England Biolabs)
5. Incubate for 30 min. at 37˚C
6. Add 40 μl 16 mg/ml Protease K (New England Biolabs)
7. Incubate for 1 h. at 50˚C with gently shaking
8. Centrifuge for 15 minutes full speed in a microcentrifuge
9. Apply supernatant to a QIAprep-spin column. Spin for 1 min. and discard filtrate
10. Wash with 0.5 ml buffer PB, spin for 1 min. and discard filtrate
11. Wash with 0.75 ml buffer PE, spin for 1 min. and discard filtrate
12. Drain any existing PE buffer with a quick spin and let dry completely

13. Place spin column in a clean microfuge tube and elute by adding 125 μl H20. Let sit for 5 min. and then spin for 3 min

Lysis buffer:

100 mM EDTA
10 mM Tris pH. 8 1% Triton X-100
200 mM NaCl
500 mM Guanidine-HCl
For further information on QIAprep spin column, PB buffer and PE buffer please refer to the QIAprep™ Plasmid Handbook from QIAGEN GmbH.

Experimental procedure

Approximately 100 to 200 ng genomic DNA or 10-20 ng doublestranded cDNA is used as template for PCR amplification in PCR buffer (10 mM Tris-HCl, pH 8.3, 50 mM KCl) containing 200 μM of each dNTP, 3.5 mM MgCl2, 2.5 Units AmpliTaq Gold™, and 100 pmol of each of the degenerate primers 522 and 538. The total volume is 50 μl. The PCR reaction is caried out in a Perkin - Elmer GeneAmp PCR System 2400. The PCR reaction is performed using a cycle profile of:

94 ˚C - 10 min; 1 cycle
94 ˚C - 1 min, 60˚C - 1 min, 72˚C - 30 sec; 2 cycles
94 ˚C - 1 min, 59˚C - 1 min, 72˚C - 30 sec; 2 cycles
94 ˚C - 1 min, 58˚C - 1 min, 72˚C - 30 sec; 2 cycles
-
-
-
94 ˚C - 1 min, 52˚C - 1 min, 72˚C - 30 sec; 2 cycles
94 ˚C - 1 min, 50˚C - 1 min, 72˚C - 30 sec; 14 cycles
72˚C - 7 min; 1 cycles

5 μl aliquots of the amplification products are analyzed by electrophoresis in 1.5% agarose gels.
Table 6 below shows the results of the test of this primerset, viz. whether a specific PCR band was detected or not.

**Table 6**

| Test of primerset 522/538 on genomic DNA from asco- and basidiomycetes | | | |
|---|---|---|---|
| **Microorganism** | **Phyllum** | **Strain collection number** | **PCR band detected** |
| Cladorinum sp. | Ascomycota | CBS 427.97 | No |
| Cytospora sp. | Ascomycota | CBS 424.97 | No |
| Cytospora sp. | Ascomycota | CBS 425.97 | No |
| Gelasinospora sp. | Ascomycota | NN 040455 | No |
| Agrocybe pediades | Basidiomycota | CBS 900.96 | Yes |
| Amylostereum Chailletii | Basidiomycota | NN strain collection | Yes |
| Bjerkandera adusta | Basidiomycota | CBS 580.95 | Yes |
| Bjerkandera sp. | Basidiomycota | NN strain collection | Yes |
| Bolbitius aleuritus | Basidiomycota | do | Yes |
| Cerrena unicolor | Basidiomycota | do | Yes |
| Coniophora arida | Basidiomycota | do | Yes |
| Conocybe sp. | Basidiomycota | do | Yes |
| Coprinus cinereus | Basidiomycota | IFO 30116 | Yes |
| Cystoderma carcharias | Basidiomycota | NN strain collection | Yes |

(continued)

| Test of primerset 522/538 on genomic DNA from asco- and basidiomycetes | | | |
|---|---|---|---|
| **Microorganism** | **Phylum** | **Strain collection number** | **PCR band detected** |
| Daedalea quercina | Basidiomycota | NN005877 | Yes |
| Exidia glandulosa | Basidiomycota | CBS 277.96 | Yes |
| Femsjonia sp. | Basidiomycota | NN strain collection | Yes |
| Fomes fomentarius | Basidiomycota | CBS 276.96 | Yes |
| Hygrophoropsis pallida | Basidiomycota | NN strain collection | Yes |
| Hyphoderma argillaceum | Basidiomycota | do | Yes |
| Hyphodontia pallidula | Basidiomycota | do | Yes |
| Hypholoma fasciculare | Basidiomycota | do | Yes |
| Irpex lacteus | Basidiomycota | do | Yes |
| Laetisaria arvalis | Basidiomycota | do | Yes |
| Lyophyllum sp. | Basidiomycota | do | Yes |
| Marasmiellus ramealis | Basidiomycota | do | Yes |
| Merismodes sp. | Basidiomycota | do | Yes |
| Merulius tremellosus | Basidiomycota | do | Yes |
| Oxyporus corticola | Basidiomycota | do | Yes |
| Oxyporus sp. | Basidiomycota | CBS 422.97 | Yes |
| Panaeolus semiovatus | Basidiomycota | CBS 819.95 | Yes |
| Paxillus involtus | Basidiomycota | CBS 100231 | Yes |
| Peniophora cinerea | Basidiomycota | NN007373 | Yes |
| Peniophora lycii | Basidiomycota | CBS 686.96 | Yes |
| Peniophora quercina | Basidiomycota | NN 009335 | Yes |
| Podaxis pistillaris | Basidiomycota | ATCC 38868 | Yes |
| Scizophyllum commune | Basidiomycota | NN strain collection | Yes |
| Scizophyllum sp. | Basidiomycota | CBS 443.97 | Yes |
| Skeletocutis sp. | Basidiomycota | NN strain collection | Yes |
| Steccherinum ochraceum | Basidiomycota | do | Yes |
| Stereum subtomentosum | Basidiomycota | do | Yes |
| Strobilurus tenacellus | Basidiomycota | do | Yes |
| Stropharia cubensis | Basidiomycota | ATCC 13966 | Yes |
| Trametes hirsuta | Basidiomycota | DSM 2987 | Yes |
| Trametes pubescens | Basidiomycota | CBS 100232 | Yes |
| Trametes zonatella | Basidiomycota | NN strain collection | Yes |
| Trechispora farinaceae | Basidiomycota | do | Yes |
| Trichaptum fuscoviolaceum | Basidiomycota | do | Yes |
| Typhula setipes | Basidiomycota | do | Yes |
| Volvariella speciosa | Basidiomycota | do | Yes |

**Example 6**

**Molecular screening (other primersets)**

Primersets 522/525, 539/540, 539/538, 539/525 and 537/525 are tested as described in Example 5 above, using 100 pmol of each of the sense and anti-sense degenerate primers. Touchdown PCR is used for amplification (ref: R.H.Don et al. (1991), Nucleic Acid Research, Vol. 19, No. 14) modified for the AmpliTaq Gold (TM). The PCR reaction is performed using a cycle profile of:

```
94 C - 10 min; 1 cycle
94 C - 1 min, 60C - 1 min, 72C - 1.5 min; 2 cycles
94 C - 1 min, 59C - 1 min, 72C - 1.5 min; 2 cycles
94 C - 1 min, 58C - 1 min, 72C - 1.5 min; 2 cycles
-
-
-
94 C - 1 min, 52C - 1 min, 72C - 1.5 min; 2 cycles
94 C - 1 min, 50C - 1 min, 72C - 1.5 min; 14 cycles
72C - 7 min; 1 cycle.
```

**Example 7**

**Purification and sequencing of PCR bands**

The PCR fragments can be purified and sequenced using the Jet sorb Gel extraction Kit (Genomed GmbH, Germany) according to the manufacturer's instructions. The nucleotide sequences of the amplified PCR fragments are determined directly on the purified PCR products using 200-300 ng as template, the Taq deoxy-terminal cycle sequencing kit (Perkin-Elmer, USA), flourescent labeled terminators and 5 pmol sequence primer on a ABI PRISMt 377 DNA Sequencer, Perkin Elmer.

The PCR fragments generated with primer set 522/538, and with approximately 10-20 ng of doublestranded cDNA from *Schizophyllum sp.* CBS 443.97 as template, was purified and sequenced as described above and the DNA sequence was deduced to (5'- to 3'-):

```
TCTGCCGCATCTGACGGTGTCTATAACCCCGTCCTCAACCTGATTATATCAGAAGAGCTTAA
CGACACCCTCGATGATGCGATGTGCCCGAACGTCGGCGAATCGGACGCCCAAACGGACGAAT
```

```
GGACGTCTATTTACGCAGCGCCCATCGCTGAGCGTCTGAACAACAACGCCGTGGGCGCTAAC
CTGACCACCACGAACGTTTACAACCTCATGTCTTTATGCCCCTTCGACACGCTTGCGAAGGA
GACGCCGAGCCCCTTCTGCGATCTCTTT (SEQ ID NO: 31)
```

and translated into amino acid sequence:

```
SAASDGVYNPVLNLIISEELNDTLDDAMCPNVGESDAQTDEWTSIYAAPIAERLNNNAVGAN
LTTTNVYNLMSLCPFDTLAKETPSPFCDLF (SEQ ID NO: 32).
```

The doublestranded cDNA was synthesized as described in Example 1.

### Example 8

**Purification and characterization of the phytase from Peniophora lycii expressed in Aspergillus oryzae**

The *Peniophora lycii* phytase was expressed in and excreted from *Aspergillus oryzae* IFO 4177.

Filter aid was added to the culture broth which was filtered through a filtration cloth. This solution was further filtered through a Seitz depth filter plate resulting in a clear solution. The filtrate was concentrated by ultrafiltration on 3kDa cut-off polyethersulphone membranes followed by diafiltration with distilled water to reduce the conductivity. The pH of the concentrated enzyme was adjusted to pH 7.5. The conductivity of the concentrated enzyme was 1.2 mS/cm.

The phytase was applied to a Q-sepharose FF column equilibrated in 20mM Tris/$CH_3COOH$, pH 7.5 and the enzyme was eluted with an increasing linear NaCl gradient (0 $\rightarrow$ 0.5M). The phytase activity eluted as a single peak. This peak was pooled and $(NH_4)_2SO_4$ was added to 1.5M final concentration. A Phenyl Toyopearl 650S column was equilibrated in 1.5M $(NH_4)_2SO_4$, 10mM succinic acid/NaOH, pH 6.0 and the phytase was applied to this column and eluted with a decreasing linear $(NH_4)_2SO_4$ gradient (1.5 $\rightarrow$ 0M). Phytase containing fractions were pooled and the buffer was exchanged for 20mM Tris/$CH_3COOH$, pH 7.5 on a Sephadex G25 column. The G25 filtrate was applied to a Q-sepharose FF column equilibrated in 20mM Tris/$CH_3COOH$, pH 7.5. After washing the column extensively with the equilibration buffer, the phytase was eluted with an increasing linear NaCl gradient (0 $\rightarrow$ 0.5M). The phytase activity was pooled and the buffer was exchanged for 20mM Tris/$CH_3COOH$, pH 7.5 by dialysis. The dialysed phytase was applied to a SOURCE 30Q column equilibrated in 20mM Tris/$CH_3COOH$, pH 7.5. After washing the column thoroughly with the equilibration buffer a phytase was eluted with an increasing linear NaCl gradient (0 $\rightarrow$ 0.3M). Fractions from the SOURCE 30Q column were analyzed by SDS-PAGE and pure phytase fractions were pooled.

The *Peniophora* phytase migrates in the gel as a band with $M_r$ = 67 kDa. N-terminal amino acid sequencing of the 67 kDa component was carried out following SDS-PAGE and electroblotting onto a PVDF-membrane. The following N-terminal amino acid sequence could be deduced:

Leu-Pro-Ile-Pro-Ala-Gln-Asn-

The sequence corresponds to amino acid residues 31-37 in the cDNA derived amino acid sequence.

Accordingly a mature amino acid sequence of the phytase when expressed in Aspergillus is supposed to be no. 31-439 of SEQ ID no 24.

### Example 9

**Further characterization of the purified phytase of Peniophora lycii**

The phytase of Peniophora lycii was expressed in Aspergillus and purified as described in Example 8.

The phytase activity is measured using the following assay:

10 $\mu$l diluted enzyme samples (diluted in 0.1 M sodium acetate, 0.01 % Tween20, pH 5.5) were added into 250 $\mu$l 5 mM sodium phytate (Sigma) in 0.1 M sodium acetate, 0.01 % Tween20, pH 5.5 (pH adjusted after dissolving the sodium phytate; the substrate was preheated) and incubated for 30 minutes at 37˚C. The reaction was stopped by adding 250 $\mu$l 10 % TCA and free phosphate was measured by adding 500 $\mu$l 7.3 g $FeSO_4$ in 100 ml molybdate reagent (2.5 g $(NH_4)_6Mo_7O_{24}.4H_2O$ in 8 ml $H_2SO_4$ diluted to 250 ml). The absorbance at 750 nm was measured on 200 $\mu$l samples in 96 well microtiter plates. Substrate and enzyme blanks were included. A phosphate standard curve was also included (0-2 mM phosphate). 1 FYT equals the amount of enzyme that releases 1 $\mu$mol phosphate/min at the given conditions.

Temperature profiles were obtained by running the assay at various temperatures (preheating the substrate).

Temperature stability was investigated by preincubating the phytases in 0.1 M sodium phosphate, pH 5.5 at various temperatures before measuring the residual activity.

The pH-stability was measured by incubating the enzyme at pH 3 (25 mM glycine-HCl), pH 4-5 (25 mM sodium acetate), pH 6 (25 mM MES), pH 7-9 (25 mM Tris-HCl) for 1 hour at 40 ˚C, before measuring the residual activity.

The pH-profiles were obtained by running the assay at the various pH using the same buffer-systems (50 mM, pH was readjusted when dissolving the substrate).

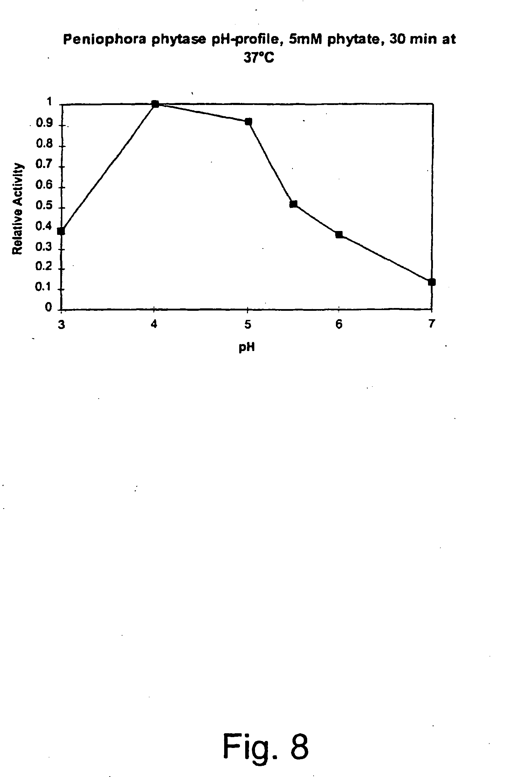

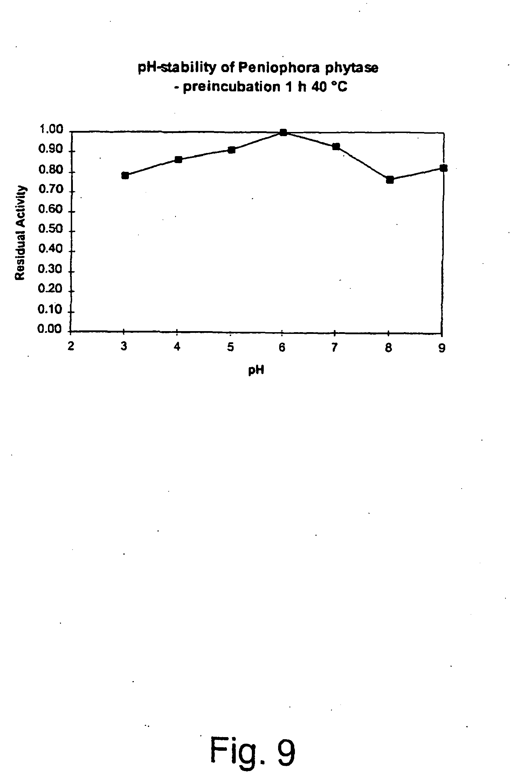

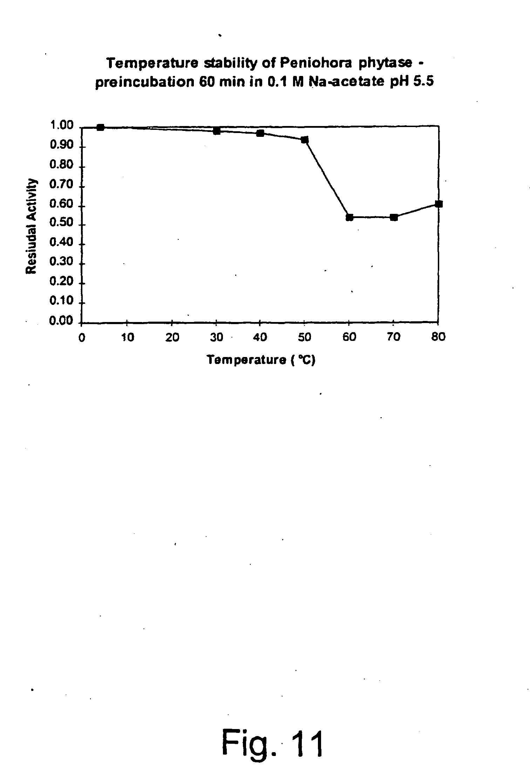

The results of the above pH-profile, pH-stability, temperature-profile and temperature stability studies are shown in figs. 8, 9, 10 and 11, respectively. From fig. 9 it appears that the phytase of Peniophora lycii is very stable (i.e. more than 80% of the maximum activity retained) for 1 hour at 40˚C in the whole range of pH 3-9. And as regards the temperature stability results shown at fig. 11, it appears that at 60-80˚C some 50-60% of the residual activity still remains. This fact is contemplated to be due to the enzyme being surprisingly capable of refolding following its thermal denaturation.

The degree of refolding will depend on the exact conditions (pH, enzyme concentration).

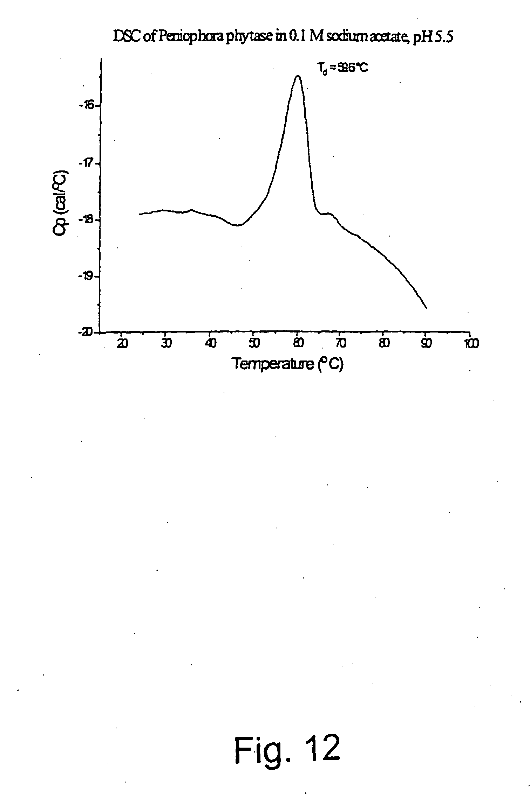

Fig. 12 shows the result of differential scanning calorimetry (DSC) measurements on the Peniophora phytase. In DSC the heat consumed to keep a constant temperature increase in the sample-cell is measured relative to a reference cell. A constant heating rate is kept (e.g. 90˚C/hour). An endothermal process (heat consuming process - e.g. the unfolding of an enzyme/protein) is observed as an increase in the heat transferred to the cell in order to keep the constant temperature increase. DSC was performed using the MC2-apparatus from MicroCal. Cells were equilibrated 20 minutes at 20˚C before scanning to 90˚C at a scan rate of 90˚/h. Samples of around 2.5 mg/ml *Peniophora* phytase in 0.1 M sodium acetate, pH 5.5 were loaded.

## Example 10

### Determination of the specific activity of the Peniophora phytase

The specific activity is determined on a highly purified sample of the phytase (the purity was checked beforehand on an SDS poly acryl amide gel showing the presence of only one component).

The protein concentration in the phytase sample was determined by amino acid analysis as follows: An aliquot of the phytase sample was hydrolyzed in 6N HCl, 0.1% phenol for 16 h at 110 C in an evacuated glass tube. The resulting amino acids were quantified using an Applied Biosystems 420A amino acid analysis system operated according to the manufacturers instructions. From the amounts of the amino acids the total mass - and thus also the concentration - of protein in the hydrolyzed aliquot can be calculated.

The activity is determined in the units of FYT. One FYT equals the amount of enzyme that liberates 1 micromol inorganic phosphate from phytate (5 mM phytate) per minute at pH 5.5, 37 C; assay described e.g. in example 11.

The specific activity is calculated to 987 FYT/mg enzyme protein.

## Example 11

### Time-resolved product-profiling of phytase-catalyzed hydrolysis of phytic acid by $^1$H NMR spectroscopy

The hydrolysis of phytic acid (PA) catalyzed by the Peniophora phytase and by a commercial Aspergillus niger phytase (Phytase Novo®) was investigated (27 mM phytate, 1 FYT/ml, pH 5.5 and 3.5, and 27˚C) by $^1$H NMR profiling the product mixture in the course of 24 hours.

In the following (Ins(p,q,r,..)$P_n$ denotes *myo*-inositol carrying in total n phosphate groups attached to positions p, q, r,.. For convenience Ins $(1,2,3,4,5,6)P_6$ (phytic acid) is abbreviated PA. Please refer, however, to the section "Nomenclature and position specificity of phytases" in the general part of this application.

The technique provide specific information about initial points of attack by the enzyme on the PA molecule, as well as information about the identity of the end product. On the other side the evolving patterns of peaks reflecting the composition of the intermediate product mixtures, provide a qualitative measure, a finger print, suitable for identification of similarities and differences between individual enzymes.

NMR, like most other analytical methods, can distinguish between stereo-isomers which are not mirror images (diastereomers), but not between a set of isomers, which are mirror-images (enantiomers), since they exhibit identical NMR spectra.

Thus, Ins$(1,2,4,5,6)P_5$ (3-phosphate removed) exhibits a NMR spectrum different from Ins$(1,2,3,4,5)P_5$ (6-phosphate removed) because the isomers are diastereomers.

However, the NMR spectra of Ins (1, 2, 4, 5, 6) $P_5$ and Ins$(2,3,4,5,6)P_5$ (1-phosphate removed) are identical because the isomers are enantiomers. The same holds for the pair Ins (1, 2, 3, 4, 5) $P_5$ and Ins$(1,2,3,5,6)P_5$ (4-phosphate removed).

Thus, by NMR it is not possible to distinguish between a 3-and a 1-phytase, and it is not possible to distinguish between a 6- and a 4-phytase (or a L-6- and a D-6-phytase using the lowest-locant rule).

Biased by the description of 3- and 6-phytases in the literature, we have used the terms 3- and 6-phytases for our enzymes, but, though unlikely, we do not actually know if we have a 1- and a 4-phytase instead.

Experimental.

NMR spectra were recorded at 300 K (27˚C) on a Bruker DRX400 instrument equipped with a 5 mm selective inverse probe head. 16 scans preceded by 4 dummy scans were accumulated using a sweep width of 2003 Hz (5 ppm) covered by 8 K data points. Attenuation of the residual HOD resonance was achieved by a 3 seconds presaturation period. The spectra were referenced to the HOD signal ($\delta$ 4.70).

PA samples for NMR analysis were prepared as follows: PA (100 mg, Phytic acid dipotassium salt, Sigma P-5681) was dissolved in deionized water (4.0 ml) and pH adjusted to 5.5 or 3.5 by addition of aqueous NaOH (4 N). Deionized water was added (ad 5 ml) and 1 ml portions, each corresponding to 20 mg of phytic acid, were transferred to screw-cap vials and the solvent evaporated (vacuum centrifuge). The dry samples were dissolved in deuterium oxide (2 ml, Merck 99.5% D) and again evaporated to dryness (stored at -18°C until use).

For NMR analysis one 20 mg phytic acid sample was dissolved in deuterium oxide (1.0 ml, Merck 99.95% D). The solution was transferred to a NMR tube and the $^1$H NMR spectrum recorded. Enzyme solution (1 FTU, dissolved in/diluted, as appropriate, with deuterium oxide) was added followed by thorough mixing (1 minute). $^1$H NMR spectra were recorded immediately after addition of enzyme (t=0), then after 5, 10, 15, 20, 25, 30, 45, 60, 75, 90, 105, 120, 135 150, 165, 180, 195, 210 minutes (= 3.5 hours), 4.5, 5.5 6.5, 7.5, 8.5, 9.5, 11.5, 13.5, 15.5, 17.5, 19.5, 21.5, and 23.5 hours. The pH in the NMR tube was measured. Additional spectra were acquired after 48 and 120 hours (5 days), where a portion of substrate (PA, 6 mg) was added to probe if the enzyme retained its catalytic activity.

By means of 2D NMR analysis of inositol phosphate mixtures obtained by partial digestion of PA, in conjunction with published NMR data (Scholz, P.; Bergmann, G., and Mayr, G.W.: Methods in Inositide Research (Ed. Irvine, R.F.), pp. 65-82, Raven Press, Ltd., New York (1990)), characteristic $^1$H NMR signals attributable to $Ins(1,2,3,4,5,6)P_6$ (PA), $Ins(1, 2, 4, 5, 6)P_5$, $Ins(1,2,3,4,5)P_5$, $Ins(1, 2, 5, 6)P_4$, $Ins(1, 2, 6)P_3$, $Ins(1,2)P_2$, and $Ins(2)P$, were identified and permitted relative quantification of these species during the course of the reaction.

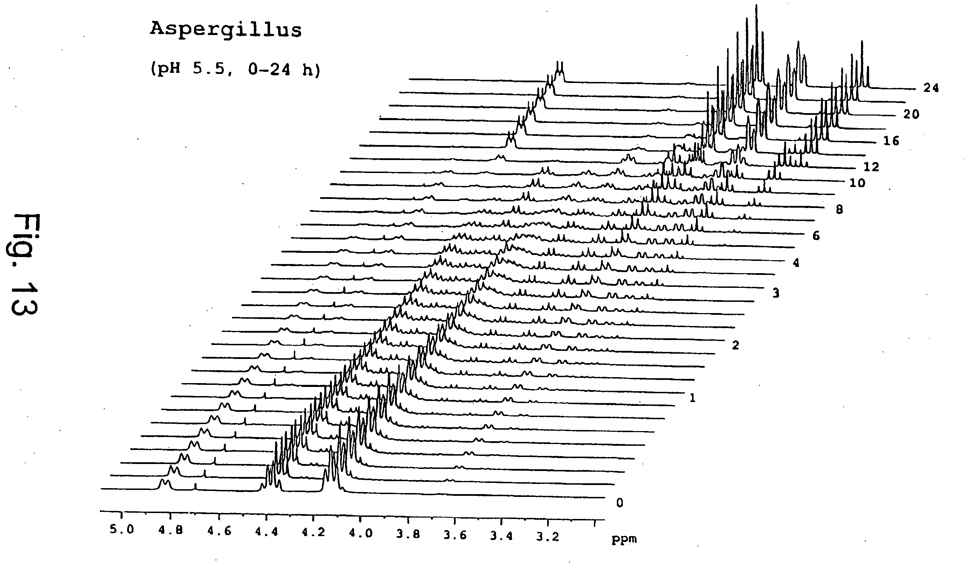

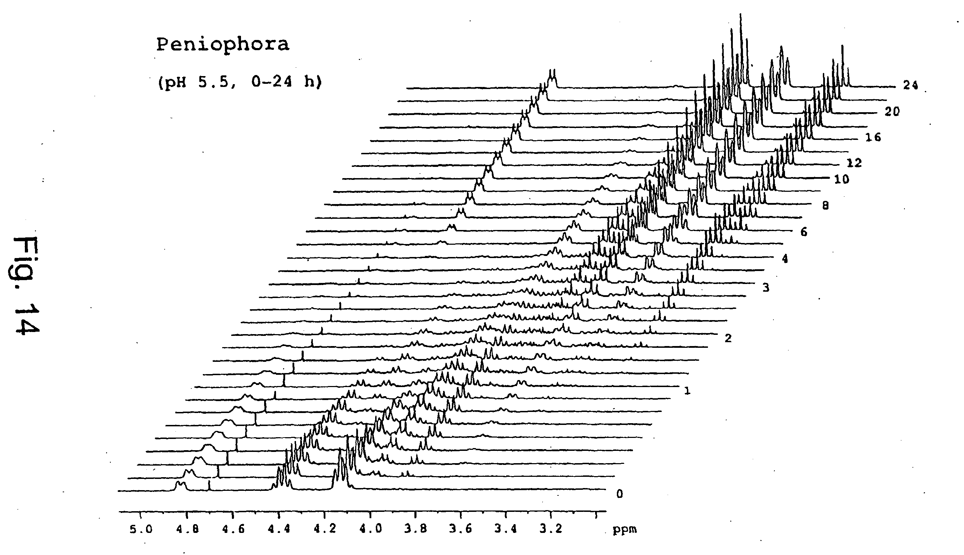

Stacked plots of product profiles for the Aspergillus phytase and the Peniophora phytase covering 24 hours of reaction time at pH 5.5 is presented in Fig. 13 and Fig. 14, respectively.

The signal at $\delta$ 3.25(t) represents H-5 in $Ins(1,2)P_2$ whereas the signal at $\delta$ 3.18(t) represents H-5 in $Ins(2)P$. $Ins(1,2)P_2$ starts accumulating after about 4 hours of reaction time with the Aspergillus phytase and after about 1 hours of reaction time with the Peniophora phytase. $Ins(2)P$ is observed after about 10 hours of reaction with the Aspergillus phytase and after about 3 hours of reaction with the Peniophora phytase. After 24 hours of reaction the amount or level of $Ins(1,2)P_2$ is very low for both phytases, whereas the amount of $Ins(2)P$ is maximum for both phytases after 24 hours.

Accordingly, the profiles observed after 24 hours of reaction time demonstrate that both phytases degrade PA to $Ins(2)P$.

For both enzymes the reaction mixture at 24 h comprised in addition to $Ins(2)P$ minor amounts of $Ins(1,2)P_2$. Prolonged reaction times (several days) resulted in disappearance of the residual $Ins(1,2)P_2$, but the fully dephosphorylated species, inositol (Ins), was not observed at all. The observation is not explained by irreversible inhibition/denaturation of the enzyme, since the enzymes retained their catalytic activities for prolonged periods, as demonstrated by their ability to digest fresh portions of PA added to the NMR tubes after keeping them 5 days at room temperature.

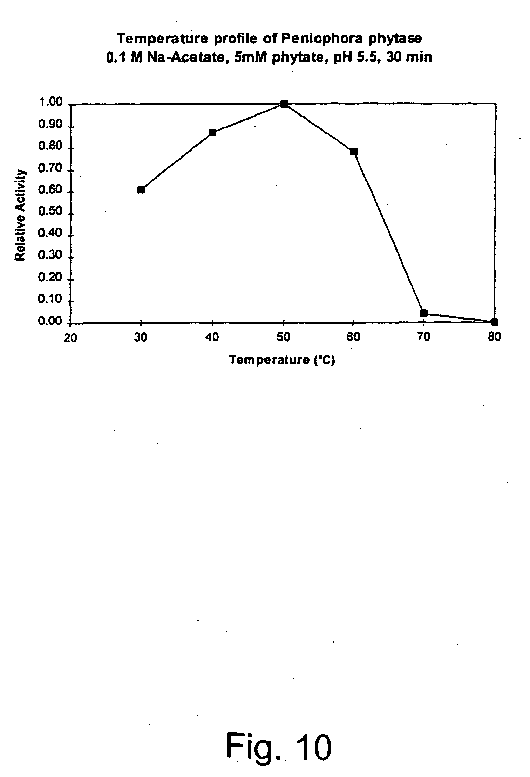

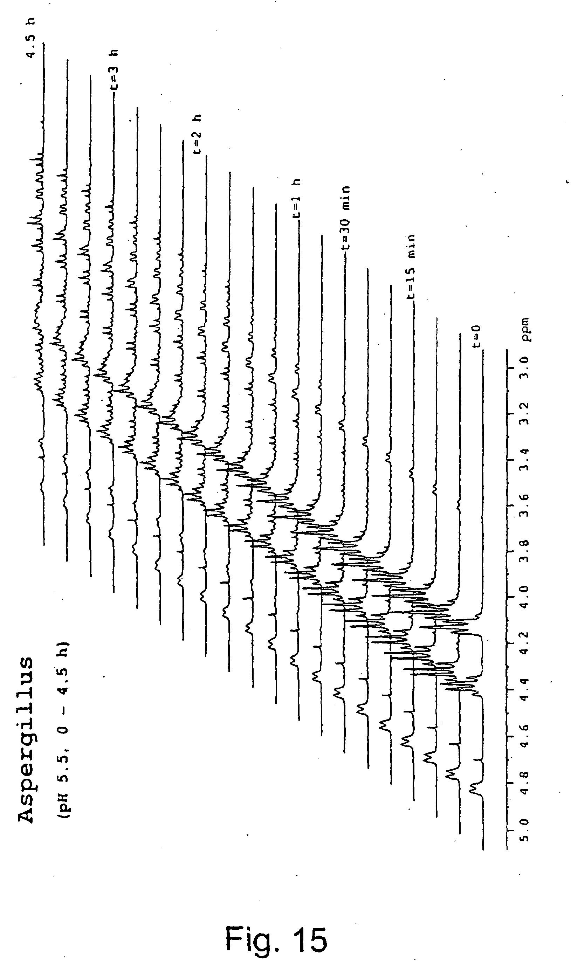

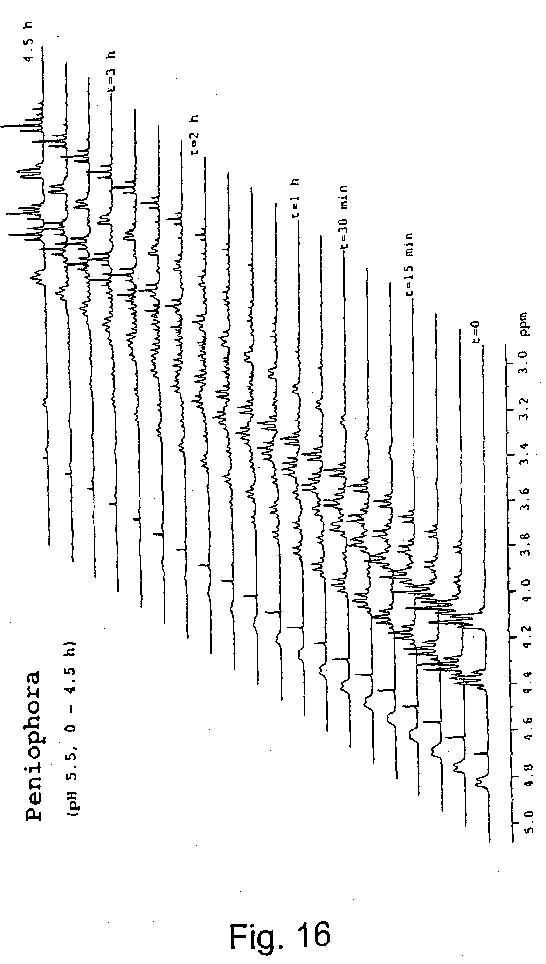

Turning now to figs. 15 and 16, these depict in more detail the profiles evolving at pH 5.5 during the initial 4.5 hours. It is inferred from fig. 10 that H-3 in $Ins(1,2,4,5,6)P_5$ (designated A) shows a signal at $\delta$ 3.66(dd), H-6 in $Ins(1,2,3,4,5)P_5$ (B) a signal at $\delta$ 3.87(t) and H-3 in $Ins(1,2,5,6)P_4$ (C) a signal at $\delta$ 3.56(dd). Now, compound A corresponds to phosphate in position 3 having been hydrolyzed, B position 6 and C position 3 and 4.

It is apparent from fig. 15 that compound A appears as the major primary product (t=5 min) using the Aspergillus phytase, whereas compound B does not appear. Compound C appears after 20-25 minutes.

From fig. 16 (the Peniophora phytase) one infers that compound B appears as the major primary product (t=5min) using the Peniophora phytase.

The signals at $\delta$ 4.82(dt, H-2), 4.38 (q, H-4/H-6), 4.13(q, H-5) and 4.11(dt,H1/H3) are attributable to the substrate, phytic acid, PA. Comparing figs. 15 and 16 it is apparent, that these peaks diminish faster with the Peniophora phytase than with the Aspergillus phytase.

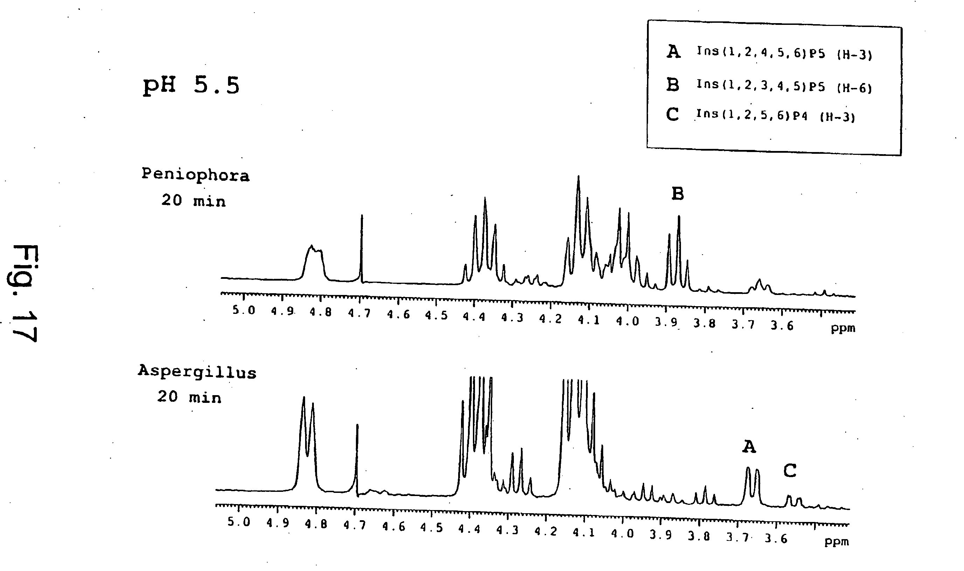

These differences are highlighted in Fig. 17, which present the profiles observed after 20 min at pH 5.5 with the above indicated diagnostic signals (A,B,C) labelled.

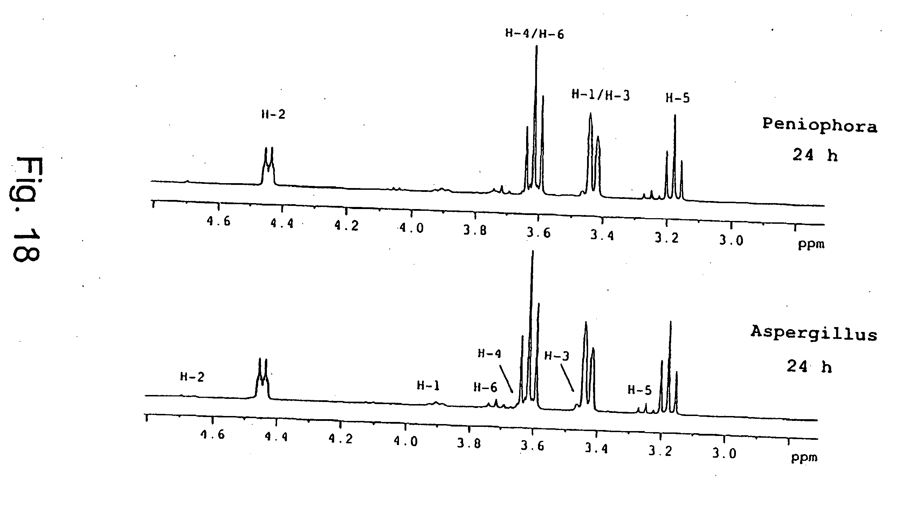

Fig. 18 shows the final result (under these conditions) of the hydrolysis of phytic acid at pH 5.5 (i.e. corresponding to the upper line of figs. 13 and 14). All signals labelled at the upper Peniophora embodiment represent the compound $Ins(2)P$, viz. the protons thereof, from the right to the left: H-5, H1 and H3, H4 and H6 and finally H-2. Relative intensity: 1:2:2:1. The corresponding signals are found in the bottom embodiment of Aspergillus. This means that the end product is in both embodiments $Ins(2)P$. However, a minor amount of $Ins(1,2)P_2$ is also detected in both embodiments, the corresponding peaks being indicated at the Aspergillus embodiment only.

Marked differences are observed:

*Aspergillus*: The initial major product was identified as $Ins(1,2,4,5,6)P_5$ (A) followed by appearance of $Ins(1,2,5,6)P_4$(C), and $Ins(1,2,6)P_3$ (D) (H-3 at $\delta$ 3.49(dd) after 1½ hours) corresponding to consecutive removal of the phosphate groups in the 3-, 4- and 5-positions. The concentration of $Ins(1,2)P_2$ (E) builds up slowly starting at 4 hours and decreases very steeply between 12 and 14 hours with a concomitant rapid increase of the $Ins(2)P$ (F) level. This is visualized in Fig. 11 representing the time dependent concentration of $Ins(1,2)P_2$ and $Ins(2)P$, respectively, determined by measuring the area under the signals corresponding to H-5 in $Ins(1,2)P_2$ ($\delta$ 3.25(t)) and $Ins(2)P$ ($\delta$ 3.18 (t)), respectively, relative to the area under the signals corresponding to the substrates (t=0).

*Peniophora*: At pH 5.5 only the 6-position is initially attacked. A characteristic feature is that PA is digested at a faster rate compared to the Aspergillus phytase. Additional characteristic features are that the end product, Ins(2)P (F) appears very early (3 hours) and builds up slowly, in contrast to the very steep increase in the Ins(2)P-level towards the end of the reaction observed for the Aspergillus phytase.

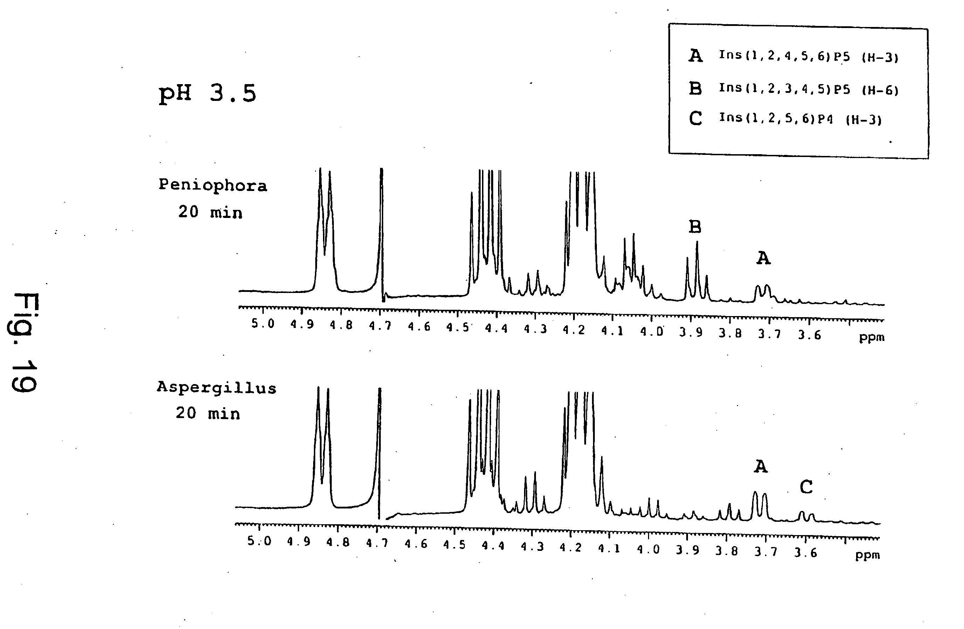

Fig. 19 is a plot similar to fig. 17, but at pH 3.5. Surprisingly, at this pH the Peniophora phytase turns up to have high initial affinity to the 6- as well as the 3-position of PA (B as well as A are observed), probably with a slight preference for the 6-position.

The data generated permit i.a. the following conclusions:

At pH 5.5 as well as 3.5 the Aspergillus phytase attacks with a high degree of selectivity PA in the 3-position, whereas the Peniophora phytase at pH 5.5 with a high degree of selectivity attacks PA in the 6-position, at pH 3.5 however it seems to hydrolyze the phosphate groups at the 3- and 6-positions at comparable rates.

At pH 5.5, the Peniophora phytase digests PA at a faster rate compared to the Aspergillus phytase.

The end-product is, at pH 3.5 as well as 5.5, under the conditions applied, Ins(2)P (F).

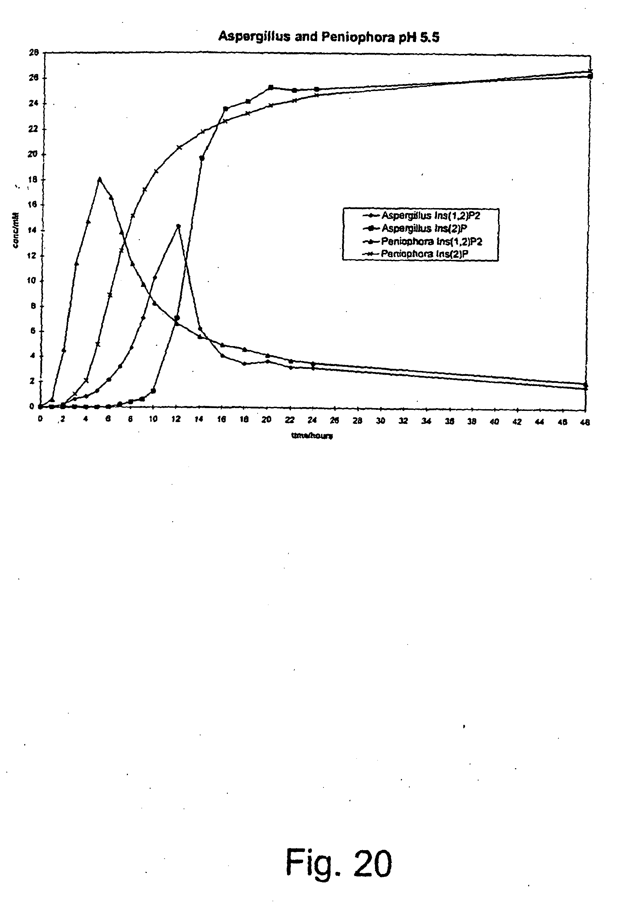

The overall reaction rates (PA→Ins(2)P) were comparable, approximately 20 hours (Fig. 20; pH 5.5).

Accordingly, the Aspergillus phytase prove to be an essentially clean 3-phytase, whereas the Peniophora phytase at pH 5.5 appear to be an essentially clean 6-phytase and at pH 3.5 a phytase of a hitherto unknown type, viz a 3+6-phytase.

The exact configuration of myo-inositol tetrakisphosphate produced by partial hydrolysis af phytic acid with the Peniophora phytase could be determined as outlined below, also allowing us to conclude whether the Peniophora phytase is a D-, L- or a D/L-6-phytase.

Other ways of determining the exact specificity is by determining optical rotation or using a chiral HPLC column.

1. HPLC-isolation of myo-inositol tetrakisphosphate produced by partial degradation of phytic acid with the Peniophora phytase. Desalting (ion exchange, dialysis, (2), (4) and (9) and references herein)
2. NMR analysis to check purity (i), determine whether several diastereomer tetrakisphosphates are produced (ii), and determine which of these are produced (iii)
3. Synthesis of relevant polyols using reduction by boronhydrid (BH) of the corresponding carbonhydrates (10)
4. Disintegration using periodate, reduction by boronhydrid and dephosphorylation following (2). Identification of polyol using HPLC
5. Oxidation of polyol using L-iditol dehydrogenase and final identification of carbonhydrid using HPLC.

References:

(2) Van der Kaay et al, Biochem. J., 312 (1995), 907-910
(4) Irving et al, J. Bacteriology, 112 (1972), 434-438
(9) Stevens, L.R. in "Methods in Inositide Research" (Irvine, R.F. Ed.), 9-30 (1990), Raven Press, Ltd., New York.
(10) Stephens, L. et al, Biochem. J., 249 (1988), 271-282

**Example 12**

**Comparative assay, Aspergillus and Peniophora phytase Release of inorganic phosphate from corn**

The present example gives a simple assay for the phytase catalyzed liberation of phosphorous from corn at pH 3.5 and 5.5. Two parameters have been focused on - velocity and level of P-liberation.

Materials and methods:

Corn was obtained from North Carolina State University (sample No. R27), and ground at a mill (Bühler Universal) at point 6.8.

A corn-suspension (16.7% w/w) was prepared by weighing 20 g of ground corn into a 250 ml blue cap bottle and adding 100 ml of buffer.

The following buffer was used:

pH 5.5: 0.22 M acetate-buffer

The pH value of 3.5 was adjusted by 8N HCl/NaOH.

*Enzymes tested:* Two phytases was tested: A commercial phytase of Aspergillus niger (Phytase Novo®) and a Peniophora phytase of the invention, purified as described in example 2.

*Dosage*: All enzymes were applied at 25 FYT/20 g corn (correspond to 1250 FYT/kg).

The bottles with the corn suspension were closed by caps, and immediately placed in a water bath at 37˚C and subjected to constant stirring. pH was measured at this stage and again after 24 hours. After 30 min of stirring a sample of 5 ml was collected.

Then the phytase enzymes were added at a dosage of 25 FYT/20 g of corn.

Samples were then collected 5, 10, 15, 20, 25, 30, 40, 50, 60 and 120 min after the addition of the phytases, and the content of released P determined as follows:

Phytase containing samples were diluted 1+4 in buffer. Then the samples were centrifuged at 3000 rpm for 5 min, and 1.0 ml of the supernatant was collected. 2.0 ml buffer and 2.0 ml MoV stop solution (cfr. the FYT assay of Example 6) was added. The samples were placed in a refrigerator at 3-5˚C until all samples could be measured at the spectrophotometer at 415nm.

pH was measured at time 0 and 20 hours.

For the determinations a phosphate standard or stock solution of 50mM was used prepared. 0.5, 1.0, 1.5 and 2.0 ml stock solution is diluted to a total volume of 50 ml using buffer. 3.0 ml of each solution is added 2.0 ml MoV stop solution.

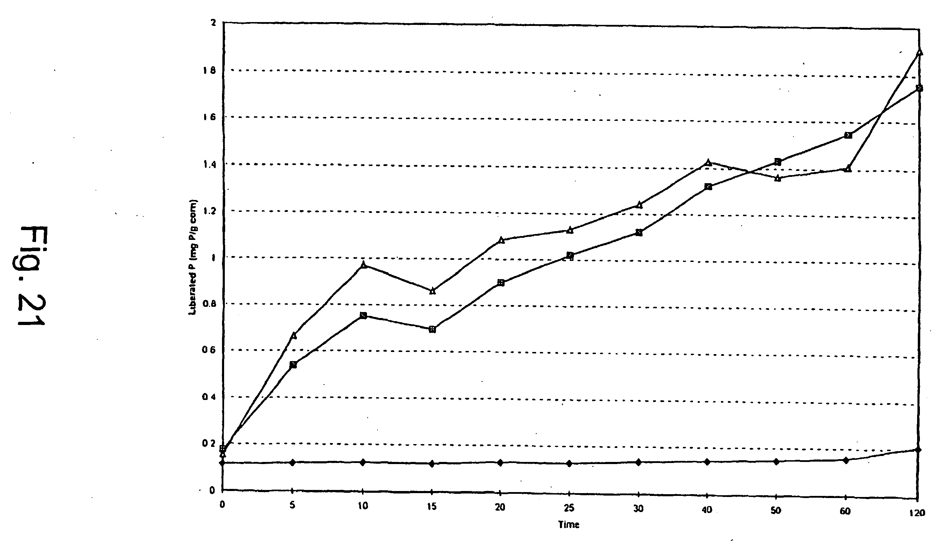

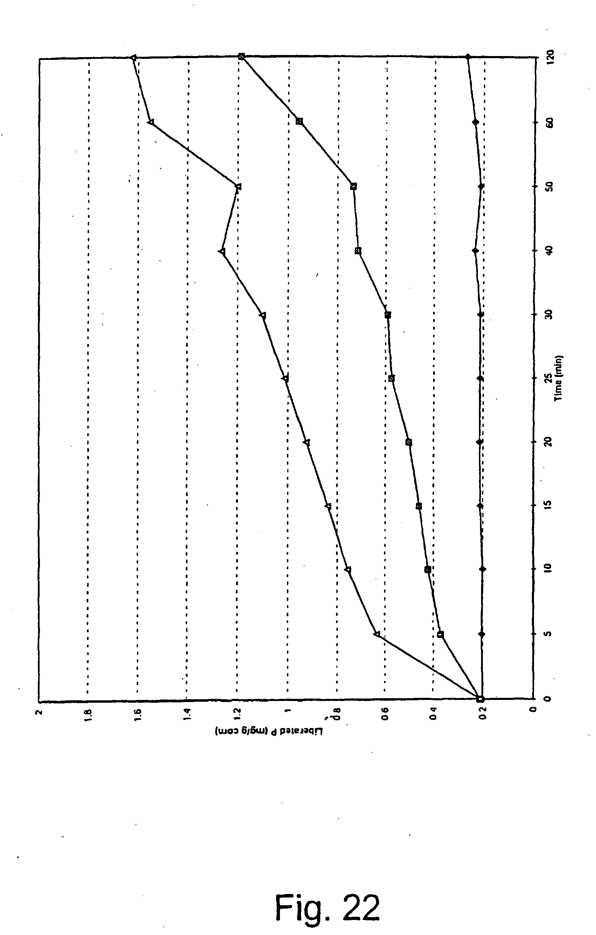

Two experiments were conducted: at pH 5.5 and at pH 3.5. The analysis results are shown at figs. 21 and 22 (pH 5.5 and 3.5, respectively). At these figures, symbol "♦" represents the control experiment, "♦" the Peniophora phytase and "■" the Aspergillus phytase.

Results and discussion:

Fig. 21 (pH 5.5) shows, that at this pH the Peniophora phytase liberates P from corn at significantly improved rate as compared to the Aspergillus phytase.

From fig. 22 (pH 3.5) it is clearly apparent that at this pH the Peniophora phytase is much faster in the liberation of phosphorous from ground corn as compared to the Aspergillus phytase (0-120 minutes).

The passage time of the digestive system of for instance chickens / broilers is normally is of the order of magnitude of 30 minutes to 2 hours, so the observed difference is for sure important, whatever the pH. Nevertheless the pH value of 3.5 is more relevant in this respect than the pH 5.5 value.

This implies that the Peniophora enzyme is surprisingly more efficient than the known Aspergillus phytase as a P-liberator in the digestive system of e.g. broilers.

**Example 13**

**Fermentation, purification and characterization of the phytase of Agrocybe pediades expressed in yeast**

A seed culture is prepared by incubation of the yeast strain in 100 ml medium A at 250 rpm over night at 30˚C. 100 ml medium B is inoculated with 2 ml seed culture and the strains incubate for 7 to 12 days at 30˚C 250 rpm.

*Agrocybe pediades* phytase was expressed in yeast as described in Example 2. The yeast clone comprises a cloned sequence encoding a phytase of the invention having the amino acid sequence shown in SEQ ID No 22.

Filter aid was added to the culture supernatant which was filtered through a filtration cloth. This solution was further filtered through a Seitz depth filter plate resulting in a clear solution. The filtrate was concentrated by ultrafiltration on 3kDa cut-off polyethersulphone membranes and refiltered on a germ filter plate. The pH of the filtrate was adjusted to pH 7.5 and the conductivity was adjusted to 2mS/cm by dilution with distilled water.

The phytase was applied to a Q-sepharose FF column equilibrated with 20mM Tris/$CH_3COOH$, pH 7.5 and the enzyme was eluted with an increasing linear NaCl gradient ($0 \rightarrow 0.5M$). The phytase containing fractions from the Q-sepharose column were pooled and $(NH_4)_2SO_4$ was added to 1.3M final concentration. A Phenyl Toyopearl 650S column was equilibrated with 1.3M $(NH_4)_2SO_4$, 10mM succinic acid/NaOH, pH 6.0 and the phytase was applied to this column and eluted with a decreasing linear $(NH_4)_2SO_4$ gradient ($1.3 \rightarrow 0M$). Phytase containing fractions were pooled and buffer was exchanged with 20mM Tris/$CH_3COOH$, pH 7.5 on a Sephadex G25 column. Phytase was further purified on a SOURCE Q column equilibrated with 20mM Tris/$CH_3COOH$, pH 7.5, and eluted with a linear NaCl gradient ($0 \rightarrow$

0.5M). Finally, phytase containing fractions from the SOURCE Q column were pooled, concentrated on a 10kDa cut-off regenerated cellulose membrane, and applied to a Superdex 200 column equilibrated in 25mM $CH_3COOH/NaOH$, 100mM NaCl, pH 5.0.

Fractions from the Superdex 200 column were analyzed by SDS-PAGE. The phytase migrates in the gel as a very broad and diffuse band with approx. Mr = 150 kDa indicating that the enzyme was highly glycosylated.

N-terminal amino acid sequencing of the 150 kDa component was carried out following SDS-PAGE and elctroblotting onto a PVDF membrane.

Two N-terminal sequences could be deduced in the relative amounts of approximately 4:1 (upper sequence: lower sequence):

```
Val-Gln-Pro-Phe-Phe-Pro-Pro-Gln-Ile-Gln-Asp-Ser-Trp-Ala-Ala-
Tyr-Thr-Pro-Tyr-Tyr-Pro-Val-Gln-
```

and

```
Thr-Phe-Val-Gln-Pro-Phe-Phe-Pro-Pro-Gln-Ile-Gln-Asp-Ser-Trp-
Ala-Ala-Tyr-Thr-Pro-Tyr-Tyr-Pro-
```

The two N-terminal amino acids "Val" and "Thr" are found in position 27 and 25, respectively, in SEQ ID NO 22. This indicates that the mature phytase enzyme of the invention, when expressed in yeast, starts at position 27 or 25 in SEQ ID No 22.

Accordingly the mature amino acid sequence of the phytase when expressed in yeast is supposed to be no. 27-453 or 25-453 of SEQ ID no 22.

## EXAMPLE 14

### Purification and characterization of the phytase from Agrocybe pediades expressed in Aspergillus oryzae

The *Agrocybe pediades* phytase was expressed in and excreted from *Aspergillus oryzae* IFO 4177.

Filter aid was added to the culture broth which was filtered through a filtration cloth. This solution was further filtered through a Seitz depth filter plate resulting in a clear solution. The filtrate was concentrated by ultrafiltration on 3kDa cut-off polyethersulphone membranes followed by diafiltration with distilled water to reduce the conductivity. The pH of the concentrated enzyme was adjusted to pH 7.5.

The phytase was applied to a Q-sepharose FF column equilibrated in 20mM $Tris/CH_3COOH$, pH 7.5 and the enzyme was eluted with an increasing linear NaCl gradient ($0 \rightarrow 0.5M$). The phytase activity eluted as a single peak. This peak was pooled and $(NH_4)_2SO_4$ was added to 1.3M final concentration. A Phenyl Toyopearl 650S column was equilibrated in 1.3M $(NH_4)_2SO_4$, 10mM succinic acid/NaOH, pH 6.0 and the phytase was applied to this column and eluted with a decreasing linear $(NH_4)_2SO_4$ gradient ($1.3 \rightarrow 0M$). Phytase containing fractions were pooled and the buffer was exchanged for 20mM $Tris/CH_3COOH$, pH 7.5 by dialysis. The phytase was applied to a SOURCE 30Q column equilibrated in 20mM $Tris/CH_3COOH$, pH 7.5 and the enzyme was eluted with an increasing linear NaCl gradient ($0 \rightarrow 0.25M$). The phytase activity was pooled and $(NH_4)_2SO_4$ was added to 1.6M final concentration. A SOURCE Phenyl column was equilibrated in 1.6M $(NH_4)_2SO_4$, 25mM succinic acid/NaOH, pH 6.0 and the phytase was applied to this column and eluted with a decreasing linear $(NH_4)_2SO_4$ gradient ($1.6 \rightarrow 0M$). Fractions from the SOURCE Phenyl column were analyzed by SDS-PAGE and pure phytase fractions were pooled. The phytase pool was dialysed against 20mM $Tris/CH_3COOH$, pH 7.5, applied to a HighTrap Q column equilibrated in the same buffer, and stepeluted with 20mM $Tris/CH_3COOH$, 0.5M NaCl, pH 7.5.

The *Agrocybe* phytase migrates on SDS-PAGE as a band with Mr = 60 kDa.

N-terminal amino acid sequencing of the 60 kDa component was carried out following SDS-PAGE and electroblotting onto a PVDF-membrane. Two N-terminal amino acid sequences could be deduced in relative amounts of approximately 2:1 (upper sequence:lower sequence).

```
Phe-Pro-Pro-Gln-Ile-Gln-Asp-Ser-Trp-Ala-Ala-Tyr-Thr-Pro-Tyr-
Tyr-Pro-Val-Gln-
```

and

```
Gln-Pro-Phe-Phe-Pro-Pro-Gln-Ile-Gln-Asp-Ser-Trp-Ala-Ala-Tyr-
Thr-Pro-Tyr-Tyr-
```

The upper sequence corresponds to amino acid residues 31-49 in the cDNA derived amino acid sequence while the lower sequence corresponds to amino acid residues 28-46.

Accordingly the mature amino acid sequence of the phytase when expressed in Aspergillus is supposed to be no. 31-453 or 28-453 of SEQ ID no 22.

Accordingly, summing up the results of example 13 and the present example, in SEQ ID NO 21, the following sequences are phytase encoding sub-sequences: position 79 to 1362, 73-1362, 91-1362 or 82-1362 (i.e. corresponding to amino acid positions 27-453, 25-453, 31-453 or 28-453, respectively, in SEQ ID NO 22).

Accordingly, there is a slight variability in the N-terminal sequence of the mature phytase enzyme. This variability is observed as well when the enzyme is expressed in a single strain, as when expressed in different strains. In yeast, the mature phytase enzyme starts at amino acid no. 27 or 25 (relative abundance about 80%:20%, respectively); in Aspergillus the mature phytase enzyme starts at amino acid no. 31 or 28 (relative abundance: about 65%:35%, respectively).

**Example 15**

**Characterization of the purified phytase of Agrocybe pediades**

The phytase of Agrocybe pediades was expressed in Aspergillus and purified as described in Example 14.

The phytase activity is measured using the following assay:

10 $\mu$l diluted enzyme samples (diluted in 0.1 M sodium acetate, 0.01 % Tween20, pH 5.5) were added into 250 $\mu$l 5 mM sodium phytate (Sigma) in 0.1 M sodium acetate, 0.01 % Tween20, pH 5.5 (pH adjusted after dissolving the sodium phytate; the substrate was preheated) and incubated for 30 minutes at 37 °C. The reaction was stopped by adding 250 $\mu$l 10 % TCA and free phosphate was measured by adding 500 $\mu$l 7.3 g $FeSO_4$ in 100 ml molybdate reagent (2.5 g $(NH_4)_6Mo_7O_{24} \cdot 4H_2O$ in 8 ml $H_2SO_4$ diluted to 250 ml). The absorbance at 750 nm was measured on 200 $\mu$l samples in 96 well microtiter plates. Substrate and enzyme blanks were included. A phosphate standard curve was also included (0-2 mM phosphate). 1 FYT equals the amount of enzyme that releases 1 $\mu$mol phosphate/min at the given conditions.

Temperature profiles were obtained by running the assay at various temperatures (preheating the substrate).

Temperature stability was investigated by preincubating the phytases in 0.1 M sodium phosphate, pH 5.5 at various temperatures before measuring the residual activity.

The pH-stability was measured by incubating the enzyme at pH 3 (25 mM glycine-HCl), pH 4-5 (25 mM sodium acetate), pH 6 (25 mM MES), pH 7-9 (25 mM Tris-HCl) for 1 hour at 40 °C, before measuring the residual activity.

The pH-profiles were obtained by running the assay at the various pH using the same buffer-systems (50 mM, pH was readjusted when dissolving the substrate).

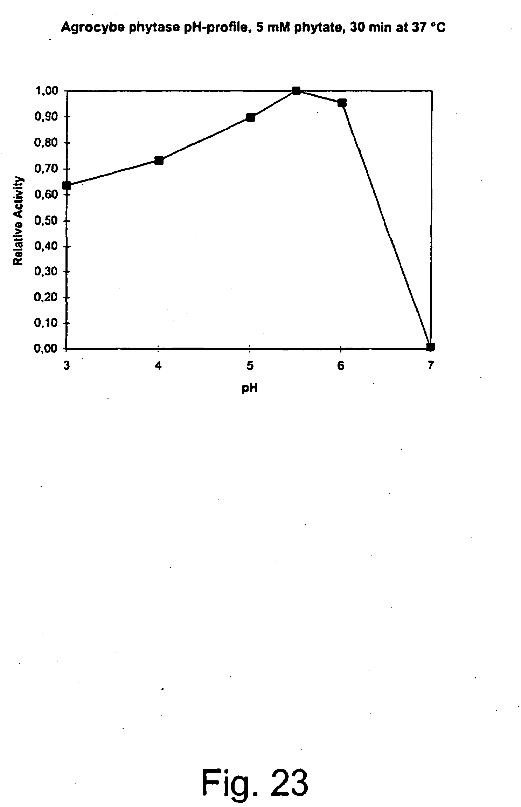

The results of the above pH-profile, pH-stability, temperature-profile and temperature stability studies are shown in fig. 23, 24, 25 and 26, respectively.

From fig. 23 it appears that the phytase of Agrocybe pediades has a reasonable activity at pH 3-6 (i.e. more than 50% of the maximum activity). At pH 4-6 more than 70% of the maximum activity is found, at pH 5-6 more than 90%. Optimum pH seems to be in the area of pH 5.5-6.

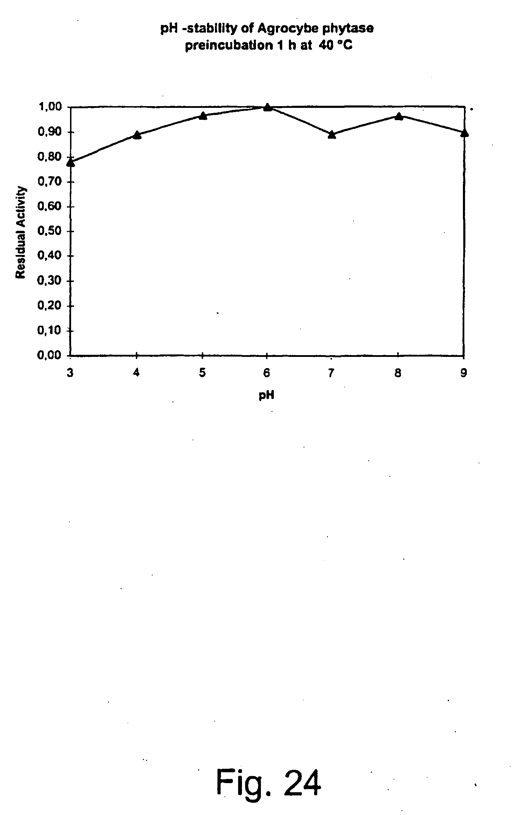

It is apparent from fig. 24 that the phytase of Agrocybe pediades is very stable (i.e. more than 80% of the maximum activity retained) for 1 hour at 40°C in the whole range of pH 3-9.

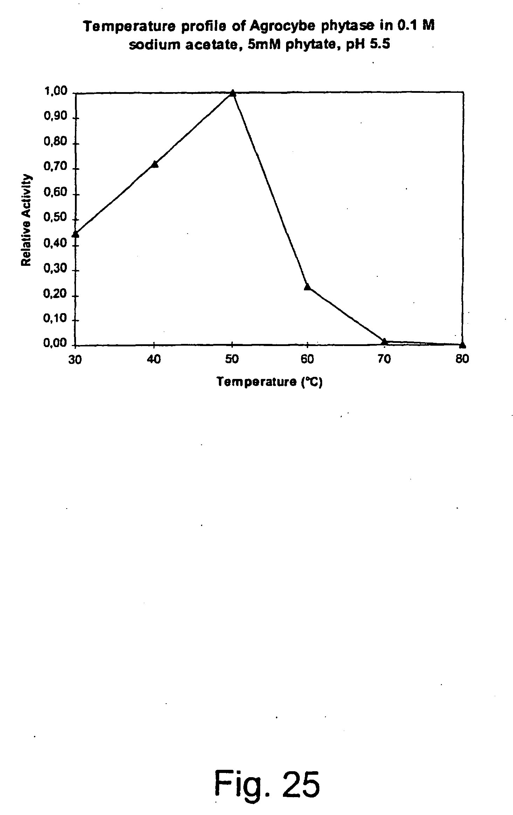

As regards the temperature profile, it is apparent from fig. 25, that the Agrocybe pediades phytase has a reasonable activity at temperatures of 35-55°C (i.e. more than 60% of the maximum activity), whereas at temperatures of 40-52˚C the activity is more than 70% of the maximum activity, and the optimum temperature is close to 50˚C.

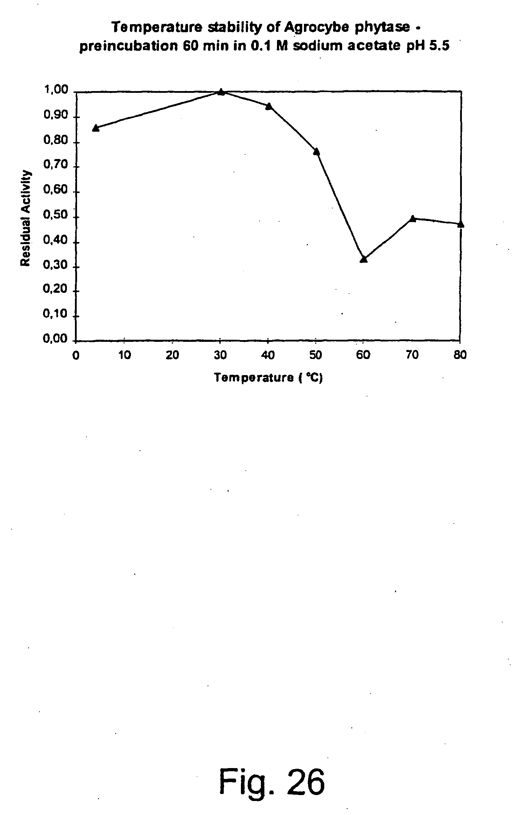

And finally, as regards the temperature stability results shown at fig. 26, the phytase is very stable at temperatures of 0 to about 55˚C (i.e. more than 60% residual activity). A sharp decline in residual activity is seen after preincubation at 60˚C. Anyhow, at 60˚C at least 20%, preferably 25% and more preferably 30% of the residual activity still remains. Also at pre-incubation temperature above 60˚C, e.g. at 70˚C and 80˚C, a surprisingly high residual activity remains, viz. more than 20%, preferably more than 30%, especially more than 40% remains.

This fact is contemplated to be due to the enzyme being surprisingly capable of refolding following its thermal denaturation. The degree of refolding will depend on the exact conditions (pH, enzyme concentration).

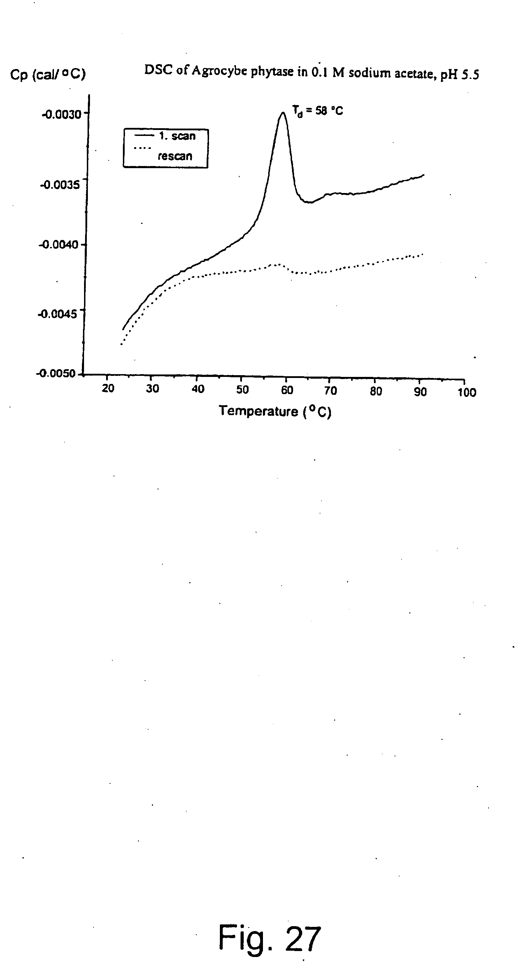

Fig. 27 shows the result of differential scanning calorimetry (DSC) measurements on the Agrocybe phytase.

In DSC the heat consumed to keep a constant temperature increase in the sample-cell is measured relative to a reference cell. A constant heating rate is kept (e.g. 90˚C/hour). An endothermal process (heat consuming process - e.g. the unfolding of an enzyme/protein) is observed as an increase in the heat transferred to the cell in order to keep the constant temperature increase.

DSC was performed using the MC2-apparatus from MicroCal. Cells were equilibrated 20 minutes at 20˚C before scanning to 90˚C at a scan rate of 90˚/h. Samples of around 2.5 mg/ml *Agrocybe* phytase in 0.1 M sodium acetate, pH 5.5 were loaded.

The temperature stability studies were confirmed by DSC, since from fig. 5 it is apparent that the *Agrocybe* phytase has a denaturation or "melting" temperature of about 58˚C at pH 5.5. The re-scan of the *Agrocybe* phytase shows a minor peak at 58˚C, and this is also indicative of the fact that a fraction of the enzyme is actually refolded folding the thermal inactivation in the first scan.

**Example 16**

**Time-resolved product-profiling of phytase-catalyzed hydrolysis of phytic acid by $^1$H NMR spectroscopy**

The hydrolysis of phytic acid (PA) catalyzed by the Agrocybe phytase and by a commercial Aspergillus niger phytase (Phytase Novo®) was investigated (27 mM phytate, 1 FYT/ml, pH 5.5, and 27˚C) by $^1$H NMR profiling the product mixture in the course of 24 hours.

In the following (Ins(p,q,r,..)$P_n$ denotes myo-inositol carrying in total n phosphate groups attached to positions p, q, r,.. For convenience Ins(1,2,3,4,5,6)$P_6$ (phytic acid) is abbreviated PA. Please refer, however, to the section "Nomenclature and position specificity of phytases" in the general part of this application.

The technique provide specific information about initial points of attack by the enzyme on the PA molecule, as well as information about the identity of the end product. On the other side the evolving patterns of peaks reflecting the composition of the intermediate product mixtures, provide a qualitative measure, a finger print, suitable for identification of similarities and differences between individual enzymes.

NMR, like most other analytical methods, can distinguish between stereo-isomers which are not mirror images (diastereomers), but not between a set of isomers, which are mirror-images (enantiomers), since they exhibit identical NMR spectra.

Thus, Ins(1,2,4,5,6)$P_5$ (3-phosphate removed) exhibits a NMR spectrum different from Ins(1,2,3,4,5)$P_5$ (6-phosphate removed) because the isomers are diastereomers.

However, the NMR spectra of Ins (1, 2, 4, 5, 6) $P_5$ and Ins (2, 3, 4, 5, 6) $P_5$ (1-phosphate removed) are identical because the isomers are enantiomers. The same holds for the pair Ins(1,2,3,4,5)$P_5$ and Ins(1,2,3,5,6)$P_5$ (4-phosphate removed).

Thus, by NMR it is not possible to distinguish between a 3-and a 1-phytase, and it is not possible to distinguish between a 6- and a 4-phytase (or a L-6- and a D-6-phytase using the lowest-locant rule).

Biased by the description of 3- and 6-phytases in the literature, we have used the terms 3- and 6-phytases for our enzymes, but, though unlikely, we do not actually know if we have a 1- and a 4-phytase instead.

Experimental.

NMR spectra were recorded at 300 K (27˚C) on a Bruker DRX400 instrument equipped with a 5 mm selective inverse probe head. 16 scans preceded by 4 dummy scans were accumulated using a sweep width of 2003 Hz (5 ppm) covered by 8 K data points. Attenuation of the residual HOD resonance was achieved by a 3 seconds presaturation period. The spectra were referenced to the HOD signal ($\delta$ 4.70).

PA samples for NMR analysis were prepared as follows: PA (100 mg, Phytic acid dipotassium salt, Sigma P-5681) was dissolved in deionized water (4.0 ml) and pH adjusted to 5.5 by addition of aqueous NaOH (4 N). Deionized water was added (ad 5 ml) and 1 ml portions, each corresponding to 20 mg of phytic acid, were transferred to screw-cap vials and the solvent evaporated (vacuum centrifuge). The dry samples were dissolved in deuterium oxide (2 ml, Merck 99.5% D) and again evaporated to dryness (stored at -18˚C until use).

For NMR analysis one 20 mg phytic acid sample was dissolved in deuterium oxide (1.0 ml, Merck 99.95% D). The solution was transferred to a NMR tube and the $^1$H NMR spectrum recorded. Enzyme solution (1 FTU, dissolved in/diluted, as appropriate, with deuterium oxide) was added followed by thorough mixing (1 minute). $^1$H NMR spectra were recorded immediately after addition of enzyme (t=0), then after 5, 10, 15, 20, 25, 30, 45, 60, 75, 90, 105, 120, 135 150, 165, 180, 195, 210 minutes (= 3.5 hours), 4.5, 5.5 6.5, 7.5, 8.5, 9.5, 11.5, 13.5, 15.5, 17.5, 19.5, 21.5, and 23.5 hours. The pH in the NMR tube was measured. Additional spectra were acquired after 48 and 120 hours (5 days), where a portion of substrate (PA, 6 mg) was added to probe if the enzyme retained its catalytic activity.

By means of 2D NMR analysis of inositol phosphate mixtures obtained by partial digestion of PA, in conjunction with published NMR data (Scholz, P.; Bergmann, G., and Mayr, G.W.: Methods in Inositide Research (Ed. Irvine, R.F.), pp. 65-82, Raven Press, Ltd., New York (1990)), characteristic $^1$H NMR signals attributable to Ins (1, 2, 3, 4, 5, 6) $P_6$ (PA), Ins (1, 2, 4, 5, 6) $P_5$, Ins (1, 2, 3, 4, 5) $P_5$, Ins(1,2,5,6)$P_4$, Ins(1,2,6)$P_3$, Ins(1,2)$P_2$, and Ins(2)P, were identified and permitted relative quantification of these species during the course of the reaction.

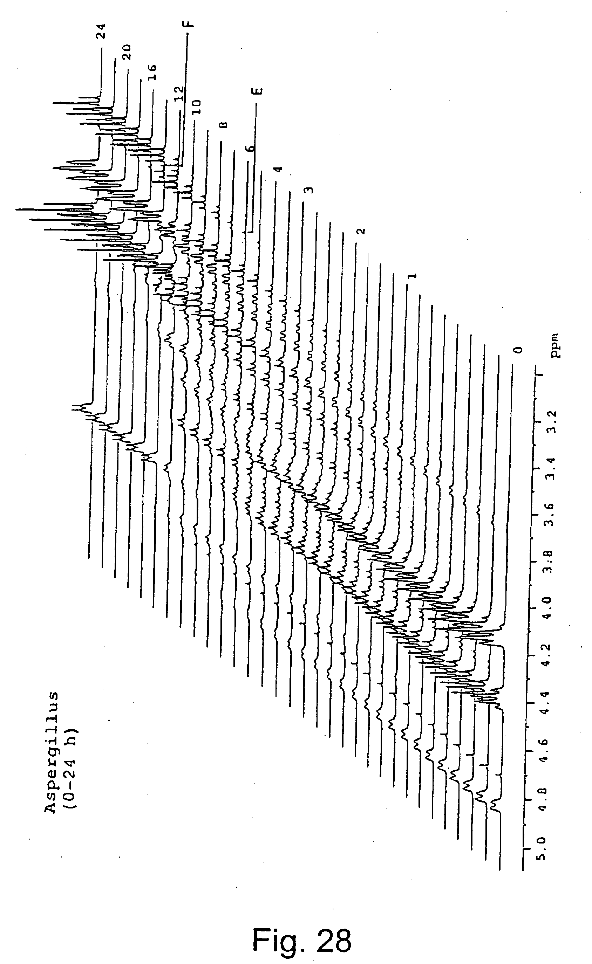

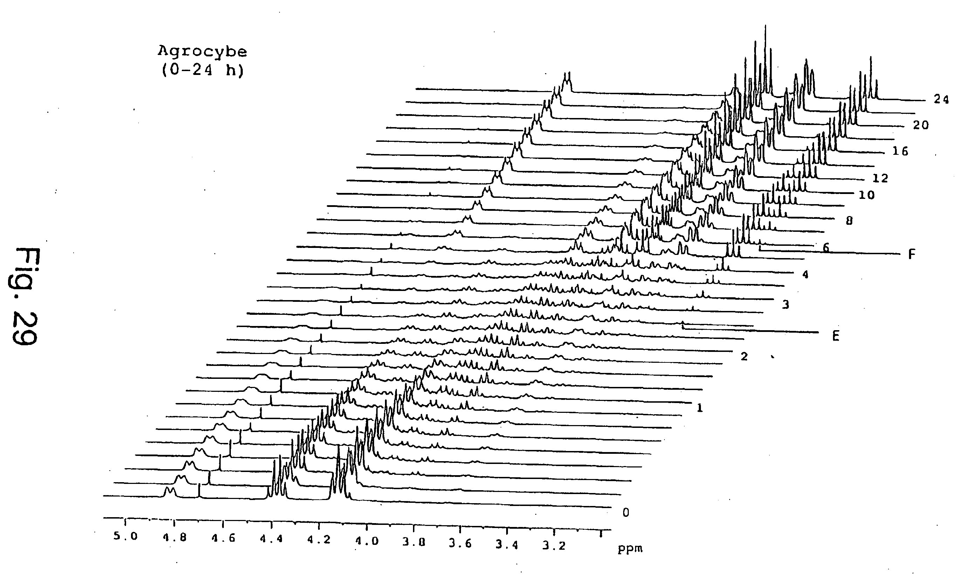

Stacked plots of product profiles for the Aspergillus phytase and the Agrocybe phytase covering 24 hours of reaction time is presented in Fig. 28 and Fig. 29, respectively.

The signal at $\delta$ 3.25(t) represents H-5 in Ins(1,2)$P_2$ whereas the signal at $\delta$ 3.18(t) represents H-5 in Ins(2)P. Ins(1,2)$P_2$ starts accumulating after about 4 hours of reaction time with the Aspergillus phytase and after about 2 hours of reaction time with the Agrocybe phytase. Ins(2)P is observed after about 10 hours of reaction with the Aspergillus phytase and after about 5 hours of reaction with the Agrocybe phytase. After 24 hours of reaction the amount or level of Ins(1,2)$P_2$ is very low for both phytases, whereas the amount of Ins(2)P is maximum for both phytases after 24 hours.

Accordingly, the profiles observed after 24 hours of reaction time demonstrate that both phytases degrade PA to Ins(2)P. The fully dephosphorylated species, inositol (Ins), was not observed at all.

For both enzymes the reaction mixture at 24 h comprised in addition to Ins(2)P minor amounts of Ins(1,2)$P_2$. Prolonged reaction times (several days) resulted in disappearance of the residual Ins(1,2)$P_2$, but the fully dephospho-rylated species, inositol (Ins), was not observed at all. The observation is not explained by irreversible inhibition/denaturation of the enzyme, since the enzymes retained their catalytic activities for prolonged periods, as demonstrated by their ability to digest fresh portions of PA added to the NMR tubes after keeping them 5 days at room temperature.

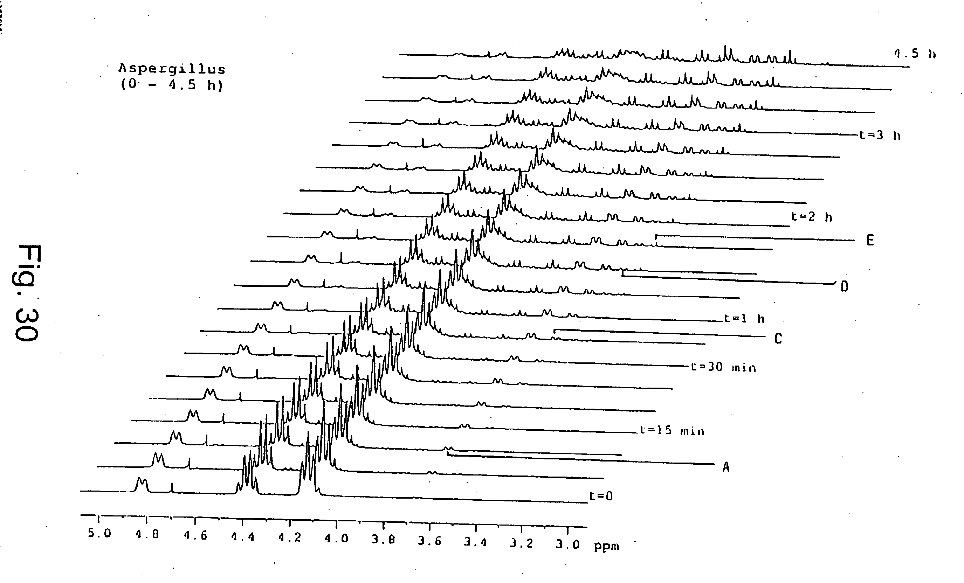

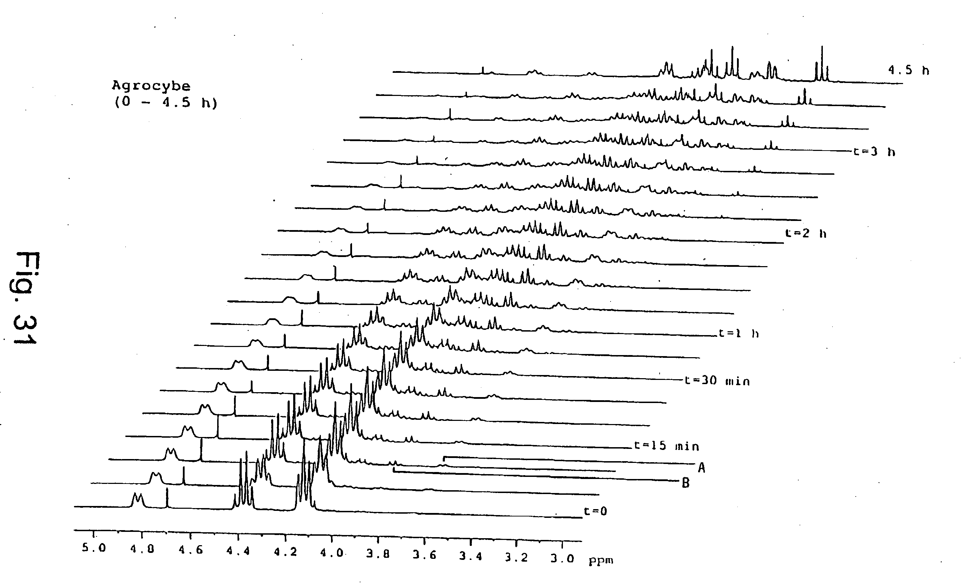

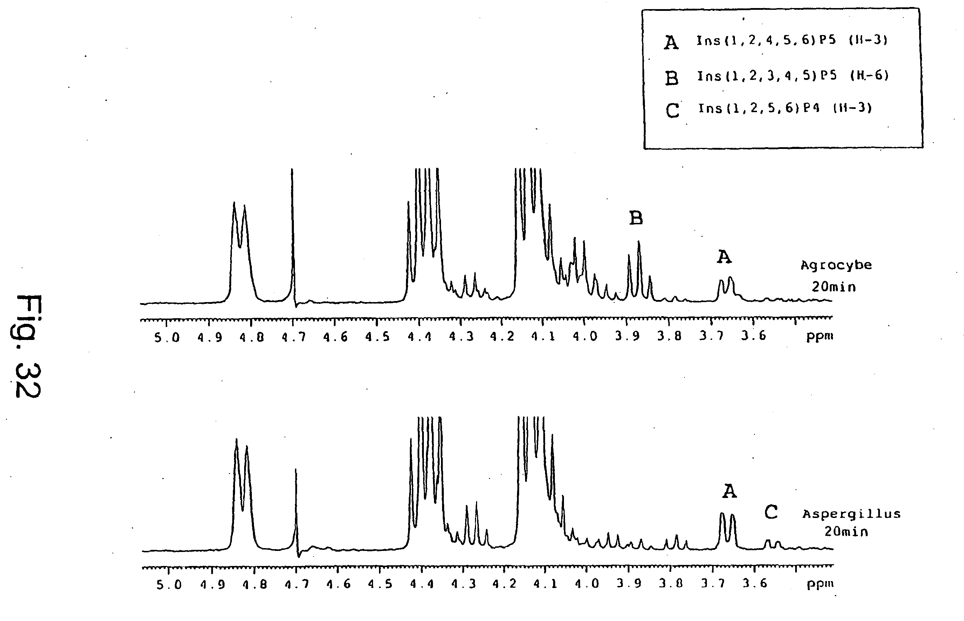

Turning now to figs. 30 and 31, these depict in more detail the profiles evolving during the initial 4.5 hours. It is inferred from fig. 32 that H-3 in Ins(1,2,4,5,6)$P_5$ (designated A) shows a signal at $\delta$ 3.66(dd), H-6 in Ins(1,2,3,4,5)$P_5$ (B) a signal at $\delta$ 3.87(t) and H-3 in Ins(1,2,5,6)$P_4$ (C) a signal at $\delta$ 3.56(dd). Now, compound A corresponds to phosphate in position 3 having been hydrolyzed, B position 6 and C position 3 and 4.

It is apparent from fig. 30 that compound A appears as the major primary product (t=5 min) using the Aspergillus phytase, whereas compound B does not appear. Compound C appears after 20-25 minutes.

From fig. 31 (the Agrocybe phytase) one infers that compound A as well as compound B appear very early, i.e. within the first 15 minutes, probably more of the compound B than A.

The signals at $\delta$ 4.82(dt, H-2), 4.38 (q, H-4/H-6), 4.13(q, H-5) and 4.11(dt,H1/H3 are attributable to the substrate, phytic acid, PA. Comparing figs. 30 and 31 it is apparent, that these peaks diminish much faster (i.e. within an hour) with the Agrocybe phytase than with the Aspergillus phytase.

These differences are highlighted in Fig. 32, which present the profiles observed after 20 min with the above indicated diagnostic signals (A,B,C) labelled.

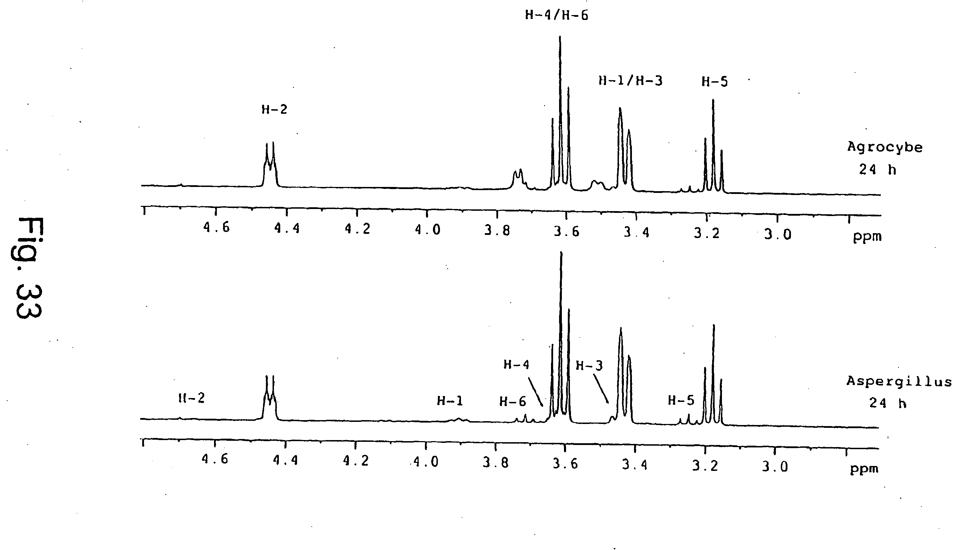

Fig. 33 shows the final result (under these conditions) of the hydrolysis of phytic acid (i.e. corresponding to the upper line of figs. 28 and 29). All signals labelled at the upper Agrocybe embodiment represent the compound Ins(2)P, viz. the protons thereof, from the right to the left: H-5, H1 and H3, H4 and H6 and finally H-2. Relative intensity: 1:2:2: 1. The corresponding signals are found in the bottom embodiment of Aspergillus. This means that the end product is in both embodiments Ins(2)P. However, a minor amount of Ins(1,2)$P_2$ is also detected in both embodiments, the corresponding peaks being indicated at the Aspergillus embodiment only.

Marked differences are observed:

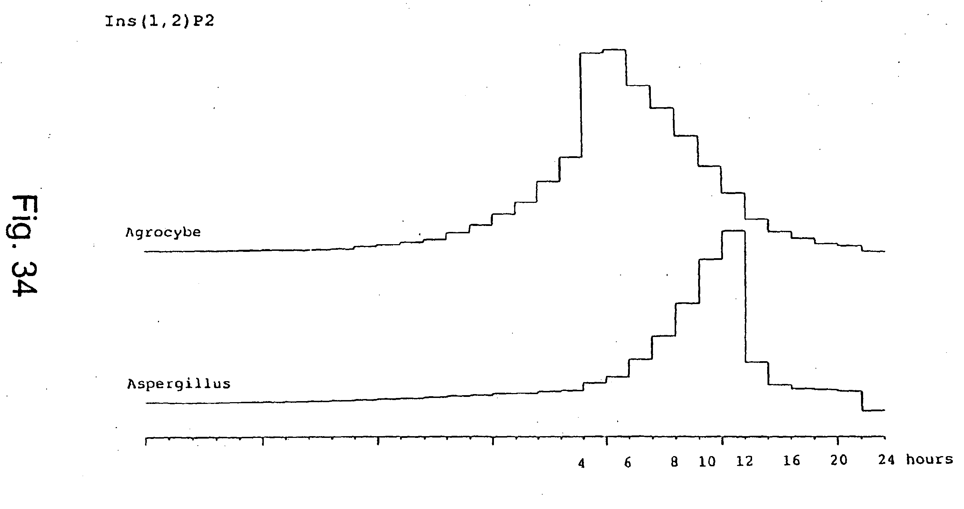

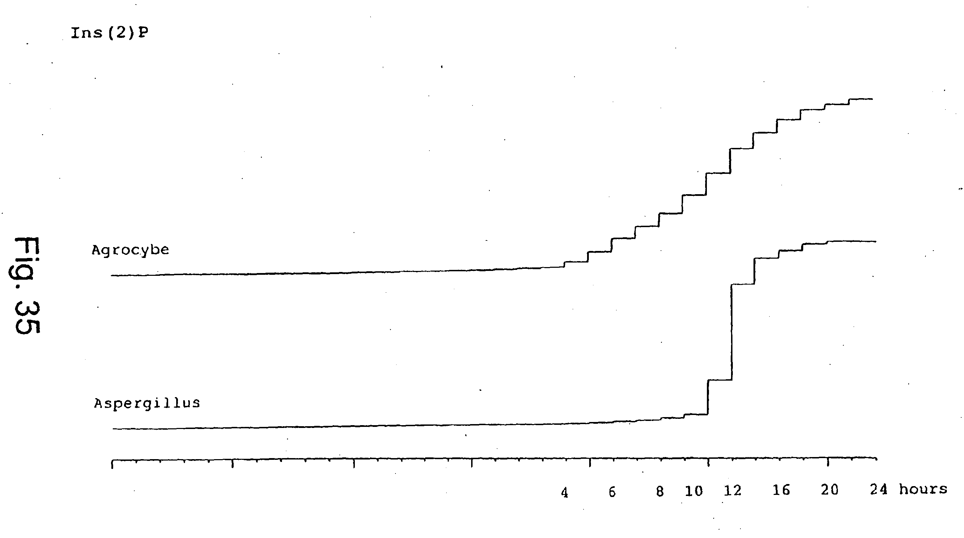

*Aspergillus*: The initial major product was identified as Ins(1,2,4,5,6)$P_5$ (A) followed by appearance of Ins (1,2,5,6)$P_4$(C), and Ins(1,2,6)$P_3$ (D) (H-3 at $\delta$ 3.49(dd) after 1½ hours) corresponding to consecutive removal of the phosphate groups in the 3-, 4- and 5-positions. The concentration of Ins(1,2)$P_2$ (E) builds up slowly starting at 2 hours and decreases very steeply between 12 and 14 hours with a concomitant rapid increase of the Ins(2)P (F) level. This is visualized in Fig. 34 and 35, representing the time dependent concentration of Ins(1,2)$P_2$ and Ins(2)P, respectively, constructed from slices along the time-dimension in Fig. 28-29 at the chemical shift values ($\delta$) of H-5 in Ins(1,2)$P_2$ and Ins(2)P, respectively (note that the time scale is only linear in segments).

*Agrocybe*: Both the 3- and 6-positions are initially attacked,

with some preference for the 6-position. A characteristic feature is that PA is digested at a faster rate compared to the Aspergillus phytase. Additional characteristic features are that the end product, Ins(2)P (F) appear very early (5 hours) and builds up slowly, in contrast to the very steep increase in the Ins(2)P-level towards the end of the reaction observed for the Aspergillus phytase.

The data generated permit i.a. the following conclusions:

The Aspergillus phytase attacks with a high degree of selectivity PA in the 3-position, whereas the Agrocybe phytase appear less specific.

The Agrocybe phytase digests PA at a faster rate compared to the Aspergillus phytase.

The end-product is in both cases, under the conditions applied, Ins (2) P (F).

The overall reaction rates (PA→Ins(2)P) were comparable, approximately 20 hours (Fig. 35).

Accordingly, the Aspergillus phytase prove to be an essentially clean 3-phytase, whereas the Agrocybe phytase appear to be less specific, however, with some preference for the 6-position.

By application of 2D-homo- and heteronuclear ($^1$H, $^{13}$C) correlation techniques, the latter circumventing problems with severely overlapping $^1$H-resonances by taking advantage of the larger chemical shift dispersion of the $^{13}$C-nuclei , in combination with suitable computer software, it would in principle be possible to identify and quantify intermediates present at any given time and thereby completely map out the reaction sequence. In other words, curves like those shown in Fig. 34 and 35 representing concentration as a function of time, could in theory be constructed for other intermediate inositol phosphates.

### Example 17

### Comparative assay, Aspergillus and Agrocybe phytase

### Release of inorganic phosphate from corn

The present example gives a simple assay for the phytase catalyzed liberation of phosphorous from corn at pH 3.5 and 5.5. Two parameters have been focused on - velocity and level of P-liberation.

<u>Materials and methods:</u>

Corn was obtained from North Carolina State University (sample No. R27), and ground at a mill (Bühler Universal) at point 6.8.

A corn-suspension (16.7% w/w) was prepared by weighing 20 g of ground corn into a 250 ml blue cap bottle and adding 100 ml of buffer.

The following buffers were used:

pH 5.5: 0.22 M acetate-buffer
pH 3.5: 0.05 M citrate-buffer.

*Enzymes tested:* Two phytases was tested: A commercial phytase of Aspergillus niger (Phytase Novo®) and an Agrocybe phytase of the invention, purified as described in example 3 and 4.

*Dosage*: All enzymes were applied at 25 FYT/20 g corn (correspond to 1250 FYT/kg).

The bottles with the corn suspension were closed by caps, and immediately placed in a water bath at 37°C and subjected to constant stirring. pH was measured at this stage and again after 24 hours. After 30 min of stirring a sample of 5 ml was collected.

Then the phytase enzymes were added at a dosage of 25 FYT/20 g of corn.

Samples were then collected 10, 20, 30, 40, 50, 60, 120 min, and approx. 20 hours after the addition of the phytases, and the content of release P determined as follows:

Phytase containing samples were diluted 1+4 in buffer. Then the samples were centrifuged at 3000 rpm for 5 min, and 1.0 ml of the supernatant was collected. 2.0 ml buffer and 2.0 ml MoV stop solution (cfr. the FYT assay of Example 15) was added. The samples were placed in a refrigerator at 3-5°C until all samples could be measured at the spectrophotometer at 415nm.

pH was measured at time 0 and 20 hours.

For the determinations a phosphate standard or stock solution of 50mM was used prepared. 0.5, 1.0, 1.5 and 2.0 ml stock solution is diluted to a total volume of 50 ml using buffer. 3.0 ml of each solution is added 2.0 ml MoV stop solution.

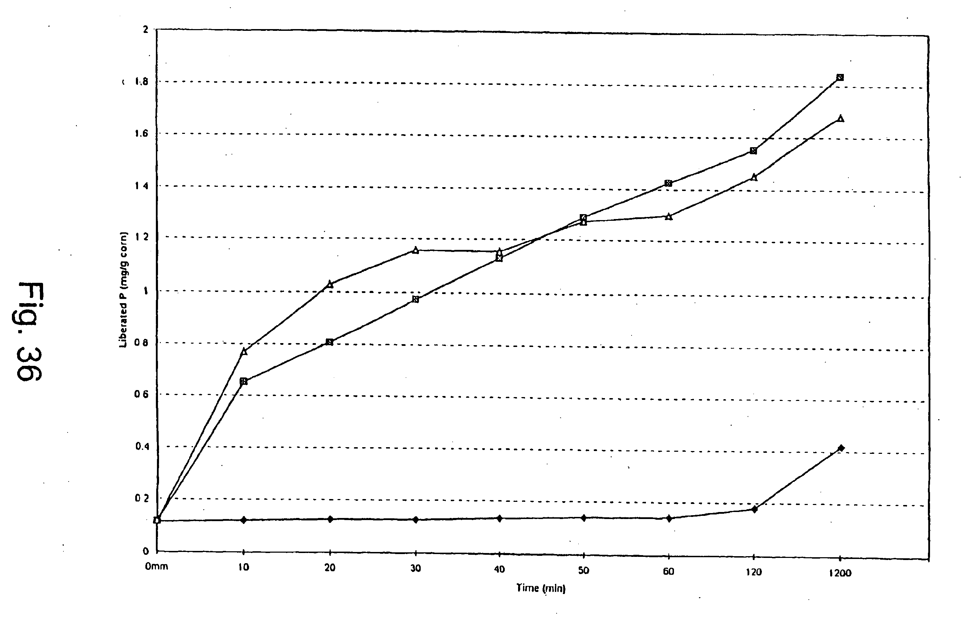

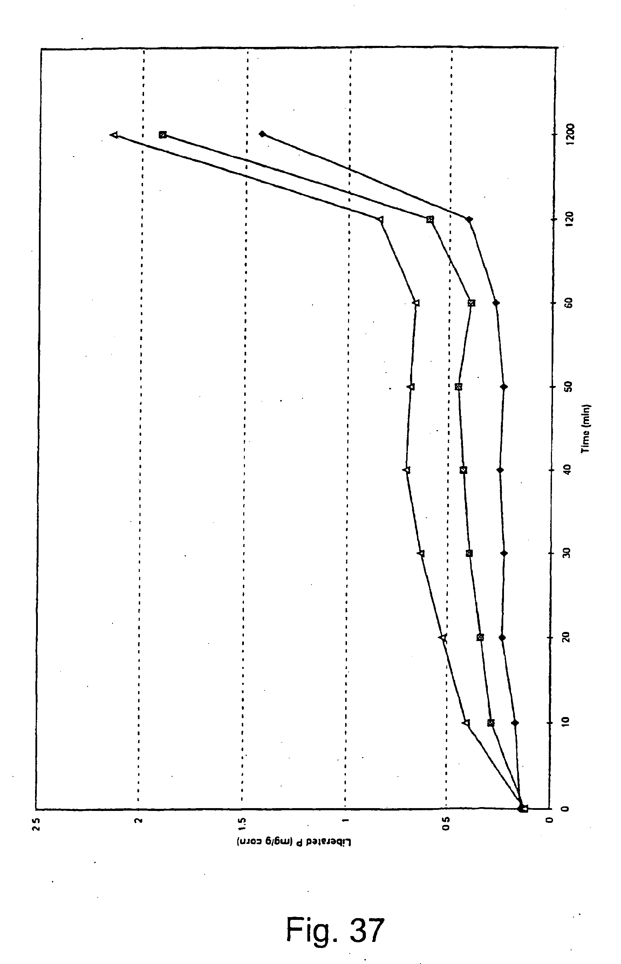

Two experiments were conducted: at pH 5.5 and at pH 3.5. The analysis results are shown at figs. 36 and 37 (pH 5.5 and 3.5, respectively). At these figures, symbol "♦" represents the control experiment, "♦" the Agrocybe phytase and "■" the Aspergillus phytase.

Results and discussion:

Figs. 36 and 37 clearly show that the Agrocybe phytase initially liberates phosphorous from ground corn faster than the Aspergillus phytase at pH 5.5 and at pH 3.5.

However, after 40 min. at pH 5.5 (fig. 36) and after 120 min. at pH 3.5 (fig. 37), the Aspergillus phytase is approximately at the same level of released phosphate as is the Agrocybe phytase.

But considering the passage time of the digestive system of for instance chickens / broilers, which normally is of the order of magnitude of 30 minutes to 2 hours, this difference is for sure important. Besides, it should be mentioned, that the pH value of 3.5 is more relevant in this respect than the pH 5.5 value.

This implies that the Agrocybe enzyme is surprisingly more efficient than the known Aspergillus phytase as a P-liberator in the digestive system of e.g. broilers.

**Example 18**

**Purification and characterization of the phytases from *Paxillus involtus* and *Trametes pubescens***

The PhyA1 phytase from *Paxillus involtus*

The *Paxillus involtus* PhyA1 phytase was expressed in and excreted from *Aspergillus oryzae* IFO 4177 as described in Examples 3 and 1.

Filter aid was added to the culture broth which was filtered through a filtration cloth. This solution was further filtered through a Seitz depth filter plate resulting in a clear solution. The filtrate was concentrated by ultrafiltration on 3kDa cut-off polyethersulphone membranes and the phytase was transferred to 10mM succinic acid/NaOH, pH 6.0 on a Sephadex G25 column. The pH of the G25 filtrate was adjusted to pH 7.0.

The phytase was applied to a Q-sepharose FF column equilibrated in 20mM HEPES/NaOH, pH 7.0. The enzyme turned out to be in the run-through from the column. $(NH_4)_2SO_4$ was added to the run-through to 2.0M final concentration. A Butyl Toyopearl 650S column was equilibrated in 2.0M $(NH_4)_2SO_4$, 10mM $CH_3COOH$/NaOH, pH 5.5 and the phytase was applied to this column and eluted with a decreasing linear $(NH_4)_2SO_4$ gradient $(2.0 \rightarrow 0M)$. Phytase containing fractions were pooled and the buffer was exchanged for 20mM HEPES/NaOH, pH 7.5 on a Sephadex G25 column. The G25 filtrate was applied to a Q-sepharose FF column equilibrated in 20mM HEPES/NaOH, pH 7.5. After washing the column extensively with the equilibration buffer, the phytase was eluted with an increasing linear NaCl gradient $(0 \rightarrow 0.5M)$. The phytase activity was pooled and the buffer was exchanged for 20mM Tris/$CH_3COOH$, pH 8.0 on a Sephadex G25 column. The G25 filtrate was applied to a SOURCE 30Q column equilibrated in 20mM Tris/$CH_3COOH$, pH 8.0. After washing the column thoroughly with the equilibration buffer en phytase was eluted with an increasing linear NaCl gradient $(0 \rightarrow 0.3M)$. Phytase containing fractions were pooled and $(NH_4)_2SO_4$ was added to 2.0M final concentration. A Phenyl Toyopearl 650S column was equilibrated in 2.0M $(NH_4)_2SO_4$, 10mM $CH_3COOH$/ NaOH, pH 5.5 and the phytase was applied to this column and eluted with a decreasing linear $(NH_4)_2SO_4$ gradient $(2.0 \rightarrow 0M)$. Phytase containing fractions were pooled and reapplied to the same Phenyl Toyopearl column after adding $(NH_4)_2SO_4$ to 2.0M final concentration. Fractions from the second Phenyl Toyopearl column were analyzed by SDS-PAGE and pure phytase fractions were pooled.

The *Paxillus involtus* PhyA1 phytase migrates on SDS-PAGE as a band with $M_r$ = 65 kDa. N-terminal amino acid sequencing of the 65 kDa component was carried out following SDS-PAGE and electroblotting onto a PVDF-membrane. The following N-terminal amino acid sequence could be deduced.

Ser-Val-Pro-Lys-Asn-Thr-Ala-Pro-Thr-Phe-Pro-Ile-Pro

The sequence corresponds to amino acid residues 21-33 in the cDNA derived amino acid sequence.

Accordingly a mature amino acid sequence of the phytase when expressed in Aspergillus is supposed to be no. 21-442 of SEQ ID no. 26. Accordingly, the predicted signal peptide at Fig. 3 does not correspond with the actual signalpeptide when this phytase is expressed in Aspergillus. This is also so for the indications regarding mature peptide of the sequence listing under the headings SEQ ID NOs: 25 and 26.

The PhyA2 phytase from *Paxillus involtus*

The *Paxillus involtus* PhyA2 phytase was expressed in and excreted from *Aspergillus oryzae* IFO 4177 as described in Examples 3 and 1.

Filter aid was added to the culture broth which was filtered through a filtration cloth. This solution was further filtered through a Seitz depth filter plate resulting in a clear solution. The filtrate was concentrated by ultrafiltration on 3kDa cut-off polyethersulphone membranes and $(NH_4)_2SO_4$ was added to 2.0M final concentration.

The phytase was applied to a Phenyl sepharose FF column equilibrated in 2.0M $(NH_4)_2SO_4$, 20mM $CH_3COOH/$NaOH, pH 5.5 and the enzyme was eluted with a decreasing linear $(NH_4)_2SO_4$ gradient ($2.0 \rightarrow 0M$). The phytase activity eluted as a single peak. This peak was pooled and $(NH_4)_2SO_4$ was added to 2.0M final concentration. A Butyl Toyopearl 650S column was equilibrated in 2.0M $(NH_4)_2SO_4$, 10mM $CH_3COOH/NaOH$, pH 5.5 and the phytase was applied to this column and eluted with a decreasing linear $(NH_4)_2SO_4$ gradient ($2.0 \rightarrow 0M$). Phytase containing fractions were pooled and the buffer was exchanged for 20mM HEPES/NaOH, pH 7.0 on a Sephadex G25 column. The G25 filtrate was applied to a Q-sepharose FF column equilibrated in 20mM HEPES/NaOH, pH 7.0. After washing the column extensively with the equilibration buffer, the phytase was eluted with an increasing linear NaCl gradient ($0 \rightarrow 0.5M$). The phytase activity was pooled and the buffer was exchanged for 20mM HEPES/NaOH, pH 7.0 by dialysis. The dialysed phytase was applied to a SOURCE 30Q column equilibrated in 20mM HEPES/NaOH, pH 7.0. After washing the column thoroughly with the equilibration buffer en phytase was eluted with an increasing linear NaCl gradient ($0 \rightarrow 0.3M$). Fractions from the SOURCE 30Q column were analyzed by SDS-PAGE and pure phytase fractions were pooled.

The *Paxillus involtus* PhyA2 phytase migrates on SDS-PAGE as a band with $M_r$ = 52 kDa. N-terminal amino acid sequencing of the 52 kDa component was carried out following SDS-PAGE and electroblotting onto a PVDF-membrane. The following N-terminal amino acid sequence could be deduced.

Asn-Ile-Ala-Pro-Lys-Phe-

The sequence corresponds to amino acid residues 25-30 in the cDNA derived amino acid sequence.

Accordingly a mature amino acid sequence of the phytase when expressed in Aspergillus is supposed to be no. 25-442 of SEQ ID no. 28. Accordingly, the predicted signal peptide at Fig. 4 does not correspond with the actual signalpeptide when this phytase is expressed in Aspergillus. This is also so for the indications regarding mature peptide of the sequence listing under the headings SEQ ID NOs: 27 and 28.

The phytase from *Trametes pubescens*

The *Trametes pubescens* phytase was expressed in and excreted from *Aspergillus oryzae* IFO 4177.

Filter aid was added to the culture broth which was filtered through a filtration cloth. This solution was further filtered through a Seitz depth filter plate resulting in a clear solution. The filtrate was concentrated by ultrafiltration on 10kDa cut-off polyethersulphone membranes followed by diafiltration with distilled water to reduce the conductivity. The pH of the concentrated enzyme was adjusted to pH 6.0 and the conductivity was adjusted to that of 10mM succinic acid/NaOH, pH 6.0 by dilution with deionised water.

The phytase was applied to a Q-sepharose FF column equilibrated in 10mM succinic acid/NaOH, pH 6.0 and the enzyme was eluted with an increasing linear NaCl gradient ($0 \rightarrow 0.5M$). The phytase activity eluted as a single peak. This peak was pooled and $(NH_4)_2SO_4$ was added to 2.0M final concentration. A Butyl Toyopearl 650S column was equilibrated in 2.0M $(NH_4)_2SO_4$, 10mM $CH_3COOH/NaOH$, pH 5.5 and the phytase was applied to this column and eluted with a decreasing linear $(NH_4)_2SO_4$ gradient ($2.0 \rightarrow 0M$). Phytase containing fractions were pooled and the buffer was exchanged for 10mM succinic acid/NaOH, pH 6.5 on a Sephadex G25 column. The G25 filtrate was applied to a Q-sepharose FF column equilibrated in 10mM succinic acid/NaOH, pH 6.5. After washing the column extensively with the equilibration buffer, the phytase was eluted with an increasing linear NaCl gradient ($0 \rightarrow 0.3M$). The phytase activity was pooled and the buffer was exchanged for 10mM succinic acid/NaOH, pH 7.0 on a Sephadex G25 column. The G25 filtrate was applied to a SOURCE 30Q column equilibrated in 10mM succinic acid/NaOH, pH 7.0. After washing the column thoroughly with the equilibration buffer en phytase was eluted with an increasing linear NaCl gradient ($0 \rightarrow 0.2M$). Fractions from the SOURCE 30Q column were analyzed by SDS-PAGE and pure phytase fractions were pooled.

The *Trametes pubescens* phytase migrates on SDS-PAGE as a band with $M_r$ = 57 kDa. N-terminal amino acid sequencing of the 57 kDa component was carried out following SDS-PAGE and electroblotting onto a PVDF-membrane. The following N-terminal amino acid sequence could be deduced.

Xxx-Ala-Cys-Leu-Asp-Val-Thr-Arg-Asp-(Ala/Val)-Gln-

The sequence corresponds to amino acid residues 31-41 in the cDNA derived amino acid sequence.

Accordingly a mature amino acid sequence of the phytase when expressed in Aspergillus is supposed to be no. 31-443 of SEQ ID no. 30. Accordingly, the predicted signal peptide at Fig. 5 does not correspond with the actual signalpeptide when this phytase is expressed in Aspergillus. This is also so for the indications regarding mature peptide of the sequence listing under the headings SEQ ID NOs: 29 and 30.

The molecular weights (kDa) of the three phytases of *Trametes pubescens* and PhyA2 and PhyA1 of *Paxillus involtus* are 65, 55 and 65 kDa, respectively.

The pH profiles of these three phytases are similar to Figs. 8 and 23 for the *Peniophora* and the *Agrocybe* phytases (optimum pH around 5-6; very little activity at pH above 7). As compared to the known phytase of *Aspergillus ficuum* (in this test having a temperature optimum of around 50˚C) PhyA1 of *Paxillus involtus* has a slightly higher temperature optimum around 60˚C, while PhyA2 has a temperature optimum of around 40˚C. The termostability of the PhyA1 phytase of *Paxillus involtus* is comparable to that of the *Aspergillus ficuum* phytase, and better than that of the PhyA2 phytase of *Paxillus involtus* and the phytase of *Trametes pubescens.* Following 60 minutes incubation at 70˚C and 80˚C, respectively, the residual activity of PhyA1 is around 60% and 40%, respectively.

In DSC, Td's of around 48˚C, 59˚C and 55˚C are found for the phytases PhyA2 and PhyA1 of *Paxillus involtus* and for the phytase of *Trametes pubescens,* respectively.

The specific activities are surprisingly high for all these phytases, viz. 1450, 1370 and 810 FYT/mg enzyme protein for the phytases of *Trametes pubescens, Paxillus involtus* PhyA2 and PhyA1, respectively ($A_{280}$ of 3.58; 5.72 and 3.08; FYT/ml of 5184, 7808 and 2497; assumed extinction coefficient of 1 l/(g x cm), respectively).

## Claims

1. An isolated polypeptide exhibiting phytase activity,

    i) the polypeptide comprising I-Q-R-H-G-A-R-[F/W]-P-T-S-G-A-X-X-R (SEQ ID NO: 3), N-W-T-[A/E]-G-F-X-X-A-S (SEQ ID NO: 5), and F-V-E-S-Q-X-[Y/F]-A-R-X-X-G-X-G-D-F-[E/A]-K-C (SEQ ID NO: 9), wherein "X" denotes any amino acid; or
    ii) the polypeptide having a degree of identity of at least 50% to the mature polypeptide part of either one of SEQ ID NO: 22, SEQ ID NO: 24, SEQ ID NO: 26, SEQ ID NO: 28, or SEQ ID NO: 30.

2. The polypeptide according to claim 1 wherein the degree of identity is at least 55%, 60%, 65%, 70%, 80%, 90%, 95%, or at least 97%.

3. The polypeptide according to any of claims 1-2 comprising at least one of the following amino acid sequences, reference being had to the alignment at Fig. 7 and using the numbering corresponding to phyA_P_lycii:

    (SEQ ID NO: 1) at position 46 to 57
    (SEQ ID NO: 2) at position 64 to 80
    (SEQ ID NO: 3) at position 68 to 83
    (SEQ ID NO: 4) at position 155 to 167
    (SEQ ID NO: 5) at position 162 to 171
    (SEQ ID NO: 6) at position 166 to 177
    (SEQ ID NO: 7) at position 358 to 373
    (SEQ ID NO: 8) at position 395 to 405
    (SEQ ID NO: 9) at position 415 to 433
    (SEQ ID NO: 10) at position 162 to 167
    (SEQ ID NO: 11) at position 273 to 278
    (SEQ ID NO: 12) at position 271 to 277
    (SEQ ID NO: 13) at position 428 to 432
    (SEQ ID NO: 14) at position 64 to 69.

4. The polypeptide according to claim 3 comprising at least one of the following sets of amino acid sequences:

    (SEQ ID NO: 10) and (SEQ ID NO: 12); or
    (SEQ ID NO: 10) and (SEQ ID NO: 13); or
    (SEQ ID NO: 14) and (SEQ ID NO: 10); or
    (SEQ ID NO: 14) and (SEQ ID NO: 12); or
    (SEQ ID NO: 14) and (SEQ ID NO: 13); or
    (SEQ ID NO: 11) and (SEQ ID NO: 13).

5. The polypeptide according to any of claims 1-4 comprising an amino acid sequence fulfilling one or more of the following conditions, reference being had to the alignment at Fig. 7 and using the numbering corresponding to phyA_ P_lycii:

(a) there are 10 amino acid residues between P46 and P57;
(b) there are 9 amino acid residues between F167 and P177;
(c) there are 10 amino acid residues between P177 and N188;
(d) there are 6 amino acid residues between C196 and D203;
(e) there are 17 amino acid residues between L353 and P371.

6. The polypeptide according to any of claims 1-5, which comprises an amino acid sequence selected from the group consisting of residues -26-427, -2-427, 1-427, 2-427 and 5-427 of SEQ ID NO 22; and/or which is encoded by

(i) nucleotides 17-1375, 89-1375, 95-13375, 98-1375 or 107-1375 of SEQ ID NO 21; or
(ii) the DNA sequence cloned into plasmid pYES 2.0 present in Escherichia coli DSM 11313.

7. The polypeptide according to any of claims 1-5, which comprises an amino acid sequence selected from the group consisting of residues -19-423, 1-423, and 2-423 of SEQ ID NO 26; and/or which is encoded by

(i) nucleotides 58-1383, 115-1383 and 118-1383 of SEQ ID NO 25; or
(ii) the DNA sequence cloned into plasmid pYES 2.0 present in Escherichia coli DSM 11842.

8. The polypeptide according to any of claims 1-5, which comprises an amino acid sequence selected from the group consisting of residues -19-423, 1-423, and 6-423 of SEQ ID NO 28; and/or which is encoded by

(i) nucleotides 48-1373, 105-1373 or 120-1373 of SEQ ID NO 27; or
(ii) the DNA sequence cloned into plasmid pYES 2.0 present in Escherichia coli DSM 11843.

9. The polypeptide according to any of claims 1-5 which comprises an amino acid sequence selected from the group consisting of residues -17-426, 1-426, and 14-426 of SEQ ID NO 30; and/or which is encoded by

(i) nucleotides 79-1407, 130-1407 or 169-1407 of SEQ ID NO 29; or
(ii) the DNA sequence cloned into plasmid pYES 2.0 present in Escherichia coli DSM 11844.

10. A DNA molecule encoding the polypeptide according to any of claims 1-9.

11. The DNA molecule according to claim 10 encoding a polypeptide exhibiting phytase activity and comprising at least one of the following DNA sequences

5' - AAY TGG ACN GMN GGN TT - 3'
(residues 10-26 of SEQ ID NO: 15)
5' - GAY AAR TWY GGN AC - 3'
(residues 10-23 of SEQ ID NO: 16)
5' - CAR GTN MAY MTN ATH CA - 3'
(residues 10-26 of SEQ ID NO: 19)

wherein N denotes any of A, C, G, T;

R denotes any of A and G;
Y denotes any of C and T;
M denotes any of A and C; and
W denotes any of A and T.

12. The DNA molecule according to any of claims 10-11, and comprising a DNA sequence selected from the following:

(i) nucleotides 17-1375; 89-1375; 95-1375; 98-1375; or 107-1375 of SEQ ID NO 21; or
(ii) nucleotides 58-1383; 115-1383; or 118-1383 of SEQ ID NO 25; or
(iii) nucleotides 48-1373; 105-1373 or 120-1373 of SEQ ID NO or
(iv) nucleotides 79-1407; 130-1407 or 169-1407 of SEQ ID NO 29; or

(v) the DNA sequences cloned into plasmid pYES 2.0 present in any of Escherichia coli DSM 11312, 11313, 11842, 11843, or 11844.

13. The DNA molecule according to any of claims 10-11, which encodes a polypeptide comprising

(i) residues -26-427; -2-427; 1-427; 2-427; or 5-427 of SEQ ID NO 22; or
(ii) residues -19-423; 1-423; or 2-423 of SEQ ID NO 26; or
(iii) residues -19-423; 1-423; or 6-423 of SEQ ID NO 28; or
(iv) residues -17-426, 1-426, or 14-426 of SEQ ID NO 30.

14. A vector comprising the DNA molecule according to any of claims 10-13.

15. A host cell comprising the DNA molecule according to any of claims 10-13 or the vector according to claim 14.

16. A process for preparing a polypeptide exhibiting phytase activity, the process comprising culturing the host cell according to claim 15 under conditions permitting the production of the polypeptide, and recovering the polypeptide from the culture broth.

17. A transgenic plant or plant part, which carries (a) a DNA molecule according to any of claims 10-13; or (b) a DNA molecule encoding a polypeptide exhibiting phytase activity, wherein the DNA molecule comprises

(i) a DNA sequence encoding residues -30-409; or 1-409 of SEQ ID NO:24; or
(ii) nucleotides 123-1439 or 213-1439 of SEQ ID NO:23; or
(iii) the DNA sequence cloned into plasmid pYES 2.0 present in Escherichia coli DSM 11312; so as to express or produce the encoded phytase.

18. A feed or food comprising at least one polypeptide of any of claims 1-9 or at least one transgenic plant or plant part according to claim 17.

19. A process for preparing a feed or food according to claim 18, wherein the at least one polypeptide or plant or plant part is added to the food or feed components.

20. A composition comprising at least one polypeptide of any of claims 1-9.

21. The composition of claim 20 which contains other feed or food enhancing enzymes.

22. The composition according to any of claims 20-21 which is an animal feed additive.

23. A process for reducing phytate levels in animal manure comprising feeding an animal with an effective amount of the feed according to claim 18.

24. Use of the polypeptide of any of claims 1-9 or the composition according to any of claims 20-22 for liberating phosphorous from a phytase substrate; or for improving the food or feed utilization.

25. Escherichia coli DSM No. 11312, Escherichia coli DSM No. 11313, Escherichia coli DSM No. 11842, Escherichia coli DSM No. 11843, or Escherichia coli DSM No. 11844.

**Patentansprüche**

1. Isoliertes Polypeptid, das Phytaseaktivität zeigt,

i) wobei das Polypeptid I-Q-R-H-G-A-R-[F/W]-P-T-S-G-A-X-X-R (SEQ ID NO: 3), N-W-T-[A/E]-G-F-X-X-A-S (SEQ ID NO: 5), und F-V-E-S-Q-X-[Y/F]-A-R-X-X-G-X-G-D-F-[E/A]-K-C (SEQ ID NO: 9) umfasst, wobei "X" eine beliebige Aminosäure bezeichnet; oder
ii) wobei das Polypeptid mit einem Grad der Identität von mindestens 50 % gegenüber dem maturierten Polypeptidbestandteil von entweder SEQ ID NO: 22, SEQ ID NO: 24, SEQ ID NO: 26, SEQ ID NO: 28, oder SEQ ID NO: 30 besitzt.

**2.** Polypeptid nach Anspruch 1, wobei der Grad der Identität bei mindestens 55 %, 60 %, 65 %, 70 %, 80 %, 90 %, 95 %, oder bei mindestens 97 % ist.

**3.** Polypeptid nach einem der Ansprüche 1-2, das zumindest eine der folgenden Aminosäuresequenzen umfasst, Bezug nehmend auf den Sequenzabgleich in Abbildung 7 unter der Verwendung der Nummerierung korrespondierend zu phyA_P_lycii:

(SEQ ID NO: 1) von Position 46 bis 57
(SEQ ID NO: 2) von Position 64 bis 80
(SEQ ID NO: 3) von Position 68 bis 83
(SEQ ID NO: 4) von Position 155 bis 167
(SEQ ID NO: 5) von Position 162 bis 171
(SEQ ID NO: 6) von Position 166 bis 177
(SEQ ID NO: 7) von Position 358 bis 373
(SEQ ID NO: 8) von Position 395 bis 405
(SEQ ID NO: 9) von Position 415 bis 433
(SEQ ID NO: 10) von Position 162 bis 167
(SEQ ID NO: 11) von Position 273 bis 278
(SEQ ID NO: 12) von Position 271 bis 277
(SEQ ID NO: 13) von Position 428 bis 432
(SEQ ID NO: 14) von Position 64 bis 69.

**4.** Polypeptid nach Anspruch 3, das mindestens einen der folgenden Sätze von Aminosäuresequenzen umfasst:

(SEQ ID NO: 10) und (SEQ ID NO: 12); oder
(SEQ ID NO: 10) und (SEQ ID NO: 13); oder
(SEQ ID NO: 14) und (SEQ ID NO: 10); oder
(SEQ ID NO: 14) und (SEQ ID NO: 12); oder
(SEQ ID NO: 14) und (SEQ ID NO: 13); oder
(SEQ ID NO: 11) und (SEQ ID NO: 13).

**5.** Polypeptid nach einem der Ansprüche 1-4, das eine Aminosäuresequenz umfasst, die eine oder mehrere der folgenden Bedingungen erfüllt, Bezug nehmend auf den Sequenzabgleich in Abbildung 7 und unter Verwendung der Nummerierung korrespondierend zu phyA_P_lycii:

(a) es gibt 10 Aminosäurereste zwischen P46 und P57;
(b) es gibt 9 Aminosäurereste zwischen F 167 und P 177;
(c) es gibt 10 Aminosäurereste zwischen P177 und N188;
(d) es gibt 6 Aminosäurereste zwischen C196 und D203;
(e) es gibt 17 Aminosäurereste zwischen L353 und P371.

**6.** Polypeptid nach einem der Ansprüche 1-5, das eine Aminosäuresequenz umfasst, die ausgewählt ist aus der Gruppe, bestehend aus den Resten -26-427, -2-427, 1-427, 2-427 und 5-427 der SEQ ID NO: 22; und/oder kodiert ist durch

(i) Nukleotide 17-1375, 89-1375, 95-1375, 98-1375 oder 107-1375 der SEQ ID NO: 21; oder
(ii) die DNA-Sequenz, die in das Plasmid pYES 2.0 kloniert ist, das in Escherichia coli DSM 11313 vorliegt.

**7.** Polypeptid nach einem der Ansprüche 1-5, das eine Aminosäuresequenz umfasst, die ausgewählt ist aus der Gruppe, bestehend aus den Resten -19-423, 1-423, und 2-423 der SEQ ID NO: 26; und/oder kodiert ist durch

(i) Nukleotide 58-1383, 115-1383 und 118-1383 der SEQ ID NO: 25; oder
(ii) die DNA-Sequenz, die in das Plasmid pYES 2.0 kloniert ist, das in Escherichia coli DSM 11842 vorliegt.

**8.** Polypeptid nach einem der Ansprüche 1-5, das eine Aminosäuresequenz umfasst, die ausgewählt ist aus der Gruppe, bestehend aus den Resten -19-423, 1-423, und 6-423 der SEQ ID NO: 28; und/oder kodiert ist durch

(i) Nukleotide 48-1373, 105-1373 oder 120-1373 der SEQ ID NO: 27; oder

(ii) die DNA-Sequenz, die in das Plasmid pYES 2.0 kloniert ist, das in Escherichia coli DSM 11843 vorliegt.

9. Polypeptid nach einem der Ansprüche 1-5, das eine Aminosäuresequenz umfasst, die ausgewählt ist aus der Gruppe, bestehend aus den Resten -17-426, 1-426, und 14-426 der SEQ ID NO: 30; und/oder kodiert ist durch

    (i) Nukleotide 79-1407, 130-1407 oder 169-1407 der SEQ ID NO: 29; oder
    (ii) die DNA-Sequenz, die in das Plasmid pYES 2.0 kloniert ist, das in Escherichia coli DSM 11844 vorliegt.

10. DNA-Molekül, das das Polypeptid nach einem der Ansprüche 1-9 kodiert.

11. DNA-Molekül nach Anspruch 10, das ein Polypeptid kodiert, das eine Phytaseaktivität aufweist und zumindest eine der folgenden DNA-Sequenzen umfasst
5' - AAY TGG ACN GMN GGN TT - 3'
(Reste 10-26 der SEQ ID NO: 15)
5' - GAY AAR TWY GGN AC - 3'
(Reste 10-23 der SEQ ID NO: 16)
5' - CAR GTN MAY MTN ATH CA - 3'
(Reste 10-26 der SEQ ID NO: 19)
wobei N entweder A, C, G, oder T bezeichnet;

    R bezeichnet entweder A oder G;
    Y bezeichnet entweder C oder T;
    M bezeichnet entweder A oder C; und
    W bezeichnet entweder A oder T.

12. DNA-Molekül nach einem der Ansprüche 10-11, das eine der folgenden DNA-Sequenzen umfasst:

    (i) Nukleotide 17-1375; 89-1375; 95-1375; 98-1375; oder 107-1375 der SEQ ID NO: 21; oder
    (ii) Nukleotide 58-1383; 115-1383; oder 118-1383 der SEQ ID NO: 25; oder
    (iii) Nukleotide 48-1373; 105-1373 oder 120-1373 der SEQ ID NO: 27; oder
    (iv) Nukleotide 79-1407; 130-1407 oder 169-1407 der SEQ ID NO: 29; oder
    (v) die DNA-Sequenz, die in das Plasmid pYES 2.0 kloniert ist und in einem beliebigen von Escherichia coli DSM 11312, 11313, 11842, 11843, oder 11844 vorliegt.

13. DNA-Molekül nach einem der Ansprüche 10-11, das ein Polypeptid kodiert, das die

    (i) Reste -26-427; -2-427; 1-427; 2-427; oder 5-427 der SEQ ID NO: 22; oder
    (ii) Reste -19-423; 1-423; oder 2-423 der SEQ ID NO: 26; oder
    (iii) Reste -19-423; 1-423; oder 6-423 der SEQ ID NO: 28; oder
    (iv) Reste -17-426, 1-426, oder 14-426 der SEQ ID NO: 30 umfasst.

14. Vektor, der das DNA-Molekül nach einem der Ansprüche 10-13 umfasst.

15. Wirtszelle, die das DNA-Molekül nach einem der Ansprüche 10-13 umfasst, oder den Vektor nach Anspruch 14.

16. Verfahren zur Herstellung eines Polypeptids, das Phytaseaktivität aufweist, wobei das Verfahren das Kultivieren der Wirtszelle nach Anspruch 15 unter Bedingungen, die das Herstellen des Polypeptids erlaubt, und das Rückgewinnen des Polypeptids aus der Zellkulturbrühe umfasst.

17. Transgene Pflanze oder Pflanzenteil, das

    (a) ein DNA-Molekül nach einem der Ansprüche 10-13, oder
    (b) ein DNA-Molekül, das ein Polypeptid, das Phytaseaktivität aufweist, kodiert, wobei das DNA-Molekül

        (i) eine DNA-Sequenz, die die Reste -30-409; oder 1-409 der SEQ ID NO: 24 kodiert; oder
        (ii) Nukleotide 123-1439; oder 213-1439 der SEQ ID NO: 23; oder
        (iii) die DNA-Sequenz, die in das Plasmid pYES 2.0 kloniert ist, das in Escherichia coli DSM 11312 vorliegt,

umfasst um somit die kodierte Phytase zu exprimieren oder zu produzieren.

**18.** Futter- oder Nahrungsmittel, das mindestens ein Polypeptid nach einem der Ansprüche 1-9, oder mindestens eine transgene Pflanze, oder einen Pflanzenteil nach Anspruch 17 umfasst.

**19.** Verfahren zur Herstellung eines Futter- oder Nahrungsmittels nach Anspruch 18, wobei mindestens das/die eine/ein Polypeptid, oder Pflanze, oder Pflanzenteil zu den Futter- oder Nahrungsmittelbestandteilen zugesetzt wird.

**20.** Zusammensetzung, die mindestens ein Polypeptid nach einem der Ansprüche 1-9 umfasst.

**21.** Zusammensetzung nach Anspruch 20, das weitere futter- oder nahrungsmittelanreichemde Enzyme enthält.

**22.** Zusammensetzung nach einem der Ansprüche 20-21, das ein Tierfutteradditiv ist.

**23.** Verfahren zur Reduktion des Phytatspiegels in Tierdung, was die Fütterung eines Tieres mit einer effektiven Menge des Futtermittels nach Anspruch 18 umfasst.

**24.** Verwendung des Polypeptids nach einem der Ansprüche 1-9, oder der Zusammensetzung nach einem der Ansprüche 20-22 zur Freisetzung von Phosphor von einem Phytasesubstrat; oder zur Steigerung der Nahrungsmittel- oder Futterverwertung.

**25.** Escherichia coli DSM No. 11312, Escherichia coli DSM No. 11313, Escherichia coli DSM No. 11842, Escherichia coli DSM No. 11843, oder Escherichia coli DSM No. 11844.

**Revendications**

**1.** Polypeptide isolé présentant une activité phytase,

i) le polypeptide comprenant I-Q-R-H-G-A-R-[F/W]-P-T-S-G-A-X-X-R (SEQ ID n° 3), N-W-T-[A/E]-G-F-X-X-A-S (SEQ ID n° 5), et F-V-E-S-Q-X-[Y/F]-A-R-X-X-G-X-G-D-F-[E/A]-K-C (SEQ ID n° 9), dans lesquelles « X » signifie n'importe quel acide aminé ; ou
ii) le polypeptide ayant un degré d'identité d'au moins 50 % avec la partie de polypeptide mature de l'une quelconque des séquences SEQ ID n° 22, SEQ ID n° 24, SEQ ID n° 26, SEQ ID n°28, ou SEQ ID n° 30.

**2.** Polypeptide selon la revendication 1, dans lequel le degré d'identité est d'au moins 55%, 60%, 65%, 70%, 80%, 90%, 95% ou d'au moins 97%.

**3.** Polypeptide selon l'une des revendications 1 à 2 comprenant au moins l'une des séquences d'acides aminés suivantes, référence étant faite à l'alignement sur la figure 7 et en utilisant la numérotation correspondant à phyA_P_lycii :

(SEQ ID n° 1) en position 46 à 57
(SEQ ID n° 2) en position 64 à 80
(SEQ ID n° 3) en position 68 à 83
(SEQ ID n° 4) en position 155 à 167
(SEQ ID n° 5) en position 162 à 171
(SEQ ID n° 6) en position 166 à 177
(SEQ ID n° 7) en position 358 à 373
(SEQ ID n° 8) en position 395 à 405
(SEQ ID n° 9) en position 415 à 433
(SEQ ID n° 10) en position 162 à 167
(SEQ ID n° 11) en position 273 à 278
(SEQ ID n° 12) en position 271 à 277
(SEQ ID n° 13) en position 428 à 432
(SEQ ID n° 14) en position 64 à 69.

**4.** Polypeptide selon la revendication 3 comprenant au moins l'un des ensembles de séquences d'acides aminés suivants :

(SEQ ID n° 10) et (SEQ ID n° 12) ; ou
(SEQ ID n° 10) et (SEQ ID n° 13) ; ou
(SEQ ID n° 14) et (SEQ ID n° 10) ; ou
(SEQ ID n° 14) et (SEQ ID n° 12) ; ou
(SEQ ID n° 14) et (SEQ ID n° 13) ; ou
(SEQ ID n° 11) et (SEQ ID n° 13).

5. Polypeptide selon l'une quelconque des revendications 1 à 4 comprenant une séquence d'acides aminés remplissant une ou plusieurs des conditions suivantes, référence étant faite à l'alignement sur la figure 7 et en utilisant la numérotation correspondant à phyA_P_lycii :

(a) 10 résidus d'acides aminés se trouvent entre P46 et P57 ;
(b) 9 résidus d'acides aminés se trouvent entre F 167 et P177 ;
(c) 10 résidus d'acides aminés se trouvent entre P177 et N188 ;
(d) 6 résidus d'acides aminés se trouvent entre C 196 et D203 ;
(e) 17 résidus d'acides aminés se trouvent entre L353 et P371.

6. Polypeptide selon l'une quelconque des revendications 1 à 5, qui comprend une séquence d'acides aminés choisie dans le groupe constitué par les résidus -26-427, -2-427, 1-427, 2-427 et 5-427 de la SEQ ID n° 22 ; et/ou qui est codé par

(i) les nucléotides 17-1375, 89-1375, 95-1375, 98-1375 ou 107-1375 de la SEQ ID n° 21 ; ou
(ii) la séquence d'ADN clonée dans le plasmide pYES 2.0 présent dans Escherichia coli DSM 11313.

7. Polypeptide selon l'une quelconque des revendications 1 à 5, qui comprend une séquence d'acides aminés choisie dans le groupe constitué par les résidus -19-423, 1-423, et 2-423 de la SEQ ID n° 26 ; et/ou qui est codé par

(i) les nucléotides 58-1383, 115-1383 et 118-1383 de la SEQ ID n° 25 ; ou
(ii) la séquence d'ADN clonée dans le plasmide pYES 2.0 présent dans Escherichia coli DSM 11842.

8. Polypeptide selon l'une quelconque des revendications 1 à 5, qui comprend une séquence d'acides aminés choisie dans le groupe constitué par les résidus -19-423, 1-423, et 6-423 de la SEQ ID n° 28. ; et/ou qui est codé par

(i) les nucléotides 48-1373, 105-1373 ou 120-1373 de la SEQ ID n° 27 ; ou
(ii) la séquence d'ADN clonée dans le plasmide pYES 2.0 présent dans Escherichia coli DSM 11843.

9. Polypeptide selon l'une quelconque des revendications 1 à 5, qui comprend une séquence d'acides aminés choisie dans le groupe constitué par les résidus -17-426, 1-426, et 14-426 de la SEQ ID n° 30 ; et/ou qui est codé par

(i) les nucléotides 79-1407, 130-1407 ou 169-1407 de la SEQ ID n° 29 ; ou
(ii) la séquence d'ADN clonée dans le plasmide pYES 2.0 présent dans Escherichia coli DSM 11844.

10. Molécule d'ADN codant le polypeptide selon l'une quelconque des revendications 1 à 9.

11. Molécule d'ADN selon la revendication 10 codant pour un polypeptide présentant une activité phytase et comprenant au moins l'une des séquences d'ADN suivantes :

5' - AAY TGG ACN GMN GGN TT - 3'
(residues 10-26 of SEQ ID NO: 15)
5' - GAY AAR TWY GGN AC - 3'
(residues 10-23 of SEQ ID NO: 16)
5' - CAR GTN MAY MTN ATH CA - 3'
(residues 10-26 of SEQ ID NO: 19)

dans lesquelles N signifie l'un quelconque parmi A, C, G, T ;

R signifie l'un quelconque parmi A et G ;
Y signifie l'un quelconque parmi C et T ;
M signifie l'un quelconque parmi A et C ; et
W signifie l'un quelconque parmi A et T.

**12.** Molécule d'ADN selon l'une quelconque des revendications 10-11, et comprenant une séquence d'ADN choisie à partir de ce qui suit :

(i) nucléotides 17-1375 ; 89-1375 ; 95-1375 ; 98-1375 ; ou 107-1375 de la SEQ ID n° 21 ; ou
(ii) nucléotides 58-1383 ; 115-1383 ; ou 118-1383 de la SEQ ID n° 25 ; ou
(iii) nucléotides 48-1373 ; 105-1373 ou 120-1373 de la SEQ ID n° 27 ; ou
(iv) nucléotides 79-1407 ; 130-1407 ou 169-1407 de la SEQ ID n° 29 ; ou
(v) séquences d'ADN clonées dans le plasmide pYES 2.0 présent dans l'un quelconque parmi Escherichia coli DSM 11312, 11313, 11842, 11843, ou 11844.

**13.** Molécule d'ADN selon l'une quelconque des revendications 10-11, laquelle code pour un polypeptide comprenant :

(i) les résidus -26-427 ; -2-427 ; 1-427 ; 2-427 ; ou 5-427 de la SEQ ID n° 22 ; ou
(ii) les résidus -19-423 ; 1-423 ; ou 2-423 de la SEQ ID n° 26 ; ou
(iii) les résidus -19-423 ; 1-423 ; ou 6-423 de la SEQ ID n° 28 ; ou
(iv) les résidus -17-426, 1-426, ou 14-426 de la SEQ ID n° 30.

**14.** Vecteur comprenant la molécule d'ADN selon l'une quelconque des revendications 10-13.

**15.** Cellule hôte comprenant la molécule d'ADN selon l'une quelconque des revendications 10-13 ou le vecteur selon la revendication 14.

**16.** Procédé pour préparer un polypeptide ayant une activité phytase, le procédé comprenant la culture de la cellule hôte selon la revendication 15 dans des conditions permettant la production du polypeptide, et la récupération du polypeptide à partir du mout de culture.

**17.** Plante ou partie de plante transgénique, laquelle porte

a. une molécule d'ADN selon l'une quelconque des revendications 10-13 ou
b. une molécule d'ADN codant un polypeptide ayant une activité phytase ; la molécule d'ADN comprenant

i. une séquence d'ADN codant les résidus -30-409 ou 1-409 de la SEQ ID n° 24 ; ou
ii. les nucléotides 123-1439 ou 213-1439 de la SEQ ID n° 23 ; ou
iii. la séquence d'ADN clonée dans le plasmide pYES 2.0 présent dans Escherichia coli DSM 11312 ; de manière à exprimer ou produire la phytase codée.

**18.** Aliments pour animaux ou aliment comprenant au moins un polypeptide selon l'une quelconque des revendications 1 à 9 ou au moins une plante ou une partie de plante transgénique selon la revendication 17.

**19.** Procédé de préparation d'aliments pour animaux ou d'un aliment selon la revendication 18, dans lequel ledit au moins un polypeptide ou ladite au moins une plante ou partie de plante est ajouté(e) aux composants de l'aliment ou des aliments pour animaux.

**20.** Composition comprenant au moins un polypeptide selon l'une quelconque des revendications 1 à 9.

**21.** Composition selon la revendication 20, qui contient d'autres enzymes améliorant les aliments pour animaux ou les aliments.

**22.** Composition selon l'une des revendications 20-21 qui est un additif pour aliments pour animaux.

**23.** Procédé pour réduire les niveaux de phytate dans le fumier animal comprenant le fait de nourrir un animal avec une quantité efficace des aliments pour animaux selon la revendication 18.

**24.** Utilisation du polypeptide selon l'une quelconque des revendications 1 à 9 ou de la composition selon l'une quelconque des revendications 20-22 pour libérer le phosphore à partir d'un substrat phytase ; ou pour améliorer l'utilisation de l'aliment ou des aliments pour animaux.

**25.** Escherichia coli DSM n° 11312, Escherichia coli DSM n° 11313, Escherichia coli DSM n° 11842, Escherichia coli DSM n° 11843, ou Escherichia coli DSM n° 11844.

Fig. 1

EP 0 958 353 B1

Fig. 2

93

EP 0 958 353 B1

GGATCCGAATTCGGCACTCGTACGGTCCCCCGGTCTACCCTCTGCTCGCCTTGGAAGATGCTCTTCGGTTTCGTCGCCCTCGCCTGTCTCTTGTCCCTCTCCGAGGTCCTTGCGACCTCCGTGCCCAAGAACACAGCGCCGACCTTCCCCATTCCGGAGA
BamHI                                                    M L F G F V A L A C L L S L S E V L A | T S V P K N T A P T F P I P E>

GTGAGCAGCGGAACTGGTCCCCGTACTCGCCCTACTTCCCTCTTGCCGAGTACAAGGCTCCTCCGGCGGGCTGCCAGATCAACCAGGTCAACATCATCCAAAGACATGGTGCCCGGTTCCCGACCTCTGGCGCGACCACCCGTATCAAGGCGGGTTTGAC
S E Q R N W S P Y S P Y F P L A E Y K A P P A G C Q I N Q V N I I Q R H G A R F P T S G A T T R I K A G L T

CAAGTTGCAAGGCGTCCAGAACTTTACCGACGCCAAATTCAACTTCATCAAGTCGTTCAAGTACCATCTCGGTAACTCGGACCTCGTTCCGTTCGGTGCAGCACAGTCCTTCGACGCTGGTCAGGAGGCCTTCGCCCGCTACTCGAAGCTTGTCAGCAAG
K L Q G V Q N F T D A K F N F I K S F K Y D L G N S D L V P F G A A Q S F D A G Q E A F A R Y S K L V S K>

AACAACCTGCCGTTCATTCGTGCCGATGGAAGTGATCGTGTTGTGGATTCTGCTACAAACTGGACTGCGGGTTTCGCTTCGGCAAGTCACAACACGGTCCAGCCCAAGCTGAACCTGATTCTCCCGCAAACTGGCAATGATACCCTGGAAGATAATATGT
N N L P F I R A D G S D R V V D S A T N W T A G F A S A S H N T V Q P K L N L I L P Q T G N D T L E D N M

GCCCTGCTGCTGGCGATTCTGACCCCCAGGTCAACGCGTGGTTGGCTGTTGCTTTCCCTTCCATCACTGCACGGCTCAACGCCGCCGCGCCCTCTGTCAACCTCACCGACACGGACGCGGTTCAACCTCGTCAGTCTCTGCGCTTTCTTGACAGTCTCGAA
C P A A G D S D P Q V N A W L A V A F P S I T A R L N A A A P S V N L T D T D A F N L V S L C A F L T V S K

GGAGAAGAAGAGTGACTTCTGCACCCTGTTCGAGGGCATCCCTGGCTCTTTCGAGGCCGTTCGCCTATGGTGGCGACCTTGACAAGTTCTACGGTACCGGTTACGGTCAGGAACTCGGACCCGTTCAAGGCGTCGGCTACGTCAACGAGCTCATCGCCCGC
E K K S D F C T L F E G I P G S F E A F A Y G G D L D K F Y G T G Y G Q E L G P V Q G V G Y V N E L I A R

CTCACCAACTCCGCCGTCCGCGACAACACCCAGACGAACCGCACACTCGACGCCTCGCCCGTAACCTTCCCGTTGAACAAGACGTTCTACGCCGATTTCTCCCACGACAACCTCATGGTCGCCGTCTTCTCCGCCATGGGCCTCTTCCGCCAGCCCGCGC
L T N S A V R D N T Q T N R T L D A S P V T F P L N K T F Y A D F S H D N L M V A V F S A M G L F R Q P A

CGCTCAGCACGTCCGTGCCGAACCCATGGCGCACGTGGCGCACGAGCTCCCTCGTCCCCTTCTCCGGACGCATGGTCGTGGAACGCCTCAGCTGTTTCGGCACGACCAAGGTTCGCGTCCTCGTGCAGGACCAGGTGCAGCCGCTCGAGTTCTGCGGGGGG
P L S T S V P N P W R T W R T S S L V P F S G R M V V E R L S C F G T T K V R V L V Q D Q V Q P L E F C G G

TGATAGGAACGGGCTGTGCACGCTTGCTAAGTTTGTGGAGAGCCAGACGTTTGCGAGGAGTGATGGTGCCGGGGGACTTTGAGAAGTGCTTCGCGACCTCGGCGTGAGGATGGACGAACAAAATTAAATTGGGGTATTTTATCGTATAATTATGGTGTGTG
D R N G L C T L A K F V E S Q T F A R S D G A G D F E K C F A T S A *

TAGAACATGGGCTCGGGGTCGATGGTGAAAAGCAAAGGTTTATCGTCTAAAAAAAAAAAAAAAAAAAAAAAAAAAATTCCTGCGGCCGC
NotI

## Fig. 3

94

EP 0 958 353 B1

```
                    40                          80                         120                        160
          .    .    .    .         .    .    .    .         .    .    .    .         .    .    .    .
GGATCCGAATTCCAGTCCCCAAGCTAATCCTCTGCTCGCCTTGGAAGATGCACCTCGGCTTCGTCACCCTCGCTTGTCTCATACACCTCTCCGAGGTCTTCGCGGCCATCCGTGCCCCGGAATATTGCTCCGAAGTTCTCAATTCCGGAAAGCGAGCAGCG
BamH1                                     [M H L G F V T L A C L I H L S E V F A] A S V P R N I A P K F S I P E S E Q R

                    200                         240                        280                        320
          .    .    .    .         .    .    .    .         .    .    .    .         .    .    .    .
AAACTGGTCGCCTTACTCTCCTTACTTTCCCCTAGCCGAATACAAGGCTCCTCCAGCAGGCTGCGAGATTAACCAAGTCAATATTATCCAACGGCATGGCGCACGGTTCCCAACCTCGGGTGCGGCCACTCGCATCGGCTGGTTTAAGCAAGCTGCAA
    N  W  S  P  Y  S  P  Y  F  P  L  A  E  Y  K  A  P  P  A  G  C  E  I  N  Q  V  H  I  I  Q  R  H  G  A  R  F  P  T  S  G  A  A  T  R  I  K  A  G  L  S  K  L  Q

                    360                         400                        440                        480
          .    .    .    .         .    .    .    .         .    .    .    .         .    .    .    .
TCCGTCCAGAATTTCACCGACCCCAAATTCGACTTCATCAAGTCGTTCACATACGATCTTGGTACTTCCGACCTCGTGCCCATTCGGCGCAGCACAATCATTCGATCCCGGCCTGGAGGTCTTCGCTCGCTATTCGAAGCTCGTCAGCTCGGACAACCTGC
    S  V  Q  N  F  T  D  P  K  F  D  F  I  K  S  F  T  Y  D  L  G  T  S  D  L  V  P  F  G  A  A  Q  S  F  D  A  G  L  E  V  F  A  R  Y  S  K  L  V  S  S  D  N  L

                    520                         560                        600                        640
          .    .    .    .         .    .    .    .         .    .    .    .         .    .    .    .
CTTTCATTCGCTCAGATGGTAGCGATCGTGTAGTCGACACTGCTACGAACTGGACTGCAGGTTTTGCTTCCGCGAGCCGCAACGCGATCCAACCCAAGCTCGACTTGATACTTCCACAAACTGGCAATGACACCCTCGAGGACAACATGTGTCCAGCTGC
    P  F  I  R  S  D  G  S  D  R  V  V  D  T  A  T  N  W  T  A  G  F  A  S  A  S  R  N  A  I  Q  P  K  L  D  L  I  L  P  Q  T  G  N  D  T  L  E  D  N  M  C  P  A  A

                    680                         720                        760                        800
          .    .    .    .         .    .    .    .         .    .    .    .         .    .    .    .
TGGCGAATCCGACCCTCAGGTCGATGCGTGGTTGGCGTCCGCCTTCCCATCTGTCACCGCGCAGCTCAACGCTGCAGCGCGCCTGGTGCCAATCTCACAGACGCCGACGCCTTCAACCTCGTCAGCCTGTGTCCCTTCATGACAGTTTCGAAGGAGCAGAAG
    G  E  S  D  P  Q  V  D  A  W  L  A  S  A  F  P  S  V  T  A  Q  L  N  A  A  A  P  G  A  N  L  T  D  A  D  A  P  N  L  V  S  L  C  P  P  M  T  V  S  K  E  Q  K

                    840                         880                        920                        960
          .    .    .    .         .    .    .    .         .    .    .    .         .    .    .    .
AGCGACTTCTGCACGTTGTTCGAGGGAATCCCTGGATCGTTCGAGGCGTTTGCCTATGCCGGCGACCTTGACAAGTTCTATGGGACCGGCTATGGCCAAGCCCTCGGACCGGTCCAAGGCGTCGGCTACATCAACGAGCTCCTTGCACGCCTGACCAACT
    S  D  F  C  T  L  F  E  G  I  P  G  S  F  E  A  F  A  Y  A  G  D  L  D  K  P  Y  G  T  G  Y  G  Q  A  L  G  P  V  Q  G  V  G  Y  I  N  E  L  L  A  R  L  T  N

                    1000                        1040                       1080                       1120
          .    .    .    .         .    .    .    .         .    .    .    .         .    .    .    .
CCGCAGTGAACGACAACACACAGACGAACCGCACACTCGACGCCGCACCAGACACGTTCCCGCTCAACAAGACCATGTACGCCGATTTCTCACACGACAACCTCATGGTCGCCGTGTTCTCCGCCATGGGCCTCTTCCGCCAATCCGCACCGCTCAGCAC
    S  A  V  N  D  N  T  Q  T  N  R  T  L  D  A  A  P  D  T  F  P  L  N  K  T  M  Y  A  D  F  S  H  D  N  L  M  V  A  V  F  S  A  M  G  L  P  R  Q  S  A  P  L  S  T

                    1160                        1200                       1240                       1280
          .    .    .    .         .    .    .    .         .    .    .    .         .    .    .    .
GTCCACACCGGATCCGAACCGCACGTGGCTCACGAGCTCTGTCGTTCCGTTCTCCGCGCGCATGGCCGTCGAACGCCTCAGCTGTGCTGGTACCACGAAGGTGCGCGTCCTGGTGCAGGACCAGGTCCAGCCACTCGAGTTCTGCGGCGGCGACCAGGAT
    S  T  P  D  P  N  R  T  N  L  T  S  S  V  V  P  F  S  A  R  M  A  V  E  R  L  S  C  A  G  T  T  K  V  R  V  L  V  Q  D  Q  V  Q  P  L  E  F  C  G  G  D  Q  D

                    1320                        1360                       1400                       1440
          .    .    .    .         .    .    .    .         .    .    .    .         .    .    .    .
GGGTTGTGCGCGCTAGACAAGTTCGTCGAGAGCCAGGCCGTATGCACGGAGTGGTGGCGCAGGTGACTTTGAGAAGTGTCTTGCGACGACGTGTGAGATGGGGTAATCTACGGTGAAGCAGCGGAGAGCCTCTCAACGAATGCAAAGGATAGGTTCGAGG
    G  L  C  A  L  D  K  F  V  E  S  Q  A  Y  A  R  S  G  G  A  G  D  F  E  K  C  L  A  T  T  V  *>
CTTACTTCATCAACCTATATCATCATAGGACAAGCCCCCCAATAGCCAGACTCGTCGTTTGACATCGTGTATGAAAATAACCCACCCACGCACTCCGCTGCCACTATTCGCGTGTATCGCATACTAGGCGTTTTCGCCCAGTTGAACATGAGCCCATTCT

                    1640
          .    .    .    .
GTCCCCAGTGAAAAAAAAAAAAAAAAAAAAAAAATTCCTGCGGCCGC
                            NotI
```

Fig. 4

Fig. 5

Fig. 6

Fig. 7

**Peniophora phytase pH-profile, 5mM phytate, 30 min at 37°C**

# Fig. 8

pH-stability of Peniophora phytase
- preincubation 1 h 40 °C

Fig. 9

Temperature profile of Peniophora phytase
0.1 M Na-Acetate, 5mM phytate, pH 5.5, 30 min

Fig. 10

**Temperature stability of Peniohora phytase - preincubation 60 min in 0.1 M Na-acetate pH 5.5**

# Fig. 11

Fig. 12

Fig. 13

Fig. 14

Fig. 15

Peniophora

(pH 5.5, 0 - 4.5 h)

4.5 h

t=3 h

t=2 h

t=1 h

t=30 min

t=15 min

t=0

5.0  4.8  4.6  4.4  4.2  4.0  3.8  3.6  3.4  3.2  3.0  ppm

Fig. 16

EP 0 958 353 B1

Fig. 17

pH 5.5

A  Ins(1,2,4,5,6)P5  (H-3)

B  Ins(1,2,3,4,5)P5  (H-6)

C  Ins(1,2,5,6)P4  (H-3)

Peniophora
20 min

B

5.0  4.9  4.8  4.7  4.6  4.5  4.4  4.3  4.2  4.1  4.0  3.9  3.8  3.7  3.6  ppm

Aspergillus
20 min

A  C

5.0  4.9  4.8  4.7  4.6  4.5  4.4  4.3  4.2  4.1  4.0  3.9  3.8  3.7  3.6  ppm

Fig. 18

Fig. 19

Fig. 20

Fig. 21

Fig. 22

**Agrocybe phytase pH-profile, 5 mM phytate, 30 min at 37 °C**

# Fig. 23

pH -stability of Agrocybe phytase
preincubation 1 h at 40 °C

# Fig. 24

Temperature profile of Agrocybe phytase in 0.1 M
sodium acetate, 5mM phytate, pH 5.5

Fig. 25

Temperature stability of Agrocybe phytase -
preincubation 60 min in 0.1 M sodium acetate pH 5.5

# Fig. 26

Fig. 27

Fig. 28

Fig. 29

Fig. 30

Fig. 31

Fig. 32

A   Ins(1,2,4,5,6)P5 (H-3)

B   Ins(1,2,3,4,5)P5 (H-6)

C   Ins(1,2,5,6)P4 (H-3)

Fig. 33

Fig. 34

Ins(2)P

Agrocybe

Aspergillus

4   6   8  10  12  16   20   24  hours

Fig. 35

EP 0 958 353 B1

Fig. 36

Fig. 37

1D-Ins(1)P

1L-Ins(1)P

# Fig. 38

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2484095 A
- EP 0420358 A
- EP 0684313 A
- WO 9114782 A
- WO 9114772 A
- US 9507743 W

- WO 9311249 A
- WO 9414953 A
- EP 238023 A
- WO 9502043 A


**Non-patent literature cited in the description**

- **THOMLINSON et al.** *Biochemistry,* 1962, vol. 1, 166-171
- **BARRIENTOS et al.** *Plant. Physiol.,* 1994, vol. 106, 1489-1495
- **PAVER ; JAGANNATHAN.** *Journal of Bacteriology,* 1982, vol. 151, 1102-1108
- **COSGROVE.** *Australian Journal of Biological Sciences,* 1970, vol. 23, 1207-1220
- **GREINER et al.** *Arch. Biochem. Biophys.,* 1993, vol. 303, 107-113
- **NAYINI et al.** *Lebensmittel Wissenschaft und Technologie,* 1984, vol. 17, 24-26
- **WODZINSKI et al.** *Adv. Appl. Microbiol.,* vol. 42, 263-303
- **YAMADA et al.** *Agric. Biol. Chem.,* 1986, vol. 322, 1275-1282
- **PIDDINGTON et al.** *Gene,* 1993, vol. 133, 55-62
- *Die Nahrung,* 1995, vol. 39 (5, 6), 483-489
- *Mycological Research,* 1993, vol. 97 (6), 725-732
- *Biochem. J.,* 1989, vol. 258, 1-2
- *Gene,* 1993, vol. 133, 55-62
- *Gene,* 1993, vol. 127, 87-94
- **JÜLICH.** *Higher Taxa of Basidiomycetes,* 1981
- **AINSWORTH ; BISBY.** Dictionary of the Fungi. 1995
- Nordic Macromycetes. 1992, vol. 2
- NORDIC MACROMYCETES. 1997, vol. 3
- **NEEDLEMAN, S.B. ; WUNSCH, C.D.** *Journal of Molecular Biology,* 1970, vol. 48, 443-453
- **EGNELL, P. et al.** *Molecular Microbiol.,* 1992, vol. 6 (9), 1115-19
- **STRYER, L.** Biochemistry. W.H., Freeman and Company
- **J. SAMBROOK ; E.F. FRITSCH ; T. MANIATIS.** Molecular Cloning, A Laboratory Manual. Cold Spring Harbor, 1989

- **FEINBERG, A. P. ; VOGELSTEIN, B.** *Anal. Biochem.,* 1983, vol. 132, 6-13
- Current protocols in Molecular Biology. John Wiley and Sons, 1995
- **SAMBROOK et al.** Molecular Cloning. A Laboratory Manual. Cold Spring Harbor, 1989
- **HAWKSWORTH et al.** Ainsworth and Bisby's Dictionary of The Fungi. CAB International, University Press, 1995
- **TAGUE et al.** *Plant, Phys.,* 1988, vol. 86, 506
- **GASSER et al.** *Science,* vol. 244, 1293
- **POTRYKUS.** *Bio/Techn.,* 1990, vol. 8, 535
- **SHIMAMOTO et al.** *Nature,* 1989, vol. 338, 274
- **HOOYKAS ; SCHILPEROORT.** *Plant Mol. Biol.,* 1992, vol. 19, 15-38
- **CHRISTOU.** *Plant J.,* 1992, vol. 2, 275-281
- **SHIMAMOTO.** *Curr. Opin. Biotechnol.,* 1994, vol. 5, 158-162
- **VASIL et al.** *Bio/Technology,* 1992, vol. 10, 667-674
- **JOSHI ; JOSHI.** *FEBS Lett.,* 1991, vol. 281, 1-8
- **POTYRKUS.** *Annu. Rev. Plant Physiol. Plant Mol. Biol.,* 1991, vol. 42, 205-225
- **CROSSWAY et al.** *Mol. Gen. Genet.,* 1986, vol. 202, 79-85
- **DE LA PENA et al.** *Nature,* 1987, vol. 325, 274-276
- **PIETRZAK et al.** *Nucleic Acids Res.,* 1986, vol. 14, 5857-5868
- **AKAMA et al.** *Plant Cell Reports,* 1992, vol. 12, 7-11
- **ZUPAN ; ZAMBRYSKI.** *Plant Physiol.,* 1995, vol. 107, 1041-1047
- **DEBLAERE et al.** *Nucleic Acids Res.,* 1985, vol. 13, 4777-4788
- **KLEE et al.** *Annu. Rev. Plant Physiol.,* 1987, vol. 38, 467-486

- **FRANCK et al.** *Cell,* 1980, vol. 21, 285-294
- **STITT ; SONNEWALD.** *Annu. Rev. Plant Physiol. Plant Mol. Biol.,* 1995, vol. 46, 341-368
- **EDWARDS ; CORUZZI.** *Annu. Rev. Genet.,* 1990, vol. 24, 275-303
- **ITO et al.** *Plant Mol. Biol.,* 1994, vol. 24, 863-878
- **SAMBROOK et al.** Molecular cloning: A laboratory manual. Cold Spring Harbor lab, 1989
- The Merck Veterinary Manual. Merck & CO., Inc, 1991, 1268
- **JEROCH et al.** *Bodenkultur,* 1994, vol. 45 (4), 361-368
- **POULTRY.** *Science,* 1996, vol. 75 (1), 62-68
- *Canadian Journal of Animal Science,* 1995, vol. 75 (3), 439-444
- **POULTRY.** *Science,* 1995, vol. 74 (5), 784-787
- **POULTRY.** *Science,* 1994, vol. 73 (10), 1590-1596
- Molecular Biological Methods for Bacillus. John Wiley and Sons, 1990
- **VAN DEN HAZEL, H.B ; KIELLAND-BRANDT, M.C. ; WINTHER, J.R.** *Eur. J. Biochem.,* 1992, vol. 207, 277-283
- **H. DALBOEGE et al.** *Mol. Gen. Genet,* 1994, vol. 243, 253-260
- **GUBLER ; HOFFMAN.** *Gene,* 1983, vol. 25, 263-269
- **BECKER ; GUARANTE.** *Methods Enzymol.,* 1991, vol. 194, 182-187
- **TOVE CHRISTENSEN et al.** *Bio/Technology,* December 1988, vol. 6, 1419-1422
- **COVE.** *Biochem. Biophys. Acta,* 1966, vol. 113, 51-56
- **HENRIK NIELSEN ; JACOB ENGELBRECHT ; STREN BRUNAK ; GUNNAR VON HEIJNE.** Identification of prokaryotic and eukaryotic signal peptides and prediction of their cleavage sites. *Protein Engineering,* 1997, vol. 10, 1-6
- **SAMBROOK et al.** Molecular Cloning, a Laboratory Manual
- **LUBERT STRYER.** Biochemistry. Freeman and Company, 1995, 132-134
- **R.H.DON et al.** *Nucleic Acid Research,* 1991, vol. 19 (14
- **SCHOLZ, P. ; BERGMANN, G. ; MAYR, G.W.** Methods in Inositide Research. Raven Press, Ltd, 1990, 65-82
- **VAN DER KAAY et al.** *Biochem. J.,* 1995, vol. 312, 907-910
- **IRVING et al.** *J. Bacteriology,* 1972, vol. 112, 434-438
- **STEVENS, L.R.** Methods in Inositide Research. Raven Press, Ltd, 1990, 9-30
- **STEPHENS, L. et al.** *Biochem. J.,* 1988, vol. 249, 271-282